# FASCICULE DE BREVET EUROPEEN

(11) **EP 2 771 329 B1**
(45) Date de publication et mention de la délivrance du brevet: **16.03.2016**
(21) Numéro de dépôt: 12791825.8
(22) Date de dépôt: 26.10.2012
(51) Int. Cl.: C07D 307/14, C07D 407/06, C07D 409/06, C07D 409/14, A61K 31/341, A61K 31/381, A61K 31/4025, A61P 17/06, A61P 11/00, A61P 19/02, A61P 29/00, A61P 35/00, A61P 37/06

(54) **NOUVEAUX COMPOSÉS DI-SUBSTITUÉS DE LA DIAMINO-3,4-CYCLOBUTÈNE-3-DIONE-1,2 UTILES DANS LE TRAITEMENT DE PATHOLOGIES MÉDIÉES PAR DES CHIMIOKINES**
NEUE DISUBSTITUIERTE 3,4-DIAMINO-3-CYCLOBUTEN-1,2-DION-VERBINDUNGEN ZUR VERWENDUNG BEI DER BEHANDLUNG CHEMOKINVERMITTELTER ERKRANKUNGEN
NOVEL DISUBSTITUTED 3,4-DIAMINO-3-CYCLOBUTENE-1,2-DIONE COMPOUNDS FOR USE IN THE TREATMENT OF CHEMOKINE-MEDIATED DISEASES

(30) Priorité: 28.10.2011 US 201161552940 P; 28.10.2011 FR 1159833
(43) Date de publication de la demande: 03.09.2014
(73) Titulaire: Galderma Research & Development, 06410 Biot (FR)
(72) Inventeur: MUSICKI, Branislav, F-06000 Nice (FR); AUBERT, Jerôme, F-06130 Grasse (FR); BOITEAU, Jean-Guy, F-06560 Valbonne (FR); CLARY, Laurence, F-06480 La Colle Sur Loup (FR); ROSSIO, Patricia, F-06130 Grasse (FR); SCHUPPLI-NOLLET, Marlène, F-06620 Le Bar Sur Loup (FR)
(74) Mandataire: Casalonga
(86) Numéro de dépôt international: PCT/FR2012/052478
(87) Numéro de publication internationale: WO 2013/061004

(56) Documents cités:
- WO-A1-02/083624
- WO-A1-2010/063802

## Description

### Domaine de l'invention :

La présente invention porte sur de nouveaux composés di-substitués de la diamino-3,4-cyclobutène-3-dione-1,2, sur les compositions pharmaceutiques contenant ces composés ainsi que sur l'utilisation de ces composés et de ces compositions pour le traitement de pathologies médiées par des chimiokines.

### Etat de la technique antérieur à l'invention :

Les chimiokines ou cytokines sont de petites protéines solubles. Leur rôle le plus connu est l'attraction et le contrôle de l'état d'activation des cellules du système immunitaire. Toutes les chimiokines exercent leurs fonctions en se fixant sur des récepteurs couplés aux protéines G. Certaines chimiokines sont considérées comme pro-inflammatoires. La sécrétion de ces chimiokines peut être induite lors de la réponse immune afin de favoriser l'arrivée de cellules du système immunitaire au niveau d'un site infectieux.
Il existe deux types de chimiokines : les chimiokines pro-inflammatoires et les chimiokines constitutives.
Les chimiokines pro-inflammatoires (ou « inductibles ») sont produites au niveau de sites d'inflammation par des cellules de tissus ou des leucocytes infiltrés, après contact avec un agent pathogène.
Les chimiokines constitutives (ou « homéostatiques ») sont produites dans les organes lymphoïdes et dans certains organes non-lymphoïdes tels que la peau et les muqueuses. Elles régulent le trafic lymphocytaire et la localisation des lymphocytes au sein de ces organes pendant la lymphopoïèse mais également pour maintenir l'immunosurveillance.
La nomenclature de ces récepteurs à chimiokines est basée sur le groupe de chimiokines auquel son ligand appartient. Ainsi, les récepteurs correspondant aux chimiokines du groupe CXC sont, par exemple, appelés CXCR1, CXCR2, CXCR3, CXCR4, etc, et les récepteurs correspondant aux chimiokines du groupe CC sont, par exemple, appelés CCR1, CCR2, CCR3, etc. Ces récepteurs présentent tous une structure tertiaire semblable, et ils sont couplés à une protéine G : ils font donc partie de la superfamille des GPCR (G Protein Coupled Receptor).
L'interleukine-8 ou IL-8 (également nommé CXCL-8) est un membre de la famille des chimiokines CXC, qui joue un rôle primordial dans le recrutement des neutrophiles vers le site d'inflammation. Deux récepteurs, CXCR1 et CXCR2 sont connus pour être spécifiquement activés par IL-8. Alors que CXCR2 se lie avec une forte affinité à IL-8 et aux chimiokines apparentées comme CXCL6, CXCL5, CXCL3, CXCL2 et CXCL1, CXCR1 se lie uniquement à IL-8. Des niveaux élevés d'IL-8 et de chimiokines apparentées (CXCL5, CXCL2 & CXCL1) ont été décrits dans les lésions d'acné inflammatoire (J Invest Dermatol. 2006;126:1071-9; Am J Pathol. 2005;166(6):1691-9; Diagn Pathol. 2007 Jan 30;2:4).
Des premiers indices démontrent l'expression de CXCR2 dans l'acné inflammatoire (Trivedi et al. J Invest Dermatol. 2006 126(5) :1171-9). Ainsi, des antagonistes doubles de CXCR1 et CXCR2 pourrait permettre de diminuer rapidement les effets délétères de la réponse inflammatoire par IL-8.

La demande de brevet WO 02/083624 (SCHERING/PHARMACOPEIA) divulgue plus particulièrement des composés substitués de la cyclobutenedione-1,2 capables de moduler l'activité des récepteurs aux chimiokines de type CXC, et plus particulièrement l'activité des récepteurs CXCR1 et CXCR2. Parmi ces composés, le composé SCH-527123 (correspondant à l'exemple 360.31 en page 281), appelé également Navarixin, est en cours de développement (Phase II) pour le traitement de la maladie pulmonaire obstructive chronique (ou COPD pour Chronic Obstructive Pulmonary Disease). Ce composé a également fait l'objet d'études de phase II dans l'asthme et dans le psoriasis, mais ces développements ont été arrêtés.

Il est aujourd'hui connu que de nombreuses pathologies d'ordre inflammatoire sont médiées par des chimiokines. Il existe cependant un besoin non satisfait à ce jour de traiter la composante inflammatoire des pathologies d'intérêt dans le domaine de la dermatologie comme par exemple l'acné, la rosacée ou encore les dermatoses neutrophiliques, notamment le psoriasis.
De la même manière, la promesse d'obtenir de nouvelles thérapies efficaces pour traiter des maladies médiées par des chimiokines à l'aide d'antagonistes des récepteurs aux chimiokines n'a pas été tenue. En effet, plusieurs études cliniques ont échoué en phase II. Une des raisons pouvant expliquer ces échecs est l' « overlap » des effets biologiques des différentes chimiokines induites en situation pathologique. A ce jour, le traditionnel « Drug discovery process » a pour objectif d'identifier des molécules ciblant un récepteur spécifique sans effet « off target ». Cette approche n'est sans doute pas la plus adaptée pour traiter des maladies inflammatoires complexes. De plus en plus d'approches semblent privilégier la recherche de molécules antagonistes à large spectre d'action (« promiscuous compounds »), lesdites approches pouvant ainsi s'avérer plus efficaces pour traiter des maladies complexes et multifactorielles (Frantz S. Drug discovery: playing dirty. Nature. 2005 Oct 13;437(7061):942-3 ; Roth BL, Sheffler DJ, Kroeze WK. Magic shotguns versus magic bullets: selectively non-selective drugs for mood disorders and schizophrenia. Nat Rev Drug Discov. 2004 Apr;3(4):353-9.)

Or, la demanderesse a découvert de nouveaux composés présentant une activité antagoniste non seulement vis-à-vis des récepteurs de type CXCR1 et CXCR2, mais également une forte activité antagoniste vis-à-vis des récepteurs aux chimiokines, notamment les récepteurs CCR6 et CXCR3. Ces nouveaux composés présentent de manière surprenante une polypharmacologie, ce qui leur confère un intérêt supplémentaire par rapport aux composés déjà connus dans le traitement de pathologies médiées par des chimiokines, et plus particulièrement des pathologies d'ordre dermatologique.
De plus, ces nouveaux composés présentent une stabilité hépatique bien inférieure à celle des composés déjà décrits capables de bloquer l'activation des récepteurs CXCR1 et CXCR2 comme par exemple le composé SCH-527123. Cette propriété particulière présente l'avantage de disposer de nouveaux composés ayant, de manière surprenante, un profil plus adapté pour le traitement par voie topique de pathologies d'ordre dermatologique. En effet, leur instabilité hépatique entraine une exposition systémique faible, voire nulle, et donc des effets secondaires limités.
Une autre particularité des composés décrits dans la présente invention est leur constante de dissociation vis-à-vis des récepteurs de type CXCR1 et CXCR2, constante qui est bien inférieure à celle des composés décrits dans la demande de brevet WO 02/083624, comme par exemple le SCH-527123. En effet, la molécule SCH-527123 a été décrite pour avoir un temps de dissociation de l'ordre de 22h (Dissociation pseudo-irréversible) (Pharmacological Characterization of SCH-527123, a Potent Allosteric CXCR1/CXCR2 Antagonist. JPET 322:477-485, 2007), alors que les temps de dissociation des composés de la présente invention sont bien inférieurs.

Des exemples de la littérature montrent qu'une dissociation rapide des antagonistes favorise une diminution de leur toxicité. Ceci a été décrit pour les antagonistes des récepteurs dopamine D2 (Am J Psychiatry (2001) 158(3):360-369), des récepteurs *N*-methyl-D-aspartate (NMDA) (Nat Rev Drug Disc (2006) 5(2):160-170.) ainsi que pour les anti-inflammatoires non stéroidiens (Lett Drug Des Discov (2006) 3(8):569-574*. et* Pharm Med (2008) 22(1):23-34). En effet, un temps de dissociation long aurait plutôt tendance à induire des effets indésirables. Avec des temps de dissociation rapide, les composés selon l'invention présentent par conséquent des effets secondaires réduits.

### Résumé de l'invention :

Un premier objet selon l'invention concerne de nouveaux composés di-substitués de la diamino-3,4- cyclobutène-3-dione-1,2 répondant à la formule générale (I) suivante: 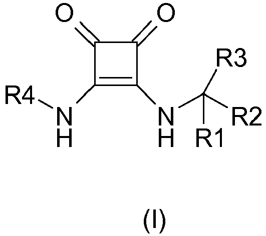 ainsi que leurs sels, solvates ou hydrates pharmaceutiquement acceptables pour lesquels les substituants R1, R2, R3 et R4 sont tels que définis ci-après dans la description détaillée de l'invention.

Un second objet selon l'invention concerne une composition pharmaceutique comprenant une quantité efficace d'un composé répondant à la formule générale (I), d'un de ses sels ou un de ses solvates ou hydrates pharmaceutiquement acceptable en association avec un solvant ou un support pharmaceutiquement acceptable.

Un troisième objet selon l'invention concerne un composé ou une composition pharmaceutique tel que décrit ci-dessus pour son utilisation en tant que médicament.

Un quatrième objet selon l'invention concerne un composé ou une composition pharmaceutique tel que décrit ci-dessus pour son utilisation dans le traitement de maladies médiées par les chimiokines.

Un cinquième objet selon l'invention concerne un composé ou composition pharmaceutique tel que décrit ci-dessus pour son utilisation dans le traitement de maladies du groupe comprenant les dermatoses neutrophiliques, et notamment le psoriasis, les dermatites atopiques, l'acné, la rosacée, l'asthme, les maladies pulmonaire obstructives chroniques, les maladies respiratoires des adultes, l'arthrite, les maladies intestinales inflammatoires, la maladie de Crohn, le rejet de greffe, la mucoviscidose et les cancers cutanés.

### Description détaillée de l'invention :

A moins qu'il en soit indiqué autrement, les définitions suivantes s'appliquent à l'ensemble de la description et des revendications.
Ces définitions s'appliquent indépendamment de savoir si un terme est utilisé seul ou en combinaison avec d'autres termes. Ainsi, par exemple, la définition du terme « aryle » s'applique aussi bien à « aryle » en tant que tel qu'à la partie « aryle » du terme « aryloxy ».

"Alkyle" désigne une chaîne hydrocarbonée saturée linéaire ou ramifiée dont le nombre d'atomes de carbone est précisé.
Lorsque le nombre d'atomes de carbone n'est pas précisé, cela signifie que la chaine alkyle contient de 1 à 20 atomes de carbone.
Les radicaux alkyles préférés contiennent de 1 à 12 atomes de carbone, et ceux encore plus préférés contiennent de 1 à 6 atomes de carbone dans la chaîne.

«Alkoxy" désigne un oxygène substitué par un radical alkyle tel que défini précédemment. Des exemples de radicaux alkoxy comprennent les radicaux méthoxy, éthoxy, n-propoxy, isopropoxy et n-butoxy.

«Aryle«» désigne un système cyclique aromatique monocyclique ou polycyclique (2 à 3 cycles) comprenant de 6 à 14 atomes de carbone, et de préférence de 6 à 10 atomes de carbone.
A titre d'exemples de radicaux aryle, on peut citer les radicaux phényle, naphtyle, indényle, tétrahydronaphtyle, indanyle, anthracényle et fluorényle.

« Hétéroaryle » désigne un système aromatique monocyclique ou polycyclique (2 à 3 cycles) comprenant de 5 à 14 atomes cycliques, de préférence de 5 à 10 atomes cycliques, dans lequel un ou plusieurs des atomes cycliques représente(nt) un ou plusieurs (de 1 à 5) hétéroatome(s) choisi(s) dans le groupe comprenant l'azote, l'oxygène et le soufre.
Les hétéroaryles préférés contiennent 5 ou 6 atomes cycliques et 1 à 3 hétéroatomes.
Le préfixe aza, oxa ou thia avant le nom de la racine hétéroaryle signifie qu'au moins un azote, un où un soufre est respectivement présent dans le cycle.
Un atome d'azote d'un hétéroaryle peut être éventuellement oxydé en N-oxyde. A titre d'exemples d'hétéroaryles appropriés, on peut citer les hètéroaryles suivants :
pyridyle, pyrazinyle, furanyle, thionyle, pyrimidinyle, isoxazolyle, isothiazolyle, oxazolyle, triazolyle, pyrazolyle, furazanyle, pyrrolyle, pyrazolyle, triazolyle, le 1,2,4-thiadiazolyle, pyrazinyle, pyridazinyle, quinoxalinyle, phtalazinyle, imidazo [1,2-a] pyridinyle, imidazo [2,1-b] triazolyle, benzofurazanyl, indolyle, azaindolyle, benzimidazolyle, benzothiényle, quinolinyle, imidazolyle, thienopyridyle, quinazolinyle, thienopyrimidyle, pyrrolopyridyle, imidazopyridyle, isoquinolinyle, benzoazaindolyle, 1,2,4 triazinyle et benzothiazolyle.

«Arylalkyle» désigne un radical dont les parties aryle et alkyle sont telles que définies ci-dessus.
A titre d'exemples d'arylalkyle, on peut citer les radicaux benzoyle, phénéthyle et naphthalènylméthyle.
La liaison à la structure à laquelle il est rattaché se fait par le radical alkyle.

«Hétéroarylalkyle» désigne un radical dont les parties hétéroaryle et alkyle sont telles que définies ci-dessus.
A titre d'exemples d'hétéroarylalkyle, on peut citer les radicaux pyridylméthyle, pyridyléthyle, imidazolylméthyle, imidazolyléthyle, pyrazolylméthyle et pyrazolyléthyle.
La liaison à la structure à laquelle il est rattaché se fait par le radical alkyle.

«Cycloalkyle» désigne un système cyclique hydrocarboné non aromatique, ayant de 3 à 10 atomes de carbone, de préférence de 5 à 10 atomes de carbone, et de un à trois cycles.

Les radicaux cycloalkyles préférés contiennent de 5 à 7 atomes cycliques.
A titre d'exemples de radicaux cycloalkyle, on peut citer les radicaux cyclopropyle, cyclopentyle, cyclohexyle, cycloheptyle, norbornyle et adamantyle.

«Cycloalkylalkyle" désigne un radical dont les parties cycloalkyle et alkyle sont telles que définies ci-dessus.
A titre d'exemples de cycloalkylalkyle, on peut citer les radicaux cyclopropylméthyle, cyclopropyléthyle, cyclobutylméthyle, cyclobutyléthyle, cyclopentylméthyle, cyclopentyléthyle, cyclohexylméthyle, cyclohexyléthyle, norbornylméthyle et adamantylméthyle.
La liaison à la structure à laquelle il est rattaché se fait par le radical alkyle.

«Hétérocycloalkyle » désigne un système cyclique hydrocarboné non aromatique, ayant de 4 à 10 atomes de carbone, de préférence de 5 à 10 atomes de carbone, et de un à trois cycles, et comprenant de un à trois hétéroatomes choisi dans le groupe constitué par l'azote, l'oxygène et le soufre.
Les radicaux hétérocycloalkyles préférés contiennent de 5 à 7 atomes cycliques.
A titre d'exemples de radicaux hétérocycloalkyle, on peut citer les radicaux tétrahydrofuranyle, tétrahydrothiophényle, tétrahydropyrannyle et 7-oxa-bicyclo-[2.2.1]-heptanyle.

« Alkyle fluoré » désigne un radical alkyle tel que défini précédemment substitué par un ou plusieurs atomes de fluor.
A titre d'exemples de radicaux alkyle fluoré, on peut citer les radicaux fluorométhyle, difluorométhyle, 2-fluoroéthyle, 2,2-difluoroéthyle et 2,2,2-trifluoroéthyle.

« Alkyle perfluoré » désigne un radical alkyle tel que défini précédemment dans lequel chaque atome d'hydrogène a été substitué par un atome de fluor.
A titre d'exemples de radicaux perfluoré, on peut citer les radicaux trifluorométhyle, et pentafluoroéthyle.

Ainsi, un premier objet selon l'invention concerne de nouveaux composé di-substitués de la diamino-3,4- cyclobutène-3-dione-1,2 répondant à la formule générale (I) suivante ou un de ses sels ou solvates pharmaceutiquement acceptable: 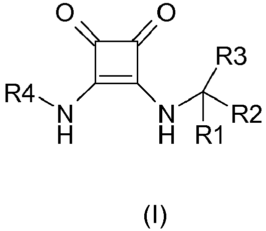
R1 représente un atome d'hydrogène ou un méthyle,
R2 représente un cycle à cinq atomes choisi parmi les structures (1), (2), (3) et (4) suivantes: 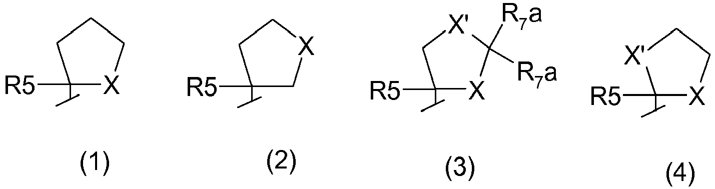 dans lesquelles R5, R7a, X et X' ont la signification donnée ci-après,
R3 représente un cycle aromatique ou hétéroaromatique sélectionné dans le groupe constitué par les cycles répondant aux formules (a) à (o) suivantes : 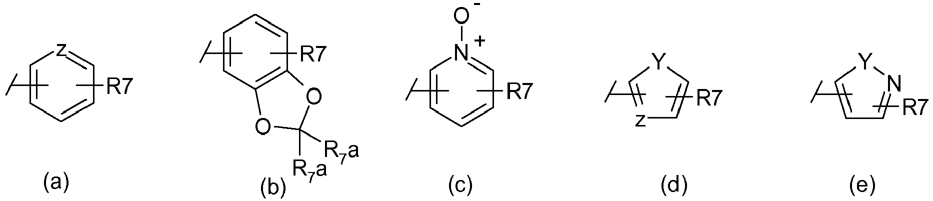 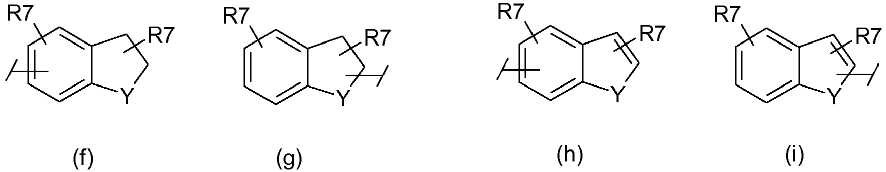 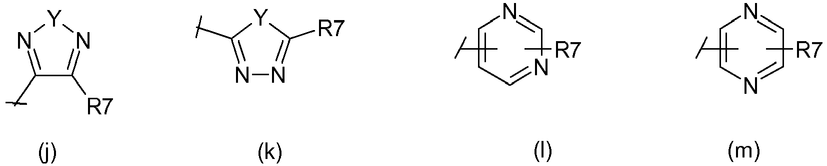 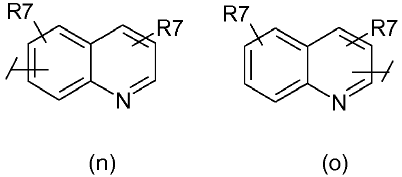 dans lesquelles R7, R7a, Y et Z ont la signification donnée ci-après, étant précisé que les cycles (a) à (o) peuvent éventuellement porter plusieurs groupes R7, identiques ou différents, le nombre total de tels groupes R7 étant au maximum égal au nombre d'atomes substituables du cycle ;
R4 représente un cycle aromatique ou hétéroaromatique sélectionné dans le groupe constitué par les cycles répondant aux formules (p) à (z) et (aa) à (ak) suivantes : 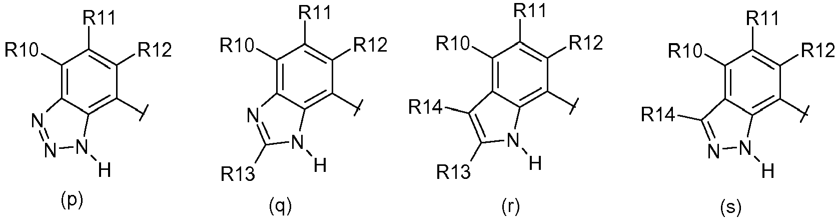 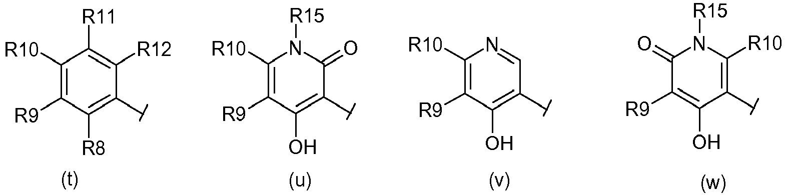 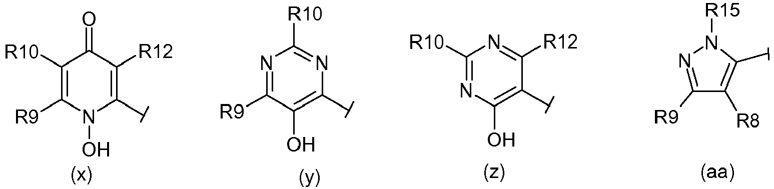 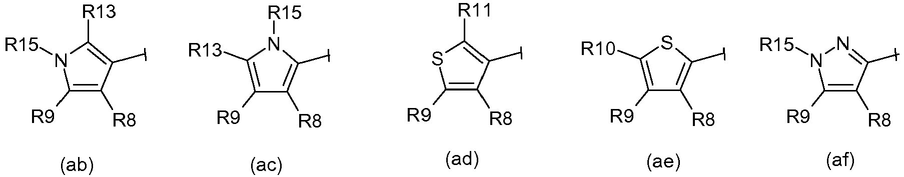 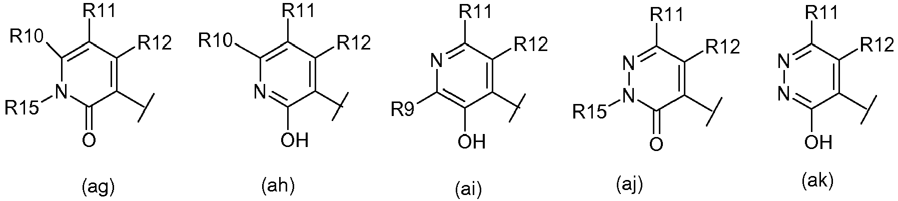 dans lesquelles R7, R8, R9, R10, R11, R12, R13, R14 et R15 ont la signification donnée ci-après,
R5 représente un atome d'hydrogène, un atome de fluor, un radical alkyle ayant de 1 à 5 atomes de carbones ou un radical alkyle fluoré ou perfluoré comportant de 1 à 5 atomes de carbones,
R6 représente un atome d'hydrogène, un radical -COOtBu ou un radical -COOBn,
R7 représente un halogène, un radical -R16, -CF3, -COR16, -OR16, -NR16R17, -NO2, -CN,-SO2R16, -SO2NR16R17, -NR16COR17, -CONR16R17, -NR16CO2R17 ou -CO2R16,
R7a représente un atome d'hydrogène ou un radical alkyle ayant de 1 à 5 atomes de carbones,
R8 représente un atome d'hydrogène, un atome d'halogène, un radical -OH, un radical -SH,-CONHOR16, -CONR16OH, -NR16R17, -SO3H, -OCOR16, -NHSO2R16, -SO2NR16R17,-NHCOR16, -CONR16R17, -NR16CO2R17, -NHSO2NR16R17, -CO2R16, pyrrolyle, imidazolyle, triazolyle ou tetrazolyle,
R9, R10, R11 et R12 sont identiques ou différents et sont indépendamment choisis dans le groupe constitué par un hydrogène, un atome d'halogène, un radical alkyle, alkoxy, -CF3,-OCF3, -OH, -NO2, -CN, -SO2R16, -SO2NR16R17, -NR16COR17, -NR16CO2R17,-CONR16R17, -COR16 ou -CO2R16,
ou bien encore, lorsque deux des radicaux R9, R10, R11 et R12 se trouvent en position ortho sur un cycle aromatique ou hétéroaromatique sélectionné dans le groupe constitué par les cycles répondant aux formules (p) à (z) et (aa) à (ak) ci-dessus, alors ils peuvent former ensemble, avec la liaison qui les réunit, un cycle aryle, hétéroaryle, cycloalkyle ou hétérocycloalkyle,
R13 et R14 sont identiques ou différents et sont indépendamment choisis dans le groupe constitué par un atome d'hydrogène, un atome d'halogène, un radical alkyle, -CF3, -OCF3,-OH, -SH, -CN, -SO2R16, -SO2NR16R17, -NHSO2NR16R17, -NR16R17,-NR16CONR16R17, -NR16COR17, -NR16CO2R17, -CONR16R17, -COR16 ou -CO2R16,
R15 représente un atome d'hydrogène, un radical -OH, -SO2R16, -COR16, -CO2R16, aryle, heteroaryle, arylalkyle, heteroarylalkyle, alkyle, cycloalkyle ou cycloalkylalkyle,
R16 et R17 sont identiques ou différents et sont indépendamment choisis dans le groupe constitué par un atome d'hydrogène, un radical aryle, heteroaryle, arylalkyle, heteroarylalkyle, alkyle, alkyle fluoré ayant de 1 à 5 atomes de carbone, cycloalkyle ou cycloalkylalkyle, un groupe -CH2COOR18 dans lequel R18 représente un radical alkyle ayant de 1 à 5 atomes de carbone,
ou bien encore lorsque R16 et R17 sont portés par un même atome d'azote ils forment un hétérocycle ayant entre 3 et 7 chainons et comportant éventuellement un ou deux hétéroatomes choisis parmi l'oxygène , le soufre et l'azote en plus de l'atome d'azote commun par lequel ils sont portés, ledit hétérocycle pouvant être substitué par un groupe alkyle ayant de 1 à 5 atomes de carbone ou un groupe -COOR18 dans lequel R18 représente un radical alkyle ayant de 1 à 5 atomes de carbone ;.
X et X', identiques ou différents, représentent un atome d'oxygène, un atome de soufre, ou un atome d'azote substitué par un radical R6,
Y représente un atome d'oxygène, un atome de soufre, ou un atome d'azote substitué par un radical R15, et
Z représente un atome de carbone ou d'azote

Dans un mode de réalisation préféré selon l'invention, les composés ainsi que leurs sels, solvates ou hydrates pharmaceutiquement acceptables, répondent à la formule générale (I) précitée dans laquelle:
R1 représente un atome d'hydrogène,
R2 représente un cycle à cinq chainons choisi parmi les structures (1), (2) et (3) suivantes: 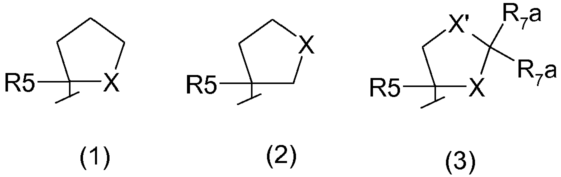 dans lesquelles R5, R7a, X et X' ont la signification donnée ci-après,
R3 représente un cycle aromatique ou hétéroaromatique sélectionné dans le groupe constitué par les cycles répondant aux formules (a), (b) et (d) suivantes : 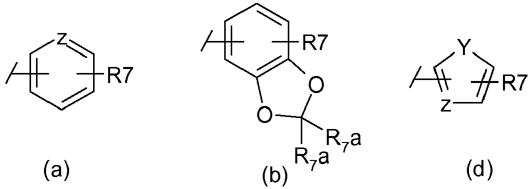 dans lesquelles R7, R7a, Y et Z ont la signification donnée ci-après, étant précisé que les cycles (a), (b) et (d) peuvent éventuellement porter plusieurs groupes R7, identiques ou différents, le nombre total de tels groupe R7 étant au maximum égal au nombre d'atomes substituables du cycle ;
R4 représente un cycle aromatique ou hétéroaromatique sélectionné dans le groupe constitué par les cycles répondant aux formules (p), (q), (t), (z), (ad), (ag) et (ah) suivantes : 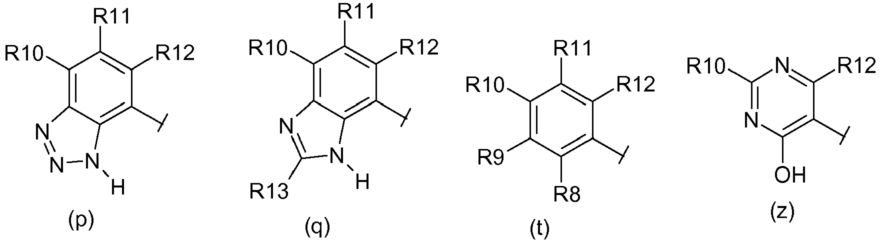 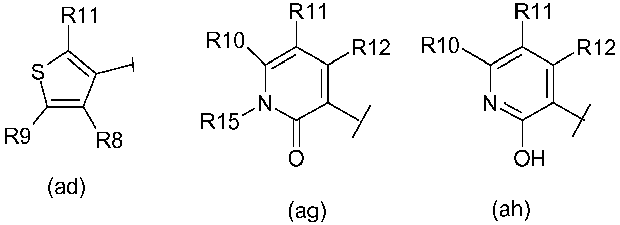 dans lesquelles R8, R9, R10, R11, R12, R13 et R15 ont la signification donnée ci-après,
R5 représente un atome d'hydrogène, un atome de fluor, un radical alkyle ayant de 1 à 5 atomes de carbones ou un radical alkyle fluoré ou perfluoré comportant de 1 à 5 atomes de carbones,
R6 représente un atome d'hydrogène, un radical -COOtBu ou un radical -COOBn,
R7 représente un halogène, un radical R16, -CF3, -COR16, -OR16, -NR16R17, -NO2, -CN,-SO2R16, -SO2NR16R17, -NR16COR17, -CONR16R17, -NR16CO2R17 ou -CO2R16,
R7a représente un hydrogène ou un radical alkyle ayant de 1 à 5 atomes de carbones,
R8 représente un atome d'hydrogène, un radical -OH, -SH, -CONHOR16, -CONR16OH,-NR16R17, -SO3H, -OCOR16, -NHSO2R16, -SO2NR16R17, -NHCOR16, -CONR16R17,-NR16CO2R17, -NHSO2NR16R17, -CO2R16, pyrrolyle, imidazolyle, triazolyle ou tétrazolyle,
R9, R10, R11 et R12 sont identiques ou différents et sont indépendamment choisis dans le groupe constitué par un atome d'hydrogène, un atome d'halogène, un radical alkyle, alkoxy,-CF3, -OCF3, -OH, -NO2, -CN, -SO2R16, -SO2NR16R17, -NR16COR17, -NR16CO2R17,-CONR16R17, -COR16 ou -CO2R16,
ou bien encore, lorsque deux des radicaux R9, R10, R11 et R12 se trouvent en position ortho sur un cycle aromatique ou hétéroaromatique sélectionné dans le groupe constitué par les cycles répondant aux formules (p), (q), (t), (z), (ad), (ag) et (ah) ci-dessus, alors ils peuvent former ensemble, avec la liaison qui les réunit, un cycle aryle, hétéroaryle, cycloalkyle ou hétérocycloalkyle,
R13 est choisi dans le groupe constitué par un atome d'hydrogène, un atome d'halogène, un radical alkyle, -CF3, -OCF3, -OH, -SH, -CN, -SO2R16, -SO2NR16R17, -NHSO2NR16R17,-NR16R17, -NR16CONR16R17, -NR16COR17, -NR16CO2R17, -CONR16R17, -COR16 ou-CO2R16,
R15 représente un atome d'hydrogène, un radical -OH, -SO2R16, -COR16, -CO2R16, aryle, hétéroaryle, arylalkyle, hétéroarylalkyle, alkyle, cycloalkyle ou cycloalkylalkyle,
R16 et R17 sont identiques ou différents et sont indépendamment choisis dans le groupe constitué par un atome d'hydrogène, un radical aryle, heteroaryle, arylalkyle, heteroarylalkyle, alkyle, alkyle fluoré ayant de 1 à 5 atomes de carbone, cycloalkyle ou cycloalkylalkyle, un groupe -CH2COOR18 dans lequel R18 représente un radical alkyle ayant de 1 à 5 atomes de carbone,
ou bien encore lorsque R16 et R17 sont portés par un même atome d'azote ils forment un hétérocycle ayant entre 3 et 7 chainons et comportant éventuellement un ou deux hétéroatomes choisis parmi l'oxygène , le soufre et l'azote en plus de l'atome d'azote commun par lequel ils sont portés, ledit hétérocycle pouvant être substitué par un groupe alkyle ayant de 1 à 5 atomes de carbone ou un groupe -COOR18 dans lequel R18 représente un radical alkyle ayant de 1 à 5 atomes de carbone ;.
X et X', identiques ou différents, représentent un atome d'oxygène, un atome de soufre, ou un atome d'azote substitué par un radical R6,
Y représente un atome d'oxygène, un atome de soufre, ou un atome d'azote substitué par un radical R15, et
Z représente un atome de carbone ou d'azote.

Dans un mode de réalisation plus particulièrement préféré selon l'invention, les composés ainsi que leurs sels, solvates ou hydrates pharmaceutiquement acceptables, répondent à la formule (I) précitée dans laquelle:
R1 représente un atome d'hydrogène,
R2 représente un cycle à cinq atomes de structure (1) suivante: 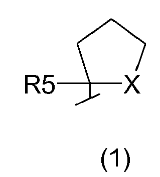 dans laquelle R5 et X ont la signification donnée ci-après,
R3 représente un cycle hétéroaromatique répondant à la formule (d) suivante : 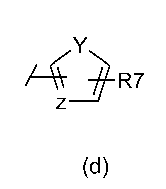 dans laquelle R7, Y et Z ont la signification donnée ci-après, étant précisé que le cycle (d) peut éventuellement porter plusieurs groupes R7, identiques ou différents, le nombre total de tels groupe R7 étant au maximum égal au nombre d'atomes substituables du cycle ;
R4 représente un cycle aromatique répondant à la formule (t) suivante : 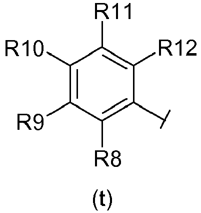 dans laquelle R8, R9, R10, R11 et R12 ont la signification donnée ci-après,
R5 représente un atome d'hydrogène, un atome de fluor, un radical alkyle ayant de 1 à 5 atomes de carbones ou un radical alkyle fluoré ou perfluoré comportant de 1 à 5 atomes de carbones,
R6 représente un atome d'hydrogène, un radical -COOtBu ou un radical -COOBn,
R7 représente un atome d'halogène, un radical R16, -CF3, -COR16, -OR16, -NR16R17,-NO2, -CN, -SO2R16, -SO2NR16R17, -NR16COR17, -CONR16R17, -NR16CO2R17 ou-CO2R16,
R8 représente un atome d'hydrogène, un radical -OH, -SH, -CONHOR16, -CONR16OH,-NR16R17, -SO3H, -OCOR16, -NHSO2R16, -SO2NR16R17, -NHCOR16, -CONR16R17,-NR16CO2R17, -NHSO2NR16R17, -CO2R16, pyrrolyle, imidazolyle, triazolyle ou tetrazolyle,
R9, R10, R11 et R12 sont identiques ou différents et sont indépendamment choisis dans le groupe constitué par un atome d'hydrogène, un atome d'halogène, un radical alkyle, alkoxy,-CF3, -OCF3, -OH, -NO2, -CN, -SO2R16, -SO2NR16R17, -NR16COR17, -NR16CO2R17,-CONR16R17, -COR16 ou -CO2R16,
ou bien encore, lorsque deux des radicaux R9, R10, R11 et R12 se trouvent en position ortho sur le cycle aromatique (t), ils peuvent former ensemble, avec la liaison qui les réunit, un cycle aryle, hétéroaryle, cycloalkyle ou hétérocycloalkyle,
R15 représente un atome d'hydrogène, un radical -OH, -SO2R16, -COR16, -CO2R16, aryle, hétéroaryle, arylalkyle, hétéroarylalkyle, alkyle, cycloalkyle ou cycloalkylalkyle,
R16 et R17 sont identiques ou différents et sont indépendamment choisis dans le groupe constitué par un atome d'hydrogène, un radical aryle, heteroaryle, arylalkyle, heteroarylalkyle, alkyle, alkyle fluoré ayant de 1 à 5 atomes de carbone, cycloalkyle ou cycloalkylalkyle, un groupe -CH2COOR18 dans lequel R18 représente un radical alkyle ayant de 1 à 5 atomes de carbone,
ou bien encore lorsque R16 et R17 sont portés par un même atome d'azote ils forment un hétérocycle ayant entre 3 et 7 chainons et comportant éventuellement un ou deux hétéroatomes choisis parmi l'oxygène , le soufre et l'azote en plus de l'atome d'azote commun par lequel ils sont portés, ledit hétérocycle pouvant être substitué par un groupe alkyle ayant de 1 à 5 atomes de carbone ou un groupe -COOR18 dans lequel R18 représente un radical alkyle ayant de 1 à 5 atomes de carbone ;.
X représente un atome d'oxygène, un atome de soufre, ou un atome d'azote substitué par un radical R6,
Y représente un atome d'oxygène, un atome de soufre, ou un atome d'azote substitué par un radical R15, et
Z représente un atome de carbone ou un d'azote.

Parmi les composés plus particulièrement préférés, on peut citer par exemple ceux choisis dans la liste comprenant:
1/- 2-hydroxy-N,N-diméthyl-3-(2-{[(5-méthyl-furan-2-yl)-(2-méthyl-tétrahydrofuran-2-yl)-méthyl]-amino}-3,4-dioxo-cyclobut-1-enylamino)-benzamide
2/- 2-hydroxy-*N*,*N-*diméthyl-3-(2-{[(5-méthyl-furan-2-yl)-(tétrahydro-thiophèn-2-yl)-méthyl]-amino}-3,4-dioxo-cyclobut-1-enylamino)-benzamide
3/- (S)-1-[2-fluoro-3-(2-{[(5-méthyl-furan-2-yl)-(tétrahydro-thiophèn-2-yl)-méthyl]-amino}-3,4-dioxo-cyclobut-1-énylamino)-benzoyl]-pyrrolidine-2-carboxylate de méthyle
4/- (S)-1-[2-fluoro-3-(2-{[(5-méthyl-furan-2-yl)-(tétrahydro-thiophen-2-yl)-méthyl]-amino}-3,4-dioxo-cyclobut-1-enylamino)-benzoyl]-pyrrolidine-2-carboxylate d'isopropyle
5/- (S)-1-[2-fluoro-3-(2-{[(5-méthyl-furan-2-yl)-(tétrahydro-thiophen-2-yl)-méthyl]-amino}-3,4-dioxo-cyclobut-1-enylamino)-benzoyl]-pyrrolidine-2-carboxylate d'éthyle
6/- (R)-1-[2-hydroxy-3-(2-{[(5-methyl-furan-2-yl)-(tetrahydro-thiophen-2-yl)-methyl]-amino}-3,4-dioxo-cyclobut-1-enylamino)-benzoyl]-pyrrolidine-2-carboxylate de méthyle
7/- (S)-1-[2-hydroxy-3-(2-{[(5-methyl-furan-2-yl)-(tetrahydro-thiophen-2-yl)-methyl]-amino}-3,4-dioxo-cyclobut-1-enylamino)-benzoyl]-pyrrolidine-2-carboxylate de méthyle
8/- 2-hydroxy-N-méthyl-3-(2-{[(S)-(5-méthyl-furan-2-yl)-tétrahydro-thiophen-2-yl-méthyl]-amino}-3,4-dioxo-cyclobut-1-enylamino)-N-(2,2,2-trifluoro-éthyl)-benzamide
9/- {[2-hydroxy-3-(2-{[(5-méthyl-furan-2-yl)-(tétrahydro-thiophen-2-yl)-méthyl]-amino}-3,4-dioxo-cyclobut-1-enylamino)-benzoyl]-méthyl-amino}-acétate de méthyle
10/- 6-chloro-2-hydroxy-N,N-diméthyl-3-(2-{[(5-méthyl-furan-2-yl)-(tétrahydro-thiophèn-2-yl)-méthyl]-amino}-3,4-dioxo-cyclobut-1-enylamino)-benzènesulfonamide
11/- 2-hydroxy-N,N-dimethyl-3-(2-{[(R)-(5-methyl-furan-2-yl)-tetrahydro-furan-2-yl-methyl]-amino}-3,4-dioxo-cyclobut-1-enylamino)-benzamide
12/- 2-hydroxy-N,N-dimethyl-3-(2-{[(S)-(5-methyl-furan-2-yl)-tetrahydro-furan-2-yl-methyl]-amino}-3,4-dioxo-cyclobut-1-enylamino)-benzamide
15/ (S)-1-[2-Fluoro-3-(2-{[(5-méthyl-furan-2-yl)-(tétrahydro-thiophen-2-yl)-méthyl]-amino}-3,4-dioxo-cyclobut-1-enylamino)-benzoyl]-pyrrolidine-2-carboxylate de méthyle
18/ (-)-2-hydroxy-N-méthyl-3-(2-{[(S)-(5-méthyl-furan-2-yl)-(tétrahydro-thiophen-2-yl)-méthyl]-amino}-3,4-dioxo-cyclobut-1-enylamino)-N-(2,2,2-trifluoro-éthyl)-benzamide
19/ (-)-{[2-hydroxy-3-(2-{[(S)-(5-méthyl-furan-2-yl)-(tétrahydro-thiophen-2-yl)-méthyl]-amino}-3,4-dioxo-cyclobut-1-enylamino)-benzoyl]-méthyl-amino}-acétate de méthyle
20/ (-)-1-[2-hydroxy-3-(2-{[((S)-5-méthyl-furan-2-yl)-(tétrahydro-thiophen-2-yl)-méthyl]-amino}-3,4-dioxo-cyclobut-1-enylamino)-benzoyl]-pyrrolidine-2-(R)-carboxylate de méthyle
21/ (-)-6-chloro-2-hydroxy-N,N-diméthyl-3-(2-{[((S)-5-méthyl-furan-2-yl)-(tétrahydro-thiophen-2-yl)-méthyl]-amino}-3,4-dioxo-cyclobut-1-enylamino)-benzamide
22/ (-)-3-[4-chloro-2-hydroxy-3-(4-méthyl-pipérazine-1-sulfonyl)-phénylamino]-4-{[(5-méthyl-furan-2-yl)-(tétrahydro-thiophen-2-yl)-méthyl]-amino}-cyclobut-3-ène-1,2-dione

Un second objet selon l'invention concerne une composition pharmaceutique comprenant une quantité efficace d'un composé répondant à la formule générale (I) ou d'un sel pharmaceutiquement acceptable dudit composé tel que décrit ci-dessus en association avec un solvant ou un support pharmaceutiquement acceptable.

Un troisième objet selon l'invention concerne les composés répondant à la formule générale (I), ainsi que leurs sels, solvates ou hydrates pharmaceutiquement acceptables ou encore une composition pharmaceutique comprenant une quantité efficace d'un composé répondant à la formule générale (I), d'un de ses sels ou un de ses solvates ou hydrates pharmaceutiquement acceptables pour leur utilisation en tant que médicament.

Un quatrième objet selon l'invention concerne les composés répondant à la formule générale (I), ainsi que leurs sels, solvates ou hydrates pharmaceutiquement acceptables ou encore une composition pharmaceutique comprenant une quantité efficace d'un composé répondant à la formule générale (I), d'un de ses sels ou un de ses solvates ou hydrates pharmaceutiquement acceptables pour leur utilisation dans le traitement de maladies médiées par les α-chimiokines.

Un cinquième objet selon l'invention concerne une méthode de traitement des maladies médiées par les α-chimiokines à l'aide d'un composé répondant à la formule générale (I) ainsi que ses sels, solvates ou hydrates pharmaceutiquement acceptables ou encore une composition pharmaceutique comprenant une quantité efficace d'un composé répondant à la formule générale (I), d'un de ses sels ou un de ses solvates ou hydrates pharmaceutiquement acceptables.

A titre d'exemples de maladies médiées par les α- chimiokines, on peut citer les dermatoses neutrophiliques, notamment le psoriasis, les dermatites atopiques, l'acné, la rosacée, l'asthme, les maladies pulmonaires obstructives chroniques, les maladies respiratoires des adultes, l'arthrite, les maladies intestinales inflammatoires, la maladie de Crohn, le rejet de greffe, la mucoviscidose et les cancers cutanés.

Par dermatoses neutrophiliques, on entend dans son sens le plus large, le syndrome de Sweet, « eccrines hydranite », le syndrome SAPHO, le syndrome de Sneddon Wilkinson, le pyoderma gangrenosum, l'érythème elevatum duitinum, le psoriasis, le psoriasis vulgaire, le psoriasis pustuleux, la pustulose palmo-plantaire, la pustulose exanthématique (PEAG), la pustulose vascularite, l'acro-pustulose de l'enfant, la maladie de Behcet, ainsi que certains maladies bulleuses comme l'herpès dérivés sous forme de dermatite, la dermatose neutrophilique à IgA, IgA intra-épiderme pustilosis, la pemphigoïde bulleuse, le pemphigus à IgA, la vascularite, le syndrome de Leroy Reiter Fiellinger, la pustulose du cuir chevelu, l'acrodermatite continue d' Hallopeau et les dermatoses liées à l'angio-immunoblastique lymphodénopathie, avec dysmielopoësis induite par le cyclophosphamide, avec des p-ANCA anticorps.

Dans un mode de réalisation préféré selon l'invention, le composé ou la composition pharmaceutique precitée est utilisé dans le traitement de maladies dermatologiques telles que les dermatoses neutrophiliques, notamment le psoriasis, les dermatites atopiques, l'acné et la rosacée.

Un autre aspect de l'invention concerne l'utilisation d'un composé répondant à la formule générale (I), ainsi que leurs sels, solvates ou hydrates pharmaceutiquement acceptable ou encore l'utilisation d'une composition pharmaceutique comprenant une quantité efficace d'un composé répondant à la formule générale (I), d'un de ses sels ou un de ses solvates ou hydrates pharmaceutiquement acceptable pour la préparation d'un médicament pour le traitement des maladies du groupe comprenant les dermatoses neutrophiliques, notamment le psoriasis, les dermatites atopiques, l'acné, la rosacée, l'asthme, les maladies pulmonaires obstructives chroniques, les maladies respiratoires des adultes, l'arthrite, les maladies intestinales inflammatoires, la maladie de Crohn et les cancers cutanés.

Les composés de formule générale (I) de la présente invention sont préparés suivant une ou plusieurs des voies de synthèse telles que décrites ci-dessous ou telles qu'elles ressortent des différents exemples de préparation donnés ci-après de façon non limitative.

La voie de synthèse générale pour la préparation des composés de formule (III) est illustrée sur le **schéma 1**. Le traitement séquentiel des intermédiaires alkyles squarates (A) avec les amines R'2-NH₂ et R'3-NH₂ donne les composés de formule (III). Dans la formule (A), R' 1 est un alkyle C1-C6, de préférence méthyle ou éthyle. La réaction est effectuée dans un solvant inerte et polaire (ou dans un mélange de solvants) comme l'éthanol, le méthanol, le diméthylsulfoxide, le diméthylformamide ou l'acétonitrile. Les amines R'2-NH₂ et R'3-NH₂ peuvent être utilisées comme bases libres ou sous forme de sels. Les réactions peuvent être conduites en présence d'une base convenable comme la triéthylamine, la diisopropyléthylamine, le carbonate de sodium ou le carbonate de potassium et à 25 °C ou de préférence à des températures élevées de 50-80 °C. Le temps de réaction est généralement compris entre 1 heure et 72 heures pour avoir une conversion complète.

Les amines R'3-NH₂ de formule (IX) sont préparées selon le **schéma 2** à partir de réactifs commerciaux en utilisant des méthodes bien connues de l'homme du métier décrites dans les manuels de synthèse organique comme par exemple « Comprehensive Organic Functional Group Transformation» Vol. 1-7 A.R.Katritzky, O. Meth-Cohn, C.W.Rees, Pergamon Press, 1998.
Les alcools primaires (IV) [dans lesquels X et R ont la même signification que X et R5 respectivement ci-dessus pour les composés de formule générale (I)] sont oxydés en aldéhydes de formule (V) dans les conditions de Swern (Mancuso, A. J.; Huang, S.-L.; Swern, D. (1978). "Oxidation of long-chain and related alcohols to carbonyls by dimethyl sulfoxide "activated" by oxalyl chloride" J. Org. Chem. 43 (12), 2480-2482) ou avec du chlorochromate de pyridinium.
L'aldéhyde de formule (V) est successivement traité avec un réactif de Grignard aryle ou hétéroaryle ou avec un dérivé lithié pour donner un alcool secondaire de formule (VI). Les azotures correspondants (VII) sont préparés à partir des alcools (VI) soit en les transformant en mésylates (VIII) qui sont ensuite traités avec les azotures métalliques (par exemple azoture de sodium), soit en les transformant directement en azoture après traitement avec l'azoture de diphénylphosphoryle (DPPA). L'azoture (VII) est finalement réduit en amine (IX) correspondante par de l'hydrogène en présence de différents catalyseurs (par exemple, palladium sur charbon actif) ou par traitement avec la triphénylphosphine suivi par hydrolyse des intermédiaires imidophosphoranes (Gololobov, Y. G. (1981), "Sixty years of staudinger reaction", Tetrahedron 37 (3), 437).

Alternativement, les amines primaires R'3-NH2 de formule (IX) peuvent être préparées selon le **schéma 3** à partir d'acides commerciaux (X) [dans lesquels X et R ont la même signification que X et R5 respectivement ci-dessus pour les composés de formule générale (I)], en les convertissant en amides de Weinreb (XI) (Nahm, S.; Weinreb, S. M. (1981), "N-methoxy-n-methylamides as effective acylating agents", Tetrahedron Letters 22, 3815), qui après réaction avec les réactifs de Grignard aryle ou hétéroaryle ou avec des dérivés lithiés aryle ou hétéroaryle donnent les cétones (XII) qui peuvent être réduites en alcools secondaires (VI).
En suivant les étapes décrites dans le schéma 2, l'alcool (VI) est éventuellement transformé en amine R'3-NH2 de formule (IX).

L'amine primaire chirale R'3-NH2 de structure (XV) peut également être préparée selon le **schéma 4** par condensation du 2-méthyl-2-propanesulfinamide énantiomériquement pur (*tert-*butanesulfinamide, Elman's sulfinamide: Liu, G. et al. J. Am. Soc. Chem. 1997, 119, 9913) avec l'aldéhyde (IV) dans des conditions douces. Cette réaction fournit les *tert*-butanesulfinyl imines (XIII).Le groupement *tert*-butanesulfinyle active les imines pour l'addition des réactifs de Grignard et sert de groupement directeur chiral important pour donner les produits (XIV) avec une diastéréosélectivité élevée. La déprotection du groupement *tert*-butanesulfinyle dans des conditions acides douces donne l'amine chirale (XV).

Les dérivés amide de l'acide 3-aminosalicylique de formule (XVIII) sont préparés selon le **schéma 5a**/ à partir de l'acide 3-nitrosalicylique (XVI) en utilisant des conditions de couplage peptidique standard *(*Recent development of peptide coupling reagents in organic synthesis Tetrahedron, Volume 60(11), 2447-2467, Han, S.-Y.;Kim, Y.-A. *)*, suivi d'une réduction du groupement nitro en groupement amino par de l'hydrogène en présence d'un catalyseur approprié (par exemple palladium sur charbon actif). Le dérivé (XVIII) réagit ensuite avec le diméthoxysquarate ou diéthoxysquarate commercial pour donner l'intermédiaire (XIX), lequel est transformé en composé (XX) après réaction avec l'amine primaire R'3-NH2.

Alternativement, le couplage de l'acide 3-aminosalicylique (XXI) avec le diméthoxysquarate ou diéthoxysquarate commercial donne, selon le **schéma 5b**/, le dérivé acide intermédiaire (XXII) qui, après réaction avec l'amine primaire R'3-NH2, peut donner le composé (XXIII). Ce dernier peut enfin être engagé dans une réaction de couplage peptidique avec une amine de formule RaRbNH pour conduire au composé de formule (XX).

A titre d'illustration, les composés suivants répondant à la formule générale (I) de la présente invention ont été préparés en suivant l'un des schémas présentés ci-dessus.

### EXEMPLE 1

### Préparation du 2-hydroxy-N,N-diméthyl-3-(2-{[(5-méthyl-furan-2-yl)-(2-méthyl-tétrahydrofuran-2-yl)-méthyl]-amino}-3,4-dioxo-cyclobut-1-enylamino)-benzamide

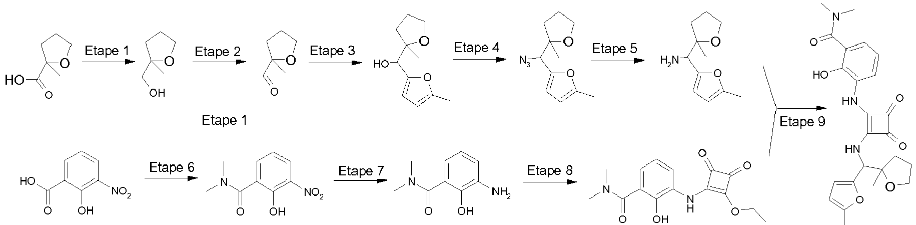

### Etape 1:

### (2-Méthyl-tétrahydrofuran-2-yl)-méthanol

19.94 g (0.145 mol, 1 éq) d'acide 2-méthyltétrahydrofurane-2-carboxylique (commercial) à 95% en solution dans 100 mL d'éther diéthylique ont été ajoutés goutte à goutte sur une suspension refroidie à 10°C de 16.6 g (0.438 mol, 3 éq) d'hydrure de lithium et d'aluminium dans 100 mL d'éther diéthylique. Le milieu réactionnel a été agité à température ambiante pendant 24 heures. Le milieu réactionnel a été refroidi et de l'eau a été ajoutée goutte à goutte suivi d'une solution saturée de chlorure d'ammonium. Le milieu a été extrait à l'éther diéthylique. Les phases organiques ont été rassemblées, lavées à l'eau, séchées sur sulfate de magnésium, filtrées et évaporées. 14.45 g de (2-méthyl-tétrahydrofuran-2-yl)-méthanol ont été obtenus. Rendement = 86%. CCM/SiO₂ : CH₂Cl₂/MeOH (95/5), révélation au KMnO₄.

### Etape 2:

### 2-Méthyl-tétrahydrofuran-2-carbaldéhyde

Une solution de 14.44 g (0.124 mol, 1 éq) de (2-méthyl-tétrahydrofuran-2-yl)-méthanol dans 140 mL de dichlorométhane a été ajoutée goutte à goutte sur un mélange de 43.0 g (0.20 mol, 1.6 éq) de chlorochromate de pyridinium dans 400 mL de dichlorométhane. 15 g de celite ont été ajoutés et le milieu réactionnel a été agité à température ambiante pendant 7 heures. Le milieu réactionnel a été filtré sur 280 g de silice et élué au dichlorométhane (4.5 L). 6.0 g de 2-méthyl-tétrahydrofuran-2-carbaldéhyde ont été obtenus sous forme d'un liquide jaune (1^{ère} fraction à 77%). 8.4 g de 2-méthyl-tétrahydrofuran-2-carbaldéhyde ont été obtenus sous forme d'un liquide orange (2^{ème} fraction à 54%). Rendement = 65%. CCM/SiO₂ : Heptane/AcOEt (40/60), révélation au KMnO₄

### Etape 3:

### (5-Méthyl-furan-2-yl)-(2-méthyl-tétrahydrofuran-2-yl)-méthanol

24 mL (60 mmol, 1.5 éq) d'une solution de n-butyllithium 2.5 M dans l'hexane ont été ajoutés goutte à goutte sur une solution de 5.0 g (60 mmol, 1.5 éq) de 2-méthylfurane dans 100 mL de tétrahydrofuranne refroidie à -70°C. Le milieu réactionnel a été agité et laissé remonter à température ambiante pendant 2 heures. Le milieu réactionnel a été refroidi à -70°C puis 6.0 g (40 mmol, 1 éq) de 2-méthyl-tétrahydrofuran-2-carbaldéhyde à 77% ont été ajoutés. Le milieu réactionnel a été agité à température ambiante pendant 3 heures. Le milieu réactionnel a été traité avec une solution saturée de chlorure d'ammonium et extrait à l'acétate d'éthyle. Les phases organiques ont été rassemblées, lavées avec une solution saturée de chlorure de sodium, filtrées sur sulfate de magnésium et évaporées. 5.21 g de (5-méthyl-furan-2-yl)-(2-méthyl-tétrahydrofuran-2-yl)-méthanol ont été obtenus. Rendement = 66%. CCM/SiO₂: Heptane/AcOEt (60/40), révélation au KMnO₄

### Etape 4:

### 2-[Azido-(2-méthyl-tétrahydrofuran-2 yl)-méthyl]-5-méthyl-furan

8.77 g (31.8 mmol, 1.2 éq) de diphénylphosphoryl azide ont été ajoutés goutte à goutte sur une solution de 5.21 g (26.5 mmol, 1 éq) de (5-méthyl-furan-2-yl)-(2-méthyl-tétrahydrofuran-2-yl)-méthanol dans 90 mL de toluène. Le milieu réactionnel a été refroidi à 0°C puis 4.75 mL (31.8 mmol, 1.2 éq) de 1,8-diazabicyclo [5.4.0] undec-7-ene ont été ajoutés goutte à goutte. Le milieu réactionnel a été agité à température ambiante pendant 41 heures. Le milieu réactionnel (hétérogène) a été traité à l'eau et à l'acétate d'éthyle puis décanté. La phase organique a été lavée à l'acide chlorhydrique 1N, séchée sur sulfate de magnésium, filtrée et évaporée. Le résidu a été chromatographié sur gel de silice HP (colonne puriFlash IR50SI-200G, spot II) élué à l'heptane/acétate d'éthyle (95/5). 2.15 g de 2-[azido-(2-méthyl-tétrahydrofuran-2-yl)-méthyl]-5-méthyl-furan ont été obtenus. Rendement = 37%. CCM/SiO₂ : Heptane/AcOEt (80/20), révélation au KMnO₄.

### Etape 5:

### C-(5-Methyl-furan-2-yl)-C-(2-methyl-tétrahydrofuran-2-yl)-méthylamine

Une solution de 2.51 g (9.7 mmol, 1 éq) de 2-[azido-(2-méthyl-tétrahydrofuran-2-yl)-méthyl]-5-méthyl-furan dans 45 mL d'éthanol a été agitée sous pression atmosphérique d'hydrogène en présence de 323 mg (15% en masse) de palladium sur charbon (Pd/C) à 10% pendant 16 heures. Le milieu réactionnel a été filtré et le filtrat a été évaporé. 1.82 g de C-(5-méthyl-furan-2-yl)-C-(2-méthyl-tétrahydrofuran-2-yl)-méthylamine ont été obtenus. Rendement = 96%. CCM/SiO₂ : Heptane/AcOEt (60/40), révélation au KMnO₄.

### Etape 6 :

### 2-Hydroxy-N,N-diméthyl-3-nitro-benzamide

42.9 mL (0.50 mol, 3 éq) de chlorure d'oxalyle ont été ajoutés goutte à goutte sur une suspension de 30 g (0.16 mol, 1 éq) d'acide 3-nitrosalicylique dans 1200 mL de dichlorométhane. 30 gouttes de N,N-diméthylformamide ont été ajoutées (fort dégagement de gaz, adaptation d'un système de piégeage de vapeurs de monoxyde de carbone toxique). Le milieu réactionnel a été agité à température ambiante pendant 24 heures. Le milieu réactionnel a été refroidi à 0-5°C puis 246 mL (0.49 mol, 3 éq) d'une solution de diméthylamine dans le tétrahydrofurane 2N ont été ajoutés. Le milieu réactionnel a été agité à température ambiante pendant 2 jours. Le milieu réactionnel a été concentré à sec et le résidu a été mis en solution dans 300 mL de soude 1N. La solution aqueuse (rouge) a été extraite 3 fois avec 300 mL de dichlorométhane. La phase aqueuse a été refroidie dans un bain d'eau-glace, et le pH a été ajusté à 2 avec environ 50 mL d'acide chlorhydrique 6N. Le mélange (devenu jaune) a été extrait 3 fois avec 300 mL de dichlorométhane. Les phases organiques ont été rassemblées, lavées 2 fois avec 250 mL d'eau puis une fois avec 250 mL d'une solution saturée de chlorure de sodium, séchées sur sulfate de magnésium anhydre et évaporées. 33.5 g de 2-hydroxy-N,N-diméthyl-3-nitro-benzamide ont été obtenus sous forme d'un solide cotoneux jaune. Rendement = 97%.

### Etape 7:

### 3-Amino-2-hydroxy-N,N-diméthyl-benzamide

Une solution de 33.5 g de 2-hydroxy-N,N-diméthyl-3-nitro-benzamide dans 600 mL d'éthanol a été ajoutée sur une suspension de 3.35 g de Pd/C 10% dans 70 mL d'éthanol. Le milieu réactionnel a été agité sous 2 bars d'hydrogène pendant la nuit. Contrôle CCM et HPLC (t=0.66 M+181). Le milieu réactionnel a été filtré sur célite et le filtrat a été évaporé. 29 g de 3-amino-2-hydroxy-N,N-diméthyl-benzamide ont été obtenus sous forme d'un solide marron huileux. Rendement = 100%.

### Etape 8:

### 3-(2-Ethoxy-3,4-dioxo-cyclobut-1-enylamino)-2-hydroxy-N,N-diméthyl-benzamide

Sous azote et à température ambiante, 39.7 g de diéthoxysquarate ont été ajoutés (en 15 minutes) sur une solution de 28 g de 3-amino-2-hydroxy-N,N-diméthyl-benzamide dans 840 mL d'éthanol refroidie à 0°C. Le milieu réactionnel a été agité pendant 2 heures à 0°C et 48 heures à température ambiante. 700 mL d'éthanol ont été rajoutés (ce qui augmente la précipitation du produit attendu). Le solide a été filtré, lavé à l'éthanol ambiant et séché. 36.9 g de 3-(2-éthoxy-3,4-dioxo-cyclobut-1-enylamino)-2-hydroxy-N,N-diméthyl-benzamide ont été obtenus sous forme d'un solide vert kaki clair. Rendement = 78%.

### Etape 9:

### 2-Hydroxy-N,N-diméthyl-3-(2-{[(5-méthyl-furan-2-yl)-(2-méthyl-tétrahydrofuran-2-yl)-méthyl]-amino}-3,4-dioxo-cyclobut-1-enylamino)-benzamide (diastéréoisomères 1 et 2)

1.82 g (9.3 mmol, 1.5 éq) de C-(5-méthyl-furan-2-yl)-C-(2-méthyl-tétrahydrofuran-2-yl)-méthylamine ont été ajoutés sur 1.89 g (6.2 mmol, 1 éq) de 3-(2-éthoxy-3,4-dioxo-cyclobut-1-enylamino)-2-hydroxy-N,N-diméthyl-benzamide solubilisés à chaud dans 100 mL de méthanol. Le milieu réactionnel a été chauffé à 60°C pendant 3 heures et demie. Le méthanol a été évaporé et le résidu a été chromatographié sur gel de silice (colonne puriFlash IR50SI-200G, spot II) élué au dichlorométhane/méthanol (gradient).
Diastéréoisomère 1, 1.00 g du 2-hydroxy-N,N-diméthyl-3-(2-{[(5-méthyl-furan-2-yl)-(2-méthyl-tétrahydrofuran-2-yl)-méthyl]-amino}-3,4-dioxo-cyclobut-1-enylamino)-benzamide a été obtenu sous forme d'un solide beige (Tf = 127-129°C). LC/MS : 98.41% [453].
RMN ¹H (DMSO, 400 MHz)_: 1.22 (s, 3H) ; 1.57-1.62 (m, 1H) ; 1.67-1.74 (m, 1H) ; 1.82-1.89 (m, 1H) ; 1.95-1.99 (m, 1H) ; 2.28 (s, 3H) ; 2.94 (s, 6H) ; 3.6 (q, J = 6.7 Hz, 1H) ; 3.8 (q, J = 7.5 Hz, 1H) ; 5.3 (d, J = 10 Hz, 1H) ; 6.06 (d, J = 3.0 Hz, 1H), 6.25 (dd, J = 3.0 Hz, 1H) ; 6.87 (m, 2H) ; 7.76 (dd, J = 6.7 Hz, 1H) ; 8.93 (d, J = 10.0 Hz, 1H) ; 9.56 (s, 1H) ; 9.92 (s, 1H).
Diastéréoisomère 2, 1.03 g du 2-hydroxy-N,N-diméthyl-3-(2-{[(5-méthyl-furan-2-yl)-(2-méthyl-tétrahydrofuran-2-yl)-méthyl]-amino}-3,4-dioxo-cyclobut-1-enylamino)-benzamide ont été obtenus. (Tf = 127-129°C), LC/MS : 97.76% [453].
RMN ¹H (DMSO-d6, 400 MHz)_: 1.16 (s, 3H) ; 1.65-1.70 (m, 1H) ; 1.84-1.98 (m, 3H) ; 2.28 (s, 3H) ; 2.94 (s, 6H) ; 3.76-3.80 (m, 2H) ; 5.3 (d, J = 9.9 Hz, 1H) ; 6.06 (dd, J = 2.9 Hz, 1H), 6.28 (d, J = 3.0 Hz, 1H) ; 6.85-6.91 (m, 2H) ; 7.75 (dd, J = 6.9 Hz, 1H) ; 8.85 (d, J = 10.0 Hz, 1H) ; 9.54 (s, 1H) ; 9.95 (s, 1H).

### 2-Hydroxy-N,N-diméthyl-3-(2-{[(5-méthyl-furan-2-yl)-(2-méthyl-tétrahydrofuran-2-yl)-méthyl]-amino}-3,4-dioxo-cyclobut-1-enylamino)-benzamide (énantiomères 1 et 2 de

### diastéreoisomère 2)

La séparation du diastéréoisomère 2 en énantiomères 1 et 2 a été effectuée sur la colonne chirale CHIRALCEL^{R} OD-H 5 µm - 250 x 4.6 mm, la phase mobile : dioxyde de carbone/méthanol (80/20), débit de 120 mL/min.
Enantiomère 1 du diastéréoisomère 2: temps de rétention à 3.91 min
Enantiomère 2 du diastéréoisomère 2: temps de rétention à 5.08 min

### EXEMPLE 2:

### Préparation du 2-hydroxy-N,N-diméthyl-3-(2-{[(5-méthyl-furan-2-yl)-(tétrahydro-thiophèn-2-yl)-méthyl]-amino}-3,4-dioxo-cyclobut-1-enylamino)-benzamide

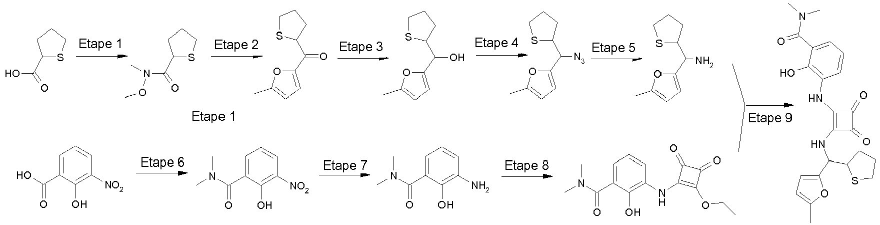

### Etape 1:

### Méthoxy-méthyl-tétrahydro-thiophène-2-carboxylamide

A une solution de 46.0 g (0.35 mol; 1.0 éq) d'acide tétrahydro-thiophène-2-carboxylique (commercial) dans 200 mL de dichlorométhane ont été rajoutés goutte à goutte 32.0 mL (0.44 mol; 1.27 éq) de chlorure de thionyle à température ambiante en 15 minutes. Le milieu réactionnel a été agité à température ambiante pendant 3 heures jusqu'à ce qu'il n'y ait plus de dégagement gazeux. Le dichlorométhane et l'excès de chlorure de thionyle ont été évaporés sous vide et le résidu a été coévaporé trois fois avec 100 mL de toluène. Le chlorure d'acide obtenu a été solubilisé dans 200 mL de dichlorométhane et 37.34 g (0.38 mol; 1.1 éq) de chlorhydrate de *N*,*O*-diméthylhydroxylamine ont été ajoutés. Le milieu réactionnel a été refroidi à -10°C et un mélange de 116 mL (0.84 mol; 2.4 éq) de triéthylamine dans 100 mL de dichlorométhane a été additionné goutte à goutte en une heure (en maintenant la température en dessous de 5°C). Après l'addition, le milieu réactionnel a été agité à température ambiante pendant une heure puis a été lavé avec 250 mL d'une solution aqueuse d'acide chlorhydrique à 1M. La phase aqueuse a été extraite au dichlorométhane. Les phases organiques ont été rassemblées, lavées avec 200 mL d'une solution aqueuse d'hydrogénophosphate de sodium 1M, séchées sur sulfate de magnésium anhydre, filtrées et évaporées. 51.0 g de méthoxy-méthyl-tétrahydro-thiophène-2-carboxylamide ont été obtenus sous forme d'une huile orange. Rendement = 84%.

### Etape 2:

### (5-Méthyl-furan-2-yl)-(tétrahydro-thiophèn-2-yl)-methanone

176 mL (440.8 mmol; 1.50 éq) de *n*-butyllithium à 2.5 M dans l'hexane ont été ajoutés goutte à goutte sur une solution de 39.8 mL (440.8 mmol; 1.50 éq) de 2-méthyl-furane dans 1 L de tétrahydrofuranne refroidie à -78°C. Le mélange a été laissé remonter à température ambiante pendant 2 heures puis a été refroidi à -78°C. Une solution de 51.00 g (291 mmol; 1.00 éq) de méthoxy-méthyl-tétrahydro-thiophène-2-carboxylamide dans 400 mL de tétrahydrofuranne a été additionnée et le mélange réactionnel a été laissé à 0°C pendant 2 heures. Le milieu réactionnel a été dilué avec 500 mL d'acétate d'éthyle puis lavé avec 1 L d'une solution aqueuse d'acide chlorhydrique 1N. La phase aqueuse a été séparée et extraite avec 500 mL d'acétate d'éthyle. Les phases organiques ont été rassemblées, lavées avec une solution saturée de chlorure de sodium, séchées sur sulfate de magnésium anhydre, filtrées et évaporées. L'huile brune obtenue a été filtrée sur silice (éluent : Heptane/AcOEt 90/10). 49.67 g de (5-méthyl-furan-2-yl)-(tétrahydro-thiophèn-2-yl)-méthanone ont été obtenus. Rendement = 87%.

### Etape 3:

### (5-Méthyl-furan-2-yl)-(tétrahydro-thiophèn-2-yl)-méthanol

6.69 g (0.18 mol; 1.20 éq) de borohydrure de sodium ont été ajoutés par petites portions sur une solution de 30.24 g (0.15 mol; 1.0 éq) de (5-méthyl-furan-2-yl)-(tétrahydro-thiophèn-2-yl)-méthanone dans 300 mL de tétrahydrofurane et 50 mL de méthanol refroidie à 0°C. Le milieu réactionnel a été agité à température ambiante pendant 3 heures. Le milieu réactionnel a été versé dans 400 mL d'acétate d'éthyle puis 200 mL d'eau ont été rajoutés. La phase aqueuse a été extraite à l'acétate d'éthyle puis les phases organiques ont été rassemblées, lavées avec une solution de chlorure de sodium saturée, séchées sur sulfate de magnésium anhydre, filtrées et évaporées. 30.22 g de (5-méthyl-furan-2-yl)-(tetrahydro-thiophen-2-yl)-méthanol ont été obtenus. Rendement quantitatif

### Etape 4 :

### 2-[Azido-(tétrahydro-thiophèn-2-yl)-méthyl]-5-méthyl-furane

A une solution de 30.21 g (0.15 mol; 1.0 éq) de (5-méthyl-furan-2-yl)-(tétrahydro-thiophen-2-yl)-méthanol dans 350 mL de toluène refroidie à 0°C ont été ajoutés 39.3 mL (0.18 mol; 1.2 éq) de diphénylphosphoryl azide puis 27.3 mL (0.18 mol; 1.2 éq) de 1,8-diazabicyclo[5.4.0]undec-7-ene goutte à goutte. Le mélange a été laissé revenir à température ambiante doucement puis a été agité pendant 2 jours. Le milieu réactionnel a été traité à l'eau et extrait à l'acétate d'éthyle. Les phases organiques ont été rassemblées, lavées avec une solution d'hydrogénophosphate de sodium 1M, séchées sur sulfate de magnésium, filtrées et évaporées. Le résidu a été chromatographié sur gel de silice (colonne puriFlash IR-50SI/800G, puriFlash) élué à l'heptane/acétate d'éthyle (95/5). 26.95 g de 2-[azido-(tétrahydro-thiophèn-2-yl)-méthyl]-5-méthyl-furane ont été obtenus (mélange des 2 diastéréoisomères). Rendement = 79%

### Etape 5 :

### C-(5-Méthyl-furan-2-yl)-C-(tétrahydro-thiophèn-2-yl)-méthylamine

Une solution de 26.95 g (0.12 mol; 1.0 éq) de 2-[azido-(tétrahydro-thiophèn-2-yl)-méthyl]-5-méthyl-furane dans 540 mL d'éthanol et en présence de 6.74 g (25% en masse) de Pd/C 10% a été agitée à température ambiante sous pression atmosphérique d'hydrogène pendant 2 jours. Le milieu réactionnel a été filtré et le filtrat a été évaporé. Le résidu a été chromatographié sur gel de silice (colonne puriFlash IR-50SI-STD/800G, puriFlash) élué au dichlorométhane/acétate d'éthyle (gradient).
Diastéréoisomère 1 : 5.52 g de C-[(R)-C-(5-méthyl-furan-2-yl)-C-tetrahydro-thiophen-2-yl]-méthylamine ont été obtenus. Rendement = 22%.
Diastéréoisomère 2 : 13.30 g de C-[(R)-C-(5-méthyl-furan-2-yl)-C-tetrahydro-thiophen-2-yl]-méthylamine ont été obtenus. Rendement = 54%.

### Etapes 6 à 8:

De manière analogue à l'EXEMPLE 1 (étapes 6 à 8), le 3-(2-éthoxy-3,4-dioxo-cyclobut-1-énylamino)-2-hydroxy-*N*,*N-*diméthyl-benzamide a été préparé.

### Etape 9:

### 2-Hydroxy-N,N-diméthyl-3-(2-{[(5-méthyl-furan-2-yl)-(tétrahydro-thiophèn-2-yl)-méthyl]-amino}-3,4-dioxo-cyclobut-1-énylamino)-benzamide (diastéréoisomère 1)

Un mélange de 694 mg (2.28 mmol; 1.0 éq) de 3-(2-éthoxy-3,4-dioxo-cyclobut-1-énylamino)-2-hydroxy-*N*,*N*-diméthyl-benzamide et de 540 mg (2.74 mmol; 1.2 éq) de C-(5-méthyl-furan-2-yl)-C-(tétrahydro-thiophèn-2-yl)-méthylamine (diastéréoisomère 1) en solution dans 20 mL de méthanol a été agité à température ambiante pendant 4 jours. L'insoluble a été filtré et séché sous vide à 50°C. 560 mg de 2-hydroxy-*N*,*N*-diméthyl-3-(2-{[(5-méthyl-furan-2-yl)-(tétrahydro-thiophèn-2-yl)-méthyl]-amino}-3,4-dioxo-cyclobut-1-énylamino)-benzamide (diastéréoisomère 1) ont été obtenus. Rendement = 54%.
RMN ¹H (DMSO-d6, 400 MHz) : 1.63-1.68 (m, 1H), 1.88-1.92 (m, 1H), 1.98-2.04 (m, 2H), 2.26 (s, 3H), 2.81 (t, J = 5.9 Hz, 2H), 2.94 (s, 6H), 3.97 (q, J = 6.6 Hz, 1H), 5.41 (q, J = 6.8-2.7 Hz, 1H), 6.06 (dd, J = 0.9-3.0 Hz, 1H), 6.26 (d, J = 3.1 Hz, 1H), 6.86-6.91 (m, 2H), 7.75 (dd, J = 2.6-7.0 Hz, 1H), 8.82 (d, J = 9.6 Hz, 1H), 9.51 (s, 1H), 9.94 (s, 1H).

### 2-Hydroxy-N,N-diméthyl-3-(2-{[(5-méthyl-furan-2-yl)-(tétrahydro-thiophèn-2-yl)-méthyl]-amino}-3,4-dioxo-cyclobut-1-énylamino)-benzamide (diastéréoisomère 2)

Un mélange de 1.29 g (4.22 mmol; 1.0 éq) de 3-(2-éthoxy-3,4-dioxo-cyclobut-1-énylamino)-2-hydroxy-*N*,*N-*diméthyl-benzamide et de 1.0 g (5.07 mmol; 1.2 éq) de C-(5-méthyl-furan-2-yl)-C-(tétrahydro-thiophèn-2-yl)-méthylamine (diastéréoisomère 2) en solution dans 40 mL de méthanol a été agité à température ambiante pendant 2 jours et demi. L'insoluble a été filtré et séché sous vide à 45°C. 1.48 g de 2-hydroxy-*N*,*N-*diméthyl-3-(2-{[(5-méthyl-furan-2-yl)-(tétrahydro-thiophèn-2-yl)-méthyl]-amino}-3,4-dioxo-cyclobut-1-énylamino)-benzamide (diastéréoisomère 2) ont été obtenus. Rendement = 77%.
RMN ¹H (DMSO-d6, 400 MHz) : 1.82-1.86 (m, 2H), 1.90-1.93 (m, 2H), 2.26 (s, 3H), 2.75-2.84 (m, 2H), 2.93 (s, 6H), 3.86 (m, 1H), 5.2 (t, J = 9.7 Hz, 1H), 6.06 (dd, J = 1.0-3.0 Hz, 1H), 6.30 (d, J = 3.1 Hz, 1H), 6.84-6.90 (m, 2H), 7.79 (dd, J = 2.3-7.2 Hz, 1H), 8.77 (d, J = 9.6 Hz, 1H), 9.34 (s, 1H), 9.94 (s, 1H).

### 2-Hydroxy-N,N-diméthyl-3-(2-{[(5-méthyl-furan-2-yl)-(tétrahydro-thiophèn-2-yl)-méthyl]-amino}-3,4-dioxo-cyclobut-1-énylamino)-benzamide (énantiomères 1 et 2)

La séparation du diastéréoisomère 1 en énantiomères a été effectuée sur la colonne chirale CHIRALPACK^{R} IC µm, phase mobile: dioxyde de carbone/éthanol (100/0.5), débit de 120 mL/min.
Enantiomère 1 du diastéréoisomère 1: temps de rétention à 6.1 min
Enantiomère 2 du diastéréoisomère 1: temps de rétention à 8.0 min

### 2-Hydroxy-N,N-diméthyl-3-(2-{[(5-méthyl-furan-2-yl)-(tétrahydro-thiophèn-2-yl)-méthyl]-amino}-3,4-dioxo-cyclobut-1-énylamino)-benzamide (énantiomères 1 et 2)

La séparation du diastéréoisomère 2 en énantiomères a été effectuée sur la colonne chirale CHIRALPACK^{R}ADH 5 µm, phase mobile: heptane/éthanol (60/40), débit de 42.5 mL/min.
Enantiomère 1 du diastéréoisomère 2: temps de rétention à 3.6 min
Enantiomère 2 du diastéréoisomère 2: temps de rétention à 4.7 min

### EXEMPLE 3

### Préparation du (S)-1-[2-fluoro-3-(2-{[(5-méthyl-furan-2-yl)-(tétrahydro-thiophèn-2-yl)-méthyl]-amino}-3,4-dioxo-cyclobut-1-énylamino)-benzoyl]-pyrrolidine-2-carboxylate de méthyle

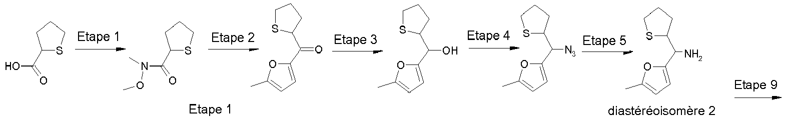

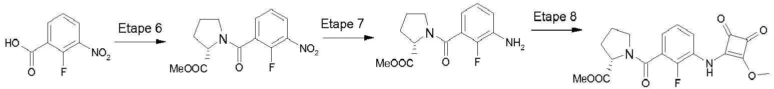

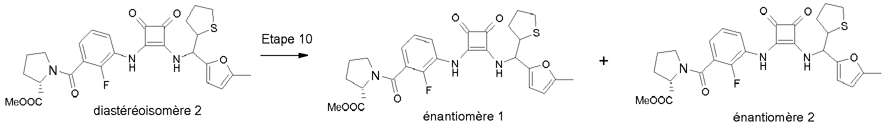

### Etapes 1 à 5:

De manière analogue à l'EXEMPLE 2 (étapes 1 à 5), C-(5-méthyl-furan-2-yl)-C-(tétrahydro-thiophèn-2-yl)-méthylamine (diastéréoisomère 2) a été préparé.

### Etape 6:

### (S)-1-(2-Fluoro-3-nitro-benzoyl)-pyrrolidine-2-carboxylate de méthyle

Un mélange de 18.51 g (0.10 mol; 1.0 éq) d'acide 2-fluoro-3-nitro-benzoïque et de 100 mL de chlorure de thionyle a été chauffé à reflux pendant 3 heures. L'excès de chlorure de thionyle a été ensuite concentré et le résidu a été coévaporé deux fois avec du toluène. Le chlorure d'acide ainsi obtenu a été repris dans 250 mL de dichlorométhane. A ce mélange refroidi à 0°C ont été rajoutés 16.56 g (0.10 mol; 1.0 éq) de chlorhydrate de l'ester méthylique de la L-proline puis 30.50 mL (0.22 mol; 2.2 éq) de triéthylamine. Après 30 minutes à 0°C et une heure à température ambiante, le milieu réactionnel a été dilué et lavé avec 250 mL d'une solution aqueuse d'acide chlorhydrique 1M puis avec 250 mL d'une solution aqueuse saturée d'hydrogénocarbonate de sodium. La phase organique a été séchée sur sulfate de magnésium anhydre, filtrée et évaporée. 25.64 g de (S)-1-(2-fluoro-3-nitro-benzoyl)-pyrrolidine-2-carboxylate de méthyle ont été obtenus. Rendement = 87%.

### Etape 7:

### (S)-1-(3-Amino-2-fluoro-benzoyl)-pyrrolidine-2-carboxylate de méthyle,

Une solution de 25.0 g (0.08 mol; 1.0 éq) de (S)-1-(2-fluoro-3-nitro-benzoyl)-pyrrolidine-2-carboxylate de méthyle dans 250 mL de méthanol en présence de 7.0 g (28% en masse) de palladium sur charbon à 10% a été agitée sous atmosphère d'hydrogène à température ambiante pendant 4 jours. Le milieu réactionnel a été filtré sur célite et lavé avec 100 mL de méthanol. Le filtrat a été évaporé. 22.80 g de (S)-1-(3-amino-2-fluoro-benzoyl)-pyrrolidine-2-carboxylate de méthyle ont été obtenus sous forme d'une huile claire. Rendement quantitatif.

### Etape 8:

### (S)-1-[2-Fluoro-3-(2-méthoxy-3,4-dioxo-cyclobut-1-énylamino)-benzoyl]-pyrrolidine-2-carboxylate de méthyle

A une solution de 20.0 g (0.08 mol; 1.0 éq) de (S)-1-(3-amino-2-fluoro-benzoyl)-pyrrolidine-2-carboxylate de méthyle dans 150 mL de méthanol ont été rajoutés 21.35 g (0.15 mol; 2.0 éq) de 3,4-diméthoxy-3-cyclobutène-1,2-dione. Le milieu réactionnel a été chauffé à 50°C pendant 3 heures et concentré. Le résidu a été élué sur un gâteau de silice (de 15 cm de diamètre et de 10 cm de haut) avec 2 L d'heptane/acétate d'éthyle (2/1), 2 L d'heptane/acétate d'éthyle (1/2) et 2 L d'acétate d'éthyle/méthanol (95/5). 19 g d'un produit ont été obtenus. Ce produit a été purifié par chromatographie sur gel de silice élué avec de l'acétate d'éthyle. 15 g de (S)-1-[2-fluoro-3-(2-méthoxy-3,4-dioxo-cyclobut-1-énylamino)-benzoyl]-pyrrolidine-2-carboxylate de méthyle ont été obtenus. Rendement = 53%.

### Etape 9:

### (S)-1-[2-Fluoro-3-(2-{[(5-méthyl-furan-2-yl)-(tétrahydro-thiophèn-2-yl)-méthyl]-amino}-3,4-dioxo-cyclobut-1-énylamino)-benzoyl]pyrrolidine-2-carboxylate de méthyle

Un mélange de 1.02 g (2.70 mmol; 1.0 éq) de (S)-1-[2-fluoro-3-(2-méthoxy-3,4-dioxo-cyclobut-1-énylamino)-benzoyl]-pyrrolidine-2-carboxylate de méthyle et de 640 mg (3.24 mmol; 1.2 éq) de C-(5-méthyl-furan-2-yl)-C-(tétrahydro-thiophèn-2-yl)-méthylamine (diastéréoisomère 2) en solution dans 25 mL de méthanol a été agité à température ambiante pendant 3 jours et demi. Le milieu réactionnel a été évaporé et le résidu a été chromatographié sur gel de silice (colonne puriFlash IR-50SI/120G, spot II) élué au dichlorométhane/acétate d'éthyle (gradient). 1.01 g de (S)-1-[2-fluoro-3-(2-{[(5-méthyl-furan-2-yl)-(tétrahydro-thiophèn-2-yl)-méthyl]-amino}-3,4-dioxo-cyclobut-1-énylamino)-benzoyl]-pyrrolidine-2-carboxylate de méthyle (couple d'énantiomères 1 et 2) ont été obtenus. Rendement = 69%. RMN ¹H (DMSO-d6, 400 MHz) : 1.81-2.07 (m, 7H), 2.26 (s, 3H), 2.29 (m, 1H), 2.75-2.84 (m, 2H), 3.35-3.38 (m, 2H), 3.68 (s, 3H), 3.86-3.90 (m, 1H), 4.49-4.53 (m, 1H), 5.17 (t, J = 9.5 Hz, 1H), 6.07 (d, J = 2.9 Hz, 1H), 6.33 (d, J = 3.1 Hz, 1H), 7.02 (t, J = 6.6 Hz, 1H), 7.26 (t, J = 7.9 Hz, 1H), 8.05 (t, J = 7.9 Hz, 1H), 7.56 (d, J = 9.6 Hz, 1H), 9.64 (s, 1H).

### Etape 10:

### (S)-1-[2-Fluoro-3-(2-{[(5-méthyl-furan-2-yl)-(tétrahydro-thiophèn-2-yl)-méthyl]-amino}-3,4-dioxo-cyclobut-1-énylamino)-benzoyl]pyrrolidine-2-carboxylate de méthyle (énantiomère 1 et énantiomère 2)

Les énantiomères ont été séparés sur la colonne CHIRALPACK ADH 5 µm avec l'éluant dioxide de carbone / (éthanol + 1% diéthylamine) 85/15, débit de 120 mL/min.
Enantiomère 1: temps de rétention à 20.9 min
Enantiomère 2: temps de rétention à 33.7 min

### EXEMPLE 4

### Préparation du (S)-1-[2-fluoro-3-(2-{[(5-méthyl-furan-2-yl)-(tétrahydro-thiophen-2-yl)-méthyl]-amino}-3,4-dioxo-cyclobut-1-enylamino)-benzoyl]-pyrrolidine-2-carboxylate d'isopropyle

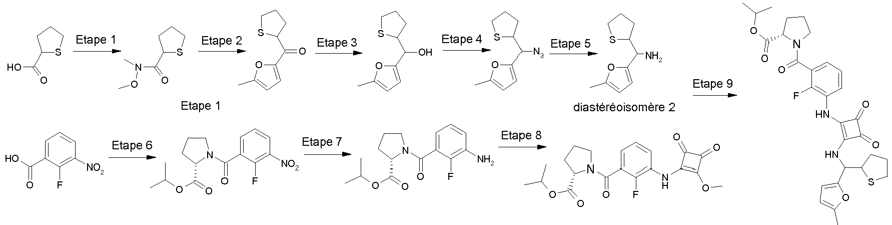

### Etapes 1 à 5:

De manière analogue à l'EXEMPLE 2 (étapes 1 à 5), C-(5-méthyl-furan-2-yl)-C-(tétrahydro-thiophèn-2-yl)-méthylamine (diastéréoisomère 2) a été préparé.

### Etape 6:

### (S)-1-(2-Fluoro-3-nitro-benzoyl) pyrrolidine-2-carboxylate d'isopropyle

Un mélange de 1.50 g (8.10 mmol; 1.0 éq) d'acide 2-fluoro-3-nitro-benzoïque et de 16 mL de chlorure de thionyle a été chauffé à reflux pendant 3 heures. L'excès de chlorure de thionyle a été ensuite concentré et le résidu a été coévaporé deux fois avec du toluène. Le chlorure d'acide ainsi obtenu a été repris dans 25 mL de dichlorométhane. A ce mélange refroidi à 0°C ont été rajoutés 1.27 g (8.10 mmol; 1.0 éq) de (S)-pyrrolidine-2-carboxylate d'isopropyle. Le milieu réactionnel a été agité à 0°C pendant 30 minutes puis à température ambiante pendant 2 heures. Le milieu réactionnel a été dilué et lavé avec une solution d'acide chlorhydrique 1M (100 mL) puis avec une solution saturée d'hydrogénocarbonate de sodium (100 mL). La phase organique a été séchée sur MgSO4, filtrée et évaporée. 2.12 g de (S)-1-(2-fluoro-3-nitro-benzoyl)-pyrrolidine-2-carboxylate d'isopropyle. Rendement = 81%.

### Etape 7:

### (S)-1-(3-Amino-2-fluoro-benzoyl)-pyrrolidine-2-carboxylate d'isopropyle

Une solution de 2.12 g (6.55 mmol; 1.0 éq) de (S)-1-(2-fluoro-3-nitro-benzoyl)-pyrrolidine-2-carboxylate d'isopropyle dans 40 mL de méthanol en présence de 0.32 g (15% en masse) de palladium sur charbon à 10% a été agitée sous atmosphère d'hydrogène à température ambiante pendant 16 heures. Le milieu réactionnel a été filtré sur célite et concentré à sec. 1.87 g de (S)-1-(3-amino-2-fluoro-benzoyl)-pyrrolidine-2-carboxylate d'isopropyle ont été obtenus sous forme d'une huile incolore. Rendement = 97%.

### Etape 8:

### (S)-1-[2-Fluoro-3-(2-méthoxy-3,4-dioxo-cyclobut-1-énylamino)-benzoyl]-pyrrolidine-2-carboxylase d'isopropyle

A une solution de 1.87 g (0.08 mol; 1.0 éq) de (S)-1-(3-amino-2-fluoro-benzoyl)-pyrrolidine-2-carboxylate d'isopropyle dans 45 mL de méthanol ont été rajoutés 1.80 g (0.01 mol; 2.0 éq) de 3,4-diméthoxy-3-cyclobutène-1,2-dione. Le milieu réactionnel a été agité à température ambiante pendant 24h. Le solvant a été évaporé et le résidu a été chromatographié sur gel de silice (colonne prépackée de 200 g, éluant heptane/acétate d'éthyle20/80 puis 0/100). 1.32 g de (S)-1-[2-fluoro-3-(2-méthoxy-3,4-dioxo-cyclobut-1-énylamino)-benzoyl]-pyrrolidine-2-carboxylate d'isopropyle ont été obtenus sous forme d'un solide amorphe jaune clair. Rendement = 51%.

### Etape 9:

### (S)-1-[2-Fluoro-3-(2-{[(5-méthyl-furan-2-yl)-(tétrahydro-thiophèn-2-yl)-méthyl]-amino}-3,4-dioxo-cyclobut-1-énylamino)-benzoyl]pyrrolidine-2-carboxylate d'isopropyle

Un mélange de 600 mg (1.48 mmol; 1.0 éq) de (S)-1-[2-fluoro-3-(2-méthoxy-3,4-dioxo-cyclobut-1-énylamino)-benzoyl]-pyrrolidine-2-carboxylate d'isopropyle et de 350 mg (1.78 mmol; 1.2 éq) de C-(5-méthyl-furan-2-yl)-C-(tétrahydro-thiophèn-2-yl)-méthylamine (diastéréoisomère 2) en solution dans 25 mL de méthanol a été chauffé à 60°C pendant 18 heures. Le milieu réactionnel a été évaporé et le résidu a été chromatographié sur gel de silice élué au dichlorométhane/acétate d'éthyle (75/25). La pâte obtenue a été cristallisée dans l'éther éthylique, filtrée et séchée sous vide à 40°C. 575 mg de (S)-1-[2-fluoro-3-(2-{[(5-méthyl-furan-2-yl)-(tétrahydro-thiophèn-2-yl)-méthyl]-amino}-3,4-dioxo-cyclobut-1-énylamino)-benzoyl]-pyrrolidine-2-carboxylate d'isopropyle ont été obtenus sous forme d'un solide blanc. Rendement = 67%.
RMN ¹H (DMSO-d6, 400 MHz) : 0.89 (m, 1H), 1.06 (m, 1H), 1.19-1.23 (m, 4H), 1.81-2.07 (m, 7H), 2.26 (s, 3H), 2.29 (m, 1H), 2.75-2.83 (m, 2H), 3.35(m, 2H), 3.88-3.90 (m, 1H), 4.42-4.45 (m, 1H), 4.92-4.95 (m, 1H), 5.17 (t, 1H), 6.07 (d, J = 2.9 Hz, 1H), 6.33 (d, J = 4 Hz, 1H), 7.01 (t, 1H), 7.26 (t, J = 8 Hz, 1H), 8.04 (t, 1H), 8.55 (d, J = 8 Hz, 1H), 9.63 (s, 1H).

### EXEMPLE 5

### Préparation du (S)-1-[2-fluoro-3-(2-{[(5-méthyl-furan-2-yl)-(tétrahydro-thiophen-2-yl)-méthyl]-amino}-3,4-dioxo-cyclobut-1-enylamino)-benzoyl]-pyrrolidine-2-carboxylate

### d'éthyle

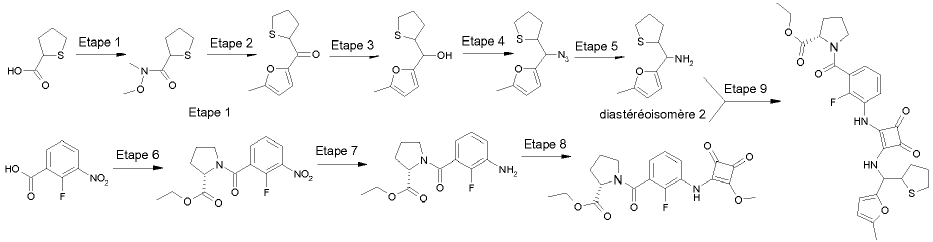

### Etapes 1 à 5:

De manière analogue à l'EXEMPLE 2 (étapes 1 à 5), C-(5-méthyl-furan-2-yl)-C-(tétrahydro-thiophèn-2-yl)-méthylamine (diastéréoisomère 2) a été préparé.

### Etapes 6 à 9:

De manière analogue à l'EXEMPLE 2 (étapes 6 à 9), et à partir du chlorhydrate de l'ester éthylique de la L-proline, (S)-1-[2-fluoro-3-(2-{[(5-méthyl-furan-2-yl)-(tétrahydro-thiophen-2-yl)-méthyl]-amino}-3,4-dioxo-cyclobut-1-enylamino)-benzoyl]-pyrrolidine-2-carboxylate d'éthyle a été préparé. Rendement = 30%.

### EXEMPLE 6

### Préparation du (R)-1-[2-hydroxy-3-(2-{[(5-methyl-furan-2-yl)-(tetrahydro-thiophen-2-yl)-methyl]-amino}-3,4-dioxo-cyclobut-1-enylamino)-benzoyl]-pyrrolidine-2-carboxylate de méthyle

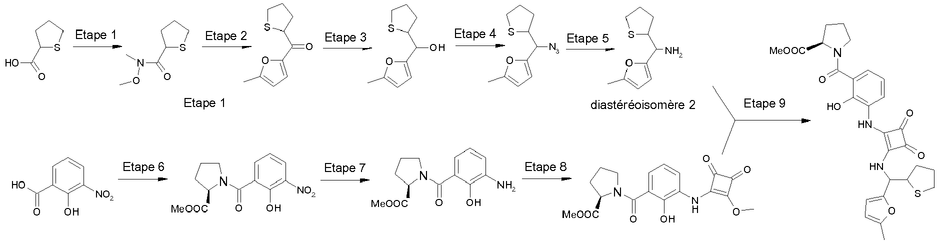

### Etapes 1 à 5:

De manière analogue à l'EXEMPLE 2 (étapes 1 à 5), C-(5-méthyl-furan-2-yl)-C-(tétrahydro-thiophèn-2-yl)-méthylamine (diastéréoisomère 2) a été préparé.

### Etape 6:

### (R)-1-(2-Hydroxy-3-nitro-benzoyl)-pyrrolidine-2-carboxylate de méthyle

Un mélange de 3.74 g (20.4 mmol, 1 éq) d'acide 3-nitrosalicylique et de 14.27 g (30.6 mmol, 1.5 éq) de bromotripyrrolidinophosphonium hexafluorophosphate dans 56 mL de dichlorométhane et en présence de 12.3 mL (71.5 mmol, 3.5 éq) de N,N-diisopropyléthylamine a été agité à température ambiante pendant 5 minutes. 5.07 g (30.6 mmol, 1.5 éq) d'hydrochlorure de (R)-pyrrolidine-2-carboxylate de méthyle en solution dans 10 mL de dichlorométhane ont été ajoutés goutte à goutte et le milieu réactionnel a été agité à température ambiante pendant 24 heures. Le milieu réactionnel a été lavé trois fois avec une solution aqueuse d'acide chlorhydrique 1 N. La phase organique a été séchée sur sulfate de sodium anhydre, filtrée et évaporée. L'huile obtenue a été chromatographiée sur gel de silice (colonne prépackée de 800 g) élué à l' heptane/acétate d'éthyle (gradient).
4.03 g de (R)-1-(2-hydroxy-3-nitro-benzoyl)-pyrrolidine-2-carboxylate de méthyle ont été obtenus sous forme d'une meringue jaune. Rendement = 67%

### Etape 7:

### (R)-1-(3-Amino-2-hydroxy-benzoyl)-pyrrolidine-2-carboxylate de méthyle

Une solution de 4.03 g (13.63 mmol; 1.0 éq) de (R)-1-(2-hydroxy-3-nitro-benzoyl)-pyrrolidine-2-carboxylate de méthyle dans 50 mL de méthanol a été agitée sous pression atmosphérique d'hydrogène en présence de 390 mg (10% en masse) de palladium sur charbon à 10% pendant 3 jours. Le milieu réactionnel a été filtré sur célite, rincé au méthanol et évaporé. Le résidu a été chromatographié sur gel de silice (colonne prépackée de 200 g) élué à l'heptane/acétate d'éthyle (gradient). 2.65 g de (R)-1-(3-amino-2-hydroxy-benzoyl)-pyrrolidine-2-carboxylate de méthyle ont été obtenus sous forme d'une huile jaune. Rendement = 74%.

### Etape 8:

### (R)-1-[2-Hydroxy-3-(2-méthoxy-3,4-dioxo-cyclobut-1-enylamino)-benzoyl]-pyrrolidine-2-carboxylate de méthyle

Un mélange de 2.64 g (10 mmol, 1 éq) de (R)-1-(3-amino-2-hydroxy-benzoyl)-pyrrolidine-2-carboxylate de méthyle et de 2.84 g (20 mmol, 2.0 éq) de 3,4-diméthoxy-3-cyclobutène-1,2-dione dans 80 mL d'éthanol a été chauffé à 50°C pendant 4 heures et demie. Le résidu a été repris avec de l'acétate d'éthyle et lavé trois fois avec une solution aqueuse de dihydrogénophosphate de sodium 1M. La phase organique a été séchée sur sulfate de sodium anhydre, filtrée et évaporée. L'huile a été chromatographiée sur gel de silice (colonne prépackée de 300 g) élué à l'heptane/acétate d'éthyle (gradient). 1.27 g de (R)-1-[2-hydroxy-3-(2-méthoxy-3,4-dioxo-cyclobut-1-enylamino)-benzoyl]-pyrrolidine-2-carboxylate de méthyle ont été obtenus sous forme d'un solide blanc. Rendement = 34%

### Etape 9:

### (R)-1-[2-Hydroxy-3-(2-{[(5-methyl-furan-2-yl)-(tétrahydro-thiophen-2-yl)-methyl]-amino}-3,4-dioxo-cyclobut-1-enylamino)-benzoyl]-pyrrolidine-2-carboxylate de méthyle

Un mélange de 463 mg (1.24 mmol, 1 éq) de (R)-1-[2-hydroxy-3-(2-methoxy-3,4-dioxo-cyclobut-1-enylamino)-benzoyl]-pyrrolidine-2-carboxylate de méthyle et de 293 mg (1.48 mmol, 1.2 éq) de C-[(R)-C-(5-méthyl-furan-2-yl)-C-tétrahydro-thiophen-2-yl]-méthylamine (diastéréoisomère 2) en solution dans 15 mL de méthanol a été chauffé à 50°C pendant 14 heures. Le milieu réactionnel a été évaporé et le résidu a été chromatographié sur gel de silice HP (colonne puriFlash PF-15SI/40G, puriFlash) élué au dichlorométhane/acétate d'éthyle (gradient). 538 mg de (R)-1-[2-hydroxy-3-(2-{[(5-methyl-furan-2-yl)-(tetrahydro-thiophen-2-yl)-methyl]-amino}-3,4-dioxo-cyclobut-1-enylamino)-benzoyl]-pyrrolidine-2-carboxylate de méthyle ont été obtenus sous forme d'un solide jaune. Rendement = 81%.
RMN ¹H (DMSO-d6, 400 MHz) : 1.82-1.99 (m, 5H), 2.03-2.05 (m, 2H), 2.26 (s, 3H), 2.75-2.84 (m, 2H), 3.59-3.67 (m, 2H), 3.84-3.88 (m, 1H), 3.89 (s, 3H), 4.53 (m, 1H), 5.19 (t, J = 9.6 Hz, 1H), 6.06 (dd, J = 1.0-3.0 Hz, 1H), 6.30 (d, J = 3.1 Hz, 1H), 6.92 (t, J = 9.6 Hz, 1H), 7.12 (d, J = 6.2 Hz, 1H), 7.88 (d, J = 7.9 Hz, 1H), 8.77 (d, J = 9.6 Hz, 1H), 9.38 (s, 1H).

### EXEMPLE 7

### Préparation du (S)-1-[2-hydroxy-3-(2-{[(5-methyl-furan-2-yl)-(tétrahydro-thiophen-2-yl)-methyl]-amino}-3,4-dioxo-cyclobut-1-enylamino)-benzoyl]-pyrrolidine-2-carboxylate de méthyle

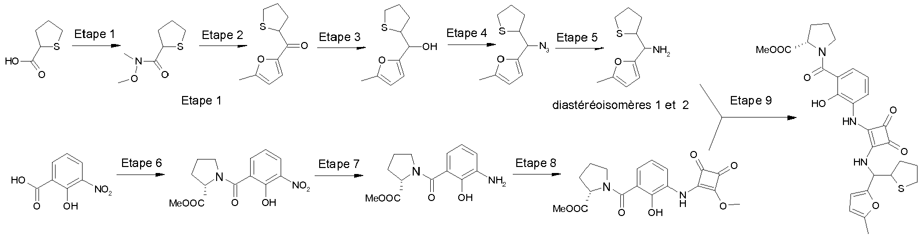

### Etapes 1 à 5:

De manière analogue à l'EXEMPLE 2 (étapes 1 à 5), C-(5-méthyl-furan-2-yl)-C-(tétrahydro-thiophèn-2-yl)-méthylamine (diastéréoisomères 1 et 2) ont été préparés.

### Etapes 6 à 9:

De manière analogue à l'EXEMPLE 6 (étapes 6 à 9), et à partir du chlorhydrate de l'ester méthylique de la L-proline et de C-(5-méthyl-furan-2-yl)-C-(tétrahydro-thiophèn-2-yl)-méthylamine (diastéréoisomère 2), (S)-1-2-hydroxy-3-(2-{[(5-methyl-furan-2-yl)-(tétrahydro-thiophen-2-yl)-méthyl]-amino}-3,4-dioxo-cyclobut-1-enylamino)-benzoyl]-pyrrolidine-2-carboxylate de méthyle a été préparé. Rendement = 11%.
RMN ¹H (DMSO-d6, 400 MHz) : 1.84-1.93 (m, 4H), 2.01-2.04 (m, 2H), 2.26 (s, 3H), 2.29-2.33 (m, 1H), 2.79-2.82 (m, 2H), 3.35-3.42 (m, 1H), 3.61-3.65 (m, 2H), 3.67 (s, 3H), 3.95-3.97 (m, 1H), 4.56 (m, 1H), 5.40 (t, J = 8.0 Hz, 1H), 6.06 (d, J = 2.0 Hz, 1H), 6.26 (d, J = 2.9 Hz, 1H), 6.92 (d, J = 7.3 Hz, 1H), 7.12 (m, 1H), 7.85 (d, J = 7.6 Hz, 1H), 8.84 (d, J = 9.7 Hz, 1H), 9.54 (s, 1H).

### Etapes 6 à 9:

De manière analogue à l'EXEMPLE 6 (étapes 6 à 9), et à partir du chlorhydrate de l'ester méthylique de la L-proline et de C-(5-méthyl-furan-2-yl)-C-(tétrahydro-thiophèn-2-yl)-méthylamine (diastéréoisomère 1), (S)-1[2-hydroxy-3-(2-{[(5-methyl-furan-2-yl)-(tétrahydro-thiophen-2-yl)-méthyl]-amino}-3,4-dioxo-cyclobut-1-enylamino)-benzoyl]-pyrrolidine-2-carboxylate de méthyle a été préparé. Rendement = 7%.
RMN ¹H (DMSO-d6, 400 MHz) : 1.64-1.68 (m, 1H), 1.88-1.93 (m, 4H), 2.03-2.06 (m, 2H), 2.27 (s, 3H), 2.76-2.81 (m, 2H), 3.38 (m, 1H), 3.61-3.64 (m, 2H), 3.67 (s, 3H), 3.85 (m, 1H), 4.52 (m, 1H), 5.18 (t, J = 9.7 Hz, 1H), 6.06 (d, J = 2.2 Hz, 1H), 6.30 (d, J = 3.1 Hz, 1H), 6.92 (d, J = 7.6 Hz, 1H), 6.94 (t, 1H), 7.12 (m, 1H), 7.85 (m, 1H), 8.77 (d, J = 9.6 Hz, 1H), 9.38 (s, 1H).

### EXEMPLE 8

### Préparation du 2-hydroxy-N-méthyl-3-(2-{[(S)-(5-méthyl-furan-2-yl)-tétrahydro-thiophen-2-yl-méthyl]-amino}-3,4-dioxo-cyclobut-1-enylamino)-N-(2,2,2-trifluoro-éthyl)-benzamide

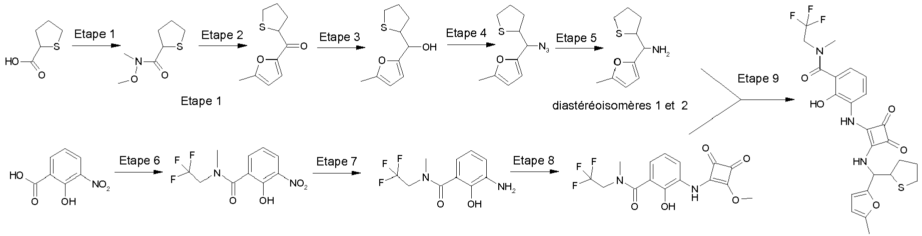

### Etapes 1 à 5:

De manière analogue à l'EXEMPLE 2 (étapes 1 à 5), C-(5-méthyl-furan-2-yl)-C-(tétrahydro-thiophèn-2-yl)-méthylamine (diastéréoisomères 1 et 2) ont été préparés.

### Etapes 6 à 9:

De manière analogue à l'EXEMPLE 6 (étapes 6 à 9), et à partir du chlorhydrate de méthyl-(2,2,2-trifluoro-éthyl)-amine et de C-(5-méthyl-furan-2-yl)-C-(tétrahydro-thiophèn-2-yl)-méthylamine (diastéréoisomère 2), 2-hydroxy-N-méthyl-3-(2-{[(S)-(5-méthyl-furan-2-yl)-tétrahydro-thiophen-2-yl-méthyl]-amino}-3,4-dioxo-cyclobut-1-enylamino)-N-(2,2,2-trifluoro-éthyl)-benzamide a été préparé. Rendement = 8%.
RMN ¹H (DMSO-d6, 400 MHz) : 1.83 (m, 1H), 1.91 (m, 1H), 2.06 (m, 2H), 2.27 (s, 3H), 2.80 (m, 2H), 2.96 (s, 2H), 3.08 (m, 1H), 3.87 (m, 1H), 4.34 (s, 1H), 5.20 (t, J = 9.6 Hz, 1H), 6.06 (d, J = 2.9 Hz, 1H), 6.30 (d, J = 3.0 Hz, 1H), 6.83 (d, J = 7.6 Hz, 1H), 6.94 (t, J = 7.8 Hz, 1H), 7.78 (d, J = 8.0 Hz, 1H), 8.73 (d, J = 9.6 Hz, 1H), 9.53 (s, 1H), 9.79 (s, 1H).

### Etapes 6 à 9:

De manière analogue à l'EXEMPLE 6 (étapes 6 à 9), et à partir du chlorhydrate de méthyl-(2,2,2-trifluoro-éthyl)-amine et de C-(5-méthyl-furan-2-yl)-C-(tétrahydro-thiophèn-2-yl)-méthylamine (diastéréoisomère 1), (S)-1-[2-hydroxy-3-(2-[{(5-methyl-furan-2-yl)-(tétrahydro-thiophen-2-yl)-méthyl]-amino}-3,4-dioxo-cyclobut-1-enylamino)-benzoyl]-pyrrolidine-2-carboxylate de méthyle a été préparé. Rendement = 10%.
RMN ¹H (DMSO-d6, 400 MHz) : 1.65 (m, 1H), 1.89 (m, 1H), 2.02 (m, 2H), 2.26 (s, 3H), 2.80 (m, 2H), 2.97 (s, 3H), 4.01 (m, 1H), 4.34 (s, 1H), 5.41 (dd, J = 6.7 Hz, 9.4 Hz, 1H), 6.06 (d, J = 3.0 Hz, 1H), 6.26 (d, J = 3.0 Hz, 1H), 6.83 (dd, J = 7.6 Hz, 1.2 Hz, 1H), 6.94 (t, J = 7.8 Hz, 1H), 7.76 (d, J = 8.0 Hz, 1H), 8.77 (d, J = 9.6 Hz, 1H), 9.52 (s, 1H), 9.81 (s, 1H).

### EXEMPLE 9

### Préparation du {[2-hydroxy-3-(2-{[(5-méthyl-furan-2-yl)-(tétrahydro-thiophen-2-yl)-méthyl]-amino}-3,4-dioxo-cyclobut-1-enylamino)-benzoyl]-méthyl-amino}-acétate de méthyle

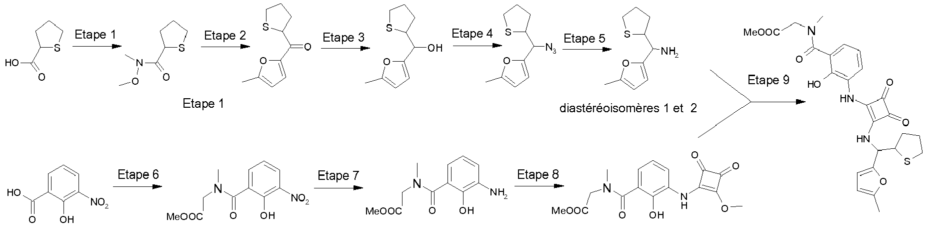

### Etapes 1 à 5:

De manière analogue à l'EXEMPLE 2 (étapes 1 à 5), les diastéréoisomères 1 et 2 de la C-(5-méthyl-furan-2-yl)-C-(tétrahydro-thiophèn-2-yl)-méthylamine ont été préparés.

### Etapes 6 à 9:

De manière analogue à l'EXEMPLE 6 (étapes 6 à 9), et à partir de la méthylamino-acétate de méthyle et de C-(5-méthyl-furan-2-yl)-C-(tétrahydro-thiophèn-2-yl)-méthylamine (diastéréoisomère 2), {[2-hydroxy-3-(2-{[(5-méthyl-furan-2-yl)-(tétrahydro-thiophen-2-yl)-méthyl]-amino}-3,4-dioxo-cyclobut-1-enylamino)-benzoyl]-méthyl-amino}-acétate de méthyle a été préparé. Rendement = 26%.
RMN ¹H (DMSO-d6, 400 MHz) : 1.81-1.98 (m, 2H), 2.05 (m, 2H), 2.26 (s, 3H), 2.78-2.85 (m, 2H), 2.95 (s, 3H), 3.67 (s, 3H), 3.84-3.88 (m, 1H), 4.20 (m, 2H), 5.19 (m, 1H), 6.07 (d, J = 3.0 Hz, 1H), 6.30 (d, J = 3.0 Hz, 1H), 6.90 (m, 1H), 7.78 (m, 1H), 8.79 (d, 1H), 9.32 (s, 1H), 10.53 (s, 1H).

### Etapes 6 à 9:

De manière analogue à l'EXEMPLE 6 (étapes 6 à 9), et à partir de la méthylamino-acétate de méthyle et de C-(5-méthyl-furan-2-yl)-C-(tétrahydro-thiophèn-2-yl)-méthylamine (diastéréoisomère 1), {[2-hydroxy-3-(2-{[(5-méthyl-furan-2-yl)-(tétrahydro-thiophen-2-yl)-méthyl]-amino}-3,4-dioxo-cyclobut-1-enylamino)-benzoyl]-méthyl-amino}-acétate de méthyle a été préparé. Rendement = 37%.
RMN ¹H (DMSO-d6, 400 MHz) : 1.65 (m, 1H), 1.88-1.92 (m, 2H), 1.98-2.05 (m, 2H), 2.26 (s, 3H), 2.79-2.82 (m, 2H), 2.96 (s, 3H), 3.67 (s, 3H), 3.95-4.00 (m, 1H), 4.22 (m, 2H), 5.40 (dd, 1H), 6.05 (dd, J = 3.0 Hz, 1H), 6.26 (d, J = 3.0 Hz, 1H), 6.90 (m, 2H), 7.76 (m, 1H), 8.80 (d, 1H), 9.52 (s, 1H), 9.81 (s, 1H).

### EXEMPLE 10

### Préparation du 6-chloro-2-hydroxy-N,N-diméthyl-3-(2-{[(5-méthyl-furan-2-yl)-(tétrahydro-thiophèn-2-yl)-méthyl]-amino}-3,4-dioxo-cyclobut-1-enylamino)-benzènesulfonamide

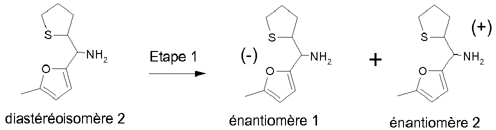

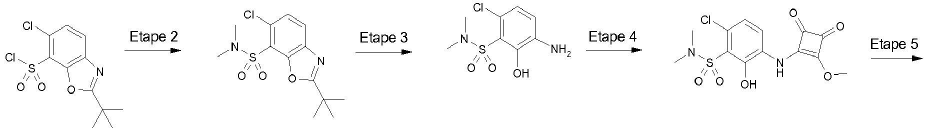

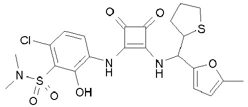

### Etape 1:

### C-(5-Méthyl-furan-2-yl)-C-(tétrahydro-thiophen-2-yl)-méthylamine

La séparation du diastéréoisomère 2 (préparé dans l'EXEMPLE 2) en énantiomères a été effectuée sur les colonnes semi-préparatives chirales CHIRALPACK^{R} AD-H 5 µm et CHIRALPACK^{R}AZ-H 5 µm, phase mobile: hexane/éthanol (70/30), débit de 5 mL/min.
Enantiomère 1 (-) du diastéréoisomère 2 : 1^{er} élué
Enantiomère 2 (+) du diastéréoisomère 2 : 2^{ème} élué

### Etape 2:

### Acide 2-tert-butyl-6-chloro-benzooxazole-7-sulfonique diméthylamide

12.9 mL (93 mmol; 3.0 éq) de triéthylamine et 93 mL de diméthylamine 2M dans le tétrahydrofuranne ont été ajoutés goutte à goutte sur une solution de 9.55 g (31 mmol; 1.0 éq) de chlorure de 2-tert-butyl-6-chloro-benzoxazole-7-sulfonyle dans 200 mL de tétrahydrofuranne refroidie à 0°C. Le milieu réactionnel a été agité à 0°C pendant 3 heures puis a été traité avec de l'eau. Le milieu réactionnel a été extrait à l'acétate d'éthyle. Les phases organiques ont été rassemblées, lavées à l'eau, séchées sur sulfate de magnésium, filtrées et évaporées. 9.12 g d'acide 2-tert-butyl-6-chloro-benzooxazole-7-sulfonique dimethylamide ont été obtenus sous forme d'un solide beige. Rendement = 93%.

### Etape 3:

### 3-Amino-6-chloro-2-hydroxy-N,N-dimethyl-benzènesulfonamide

11 mL (205 mmol; 1.20 V) d'acide sulfurique et 11 mL d'eau ont été ajoutés goutte à goutte sur une solution de 9.12 g (28.8 mmol; 1.0 éq) d'acide 2-tert-butyl-6-chloro-benzooxazole-7-sulfonique dimethylamide dans 41 mL de 1,4-dioxanne. Le milieu réactionnel a été chauffé à reflux pendant 6 heures et demie. Le milieu réactionnel a été concentré et 440 mL de soude 1N ont été ajoutés (pH à 8). La solution a été extraite à l'acétate d'éthyle. Les phases organiques ont été rassemblées, séchées sur sulfate de magnésium, filtrées et évaporées. 6.94 g de 3-amino-6-chloro-2-hydroxy-N,N-diméthyl-benzènesulfonamide ont été obtenus sous forme d'un solide marron. Rendement = 96%

### Etape 4:

### 6-Chloro-3-(2-éthoxy-3,4-dioxo-cyclobut-1-enylamino)-2-hydroxy-N,N-diméthyl-benzènesulfonamide

Un mélange de 6.94 g (27.7 mmol; 1.0 éq) de 3-amino-6-chloro-2-hydroxy-N,N-dimethyl-benzenesulfonamide et de 9.42 g (55.4 mmol; 2.0 éq) de 3,4-diéthoxy-3-cyclobutène-1,2-dione dans 70 mL d'éthanol a été agité à température ambiante pendant 2 heures (4% de produit formé). Le mileu réactionnel a été chauffé à 50°C pendant 5 jours. L'insoluble a été filtré et séché sous vide à 45°C. 7.67 g de 6-chloro-3-(2-éthoxy-3,4-dioxo-cyclobut-1-enylamino)-2-hydroxy-N,N-diméthyl-benzènesulfonamide ont été obtenus sous forme d'un solide jaune. Rendement = 73%

### Etape 5:

### (-)-6-Chloro-2-hydroxy-N,N-diméthyl-3-(2-{[(5-méthyl-furan-2-yl)-(tétrahydro-thiophen-2-yl)-methyl]-amino}-3,4-dioxo-cyclobut-1-enylamino)-benzènesulfonamide

Un mélange de 500 mg (1.33 mmol; 1.0 éq) de 6-chloro-3-(2-éthoxy-3,4-dioxo-cyclobut-1-enylamino)-2-hydroxy-N,N-diméthyl-benzènesulfonamide et de 316 mg (1.60 mmol; 1.2 éq) de (-)-C-(5-méthyl-furan-2-yl)-C-(tétrahydro-thiophen-2-yl)-méthylamine en solution dans 20 mL de méthanol a été chauffé à 50°C pendant 16 heures. L'insoluble a été filtré, lavé avec un peu de méthanol et séché sous vide à 45°C. 615 mg de (-)-6-chloro-2-hydroxy-N,N-diméthyl-3-(2-{[(5-méthyl-furan-2-yl)-(tétrahydro-thiophen-2-yl)-methyl]-amino}-3,4-dioxo-cyclobut-1-enylamino)-benzènesulfonamide (diastéréoisomère 1) ont été obtenus sous forme d'un solide blanc cassé. Rendement = 88%
RMN ¹H (DMSO-d6, 400 MHz) : 1.81-1.98 (m, 2H), 2.05 (m, 2H), 2.27 (s, 3H), 2.75-2.85 (m, 2H), 2.87 (s, 6H), 3.17 (m, 2H), 3.84-3.90 (m, 1H), 4.10 (m, 1H), 5.19 (m, 1H), 6.07 (d, J = 3.0 Hz, 1H), 6.31 (d, J = 3.0 Hz, 1H), 7.20 (m, 1H), 8.02 (d, J = 12.0 Hz, 1H), 8.84 (d, 1H), 9.48 (s, 1H), 10.53 (s, 1H).

### Etape 5 bis:

### (+)-6-Chloro-2-hydroxy-N,N-diméthyl-3-(2-{[(5-méthyl-furan-2-yl)-(tétrahydro-thiophen-2-yl)-methyl]-amino}-3,4-dioxo-cyclobut-1-enylamino)-benzènesulfonamide

Un mélange de 500 mg (1.33 mmol; 1.0 éq) de 6-chloro-3-(2-éthoxy-3,4-dioxo-cyclobut-1-enylamino)-2-hydroxy-N,N-diméthyl-benzènesulfonamide et de 316 mg (1.60 mmol; 1.2 éq) de (+)-C-(5-méthyl-furan-2-yl)-C-(tétrahydro-thiophen-2-yl)-méthylamine en solution dans 20 mL de méthanol a été chauffé à 50°C pendant 16 heures. L'insoluble a été filtré, lavé avec un peu de méthanol et séché sous vide à 45°C. 595 mg de (+)-6-chloro-2-hydroxy-N,N-diméthyl-3-(2-{[(5-méthyl-furan-2-yl)-(tétrahydro-thiophen-2-yl)-methyl]-amino}-3,4-dioxo-cyclobut-1-enylamino)-benzènesulfonamide (diastéréoisomère 2) ont été obtenus sous forme d'un solide blanc cassé. Rendement = 85%
RMN ¹H (DMSO-d6, 400 MHz) : 1.81-1.98 (m, 2H), 2.05 (m, 2H), 2.27 (s, 3H), 2.75-2.85 (m, 2H), 2.87 (s, 6H), 3.17 (m, 2H), 3.84-3.90 (m, 1H), 4.10 (m, 1H), 5.19 (m, 1H), 6.07 (d, J = 3.0 Hz, 1H), 6.31 (d, J = 3.0 Hz, 1H), 7.20 (m, 1H), 8.02 (d, J = 12.0 Hz, 1H), 8.84 (d, 1H), 9.48 (s, 1H), 10.53 (s, 1H).

### EXEMPLE 11

### Préparation du 2-hydroxy-N,N-dimethyl-3-(2-{[(R)-(5-methyl-furan-2-yl)-tetrahydrofuran-2-yl-methyl]-amino}-3,4-dioxo-cyclobut-1-enylamino)-benzamide

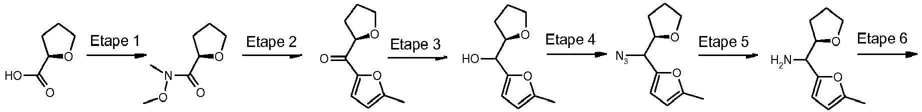

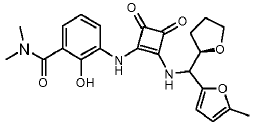

### Etape 1:

### (R)-Tétrahydro-furan-2-méthoxy-méthyl-carboxamide

A une solution de 10.0 g (86.1 mmol; 1.0 éq) d'acide tétrahydro-furan-2-carboxylique dans 50 mL de dichlorométhane ont été rajoutés goutte à goutte 8.0 mL (110.1 mmol; 1.28 éq) de chlorure de thionyle à température ambiante en 15 minutes. Le milieu réactionnel a été agité à température ambiante pendant 2 heures jusqu'à ce qu'il n'y ait plus de dégagement gazeux. Le dichlorométhane et l'excès de chlorure de thionyle ont été évaporés sous vide et le résidu a été coévaporé trois fois avec 50 mL de dichlorométhane. Le chlorure d'acide obtenu a été solubilisé dans 50 mL de dichlorométhane et 9.24 g (94.7 mmol; 1.10 éq) de chlorhydrate de *N*,*O*-diméthylhydroxylamine ont été ajoutés. Le milieu réactionnel a été refroidi à 0°C et un mélange de 27.5 mL (207.0 mol; 2.40 éq) de triéthylamine dans 50 mL de dichlorométhane a été additionné goutte à goutte en 90 minutes (en maintenant la température en dessous de 5°C). Après l'addition, le milieu réactionnel a été agité à température ambiante pendant une heure puis a été lavé avec 250 mL d'une solution aqueuse d'acide chlorhydrique à 1M. La phase aqueuse a été extraite avec 50 mL dichlorométhane. Les phases organiques ont été rassemblées, lavées avec 50 mL d'une solution aqueuse d'hydrogénophosphate de sodium saturée, séchées sur sulfate de magnésium anhydre, filtrées et évaporées. 8.5 g de (R)-Tetrahydro-furan-2- methoxy-methyl- carboxamide ont été obtenus sous forme d'une huile. Rendement = 62%.

### Etape 2:

### (5-Méthyl-furan-2-yl)-(R)-tétrahydro-furan-2-yl-méthanone

87 mL (215.6 mmol; 1.50 éq) de *n*-butyllithium à 2.5 M dans l'hexane ont été ajoutés goutte à goutte sur une solution de 19.5 mL (215.6 mmol; 1.50 éq) de 2-méthyl-furane dans 550 mL de tétrahydrofurane refroidie à -78°C. Le mélange a été laissé remonter à température ambiante pendant 2 heures puis a été refroidi à -78°C. Une solution de 22.88 g (143.7 mmol; 1.00 éq) de (R)-tetrahydro-furan-2- methoxy-methyl-carboxamide dans 200 mL de tétrahydrofurane a été additionnée et le mélange réactionnel a été laissé à 0°C pendant 2 heures. Le milieu réactionnel a été dilué avec 200 mL d'acétate d'éthyle puis lavé avec 300 mL d'une solution aqueuse d'acide chlorhydrique 1N. La phase aqueuse a été séparée et extraite avec 200 mL d'acétate d'éthyle. Les phases organiques ont été rassemblées, lavées avec une solution saturée de chlorure de sodium, séchées sur sulfate de magnésium anhydre, filtrées et évaporées. L'huile brune obtenue a été filtrée sur silice (éluant : Heptane/Acétate d'éthyle 90/10). 17.72 g de (5-méthyl-furan-2-yl)-(R)-tétrahydro-furan-2-yl-méthanone ont été obtenus. Rendement = 68%.

### Etape 3:

### (5-Méthyl-furan-2-yl)-(R)-tétrahydro-furan-2-yl-méthanol

2.27 g (59.9 mmol; 1.20 éq) de borohydrure de sodium ont été ajoutés par petites portions sur une solution de 9.00 g (49.9 mmol; 1.0 éq) de (5-méthyl-furan-2-yl)-(R)-tétrahydro-furan-2-yl-méthanone dans 100 mL de tétrahydrofurane refroidie à 0°C. Le milieu réactionnel a été agité à température ambiante pendant 3 heures. Le milieu réactionnel a été versé dans 200 mL d'acétate d'éthyle puis 100 mL d'eau ont été rajoutés. La phase aqueuse a été extraite à l'acétate d'éthyle puis les phases organiques ont été rassemblées, lavées avec une solution de chlorure de sodium saturée, séchées sur sulfate de magnésium anhydre, filtrées et évaporées. 8.10 g de (5-méthyl-furan-2-yl)-(R)-tétrahydro-furan-2-yl-méthanol ont été obtenus. Rendement = 92%.

### Etape 4 :

### 2-((R)-Azido-tétrahydro-furan-2-yl-méthyl)-5-méthyl-furane

A une solution de 8.40 g (46.1 mmol; 1.0 éq) de (5-méthyl-furan-2-yl)-(R)-tétrahydro-furan-2-yl-méthanol dans 300 mL de toluène refroidie à 0°C ont été ajoutés 11.9 mL (55.3 mmol; 1.2 éq) de diphénylphosphoryl azide puis 8.3 mL (55.3 mmol; 1.2 éq) de 1,8-diazabicyclo[5.4.0]undec-7-ene goutte à goutte. Le mélange a été laissé revenir à température ambiante doucement puis a été agité pendant 24 heures. Le milieu réactionnel a été traité à l'eau et extrait à l'acétate d'éthyle. Les phases organiques ont été rassemblées, lavées avec une solution de dihydrogénophosphate de sodium 1M, séchées sur sulfate de magnésium, filtrées et évaporées. Le résidu a été chromatographié sur gel de silice (colonne puriFlash IR-50SI/300G, puriFlash) élué à l'heptane/acétate d'éthyle (90/10). 6.0 g de 2-((R)-azido-tétrahydro-furan-2-yl-méthyl)-5-méthyl-furan ont été obtenus (mélange des 2 diastéréoisomères). Rendement = 63%.

### Etape 5 :

### C[(R)-C-(5-Méthyl-furan-2-yl)-C-tétrahydro-furan-2-yl]-méthylamine

Une solution de 6.00 g (29.0 mmol; 1.0 éq) de 2-((R)-azido-tétrahydro-furan-2-yl-methyl)-5-méthyl-furane dans 250 mL de méthanol et en présence de 600 mg (10% en masse) de Pd/C 10% a été agitée à température ambiante sous pression atmosphérique d'hydrogène pendant 5 jours. Le milieu réactionnel a été filtré sur célite et le filtrat a été évaporé. Le résidu a été chromatographié sur gel de silice (colonne puriFlash IR-50SI-STD/300G, puriFlash) élué au dichlorométhane/acétate d'éthyle (gradient). 4.6 g de C-[(R)-C-(5-méthyl-furan-2-yl)-C-tétrahydro-furan-2-yl]-méthylamine ont été obtenus (mélange des 2 diastéréoisomères). Rendement = 70%.

### Etape 6:

### 2-Hydroxy-N,N-diméthyl-3-(2-{[(R)-(5-méthyl-furan-2-yl)-tétrahydro-furan-2-yl-méthyl]-amino}-3,4-dioxo-cyclobut-1-enylamino)-benzamide

Un mélange de 4.5 g (14.7 mmol; 1.0 éq) de 3-(2-éthoxy-3,4-dioxo-cyclobut-1-énylamino)-2-hydroxy-*N,N*-diméthyl-benzamide et de 2.7 g (14.7 mmol; 1.0 éq) de C-[(R)-C-(5-méthyl-furan-2-yl)-C-tétrahydro-furan-2-yl]-méthylamine en solution dans 100 mL de méthanol a été agité à 50°C pendant une nuit. Le milieu réactionnel a été évaporé et purifié par chromatographie sur gel de silice (éluent : Dichlorométhane/Acétate d'éthyle 70/30) et a été recristallisé dans un mélange Heptane/Dichlorométhane (75/25). 5.6 g de 2-hydroxy-N,N-diméthyl-3-(2-{[(R)-(5-méthyl-furan-2-yl)-tétrahydro-furan-2-yl-méthyl]-amino}-3,4-dioxo-cyclobut-1-enylamino)-benzamide ont été obtenus. Rendement = 54%.
RMN ¹H (DMSO-d6, 400 MHz) : 1.75-1.80 (m, 2H), 1.80-1.87 (m, 1H), 1.88-2.02 m, 1H), 2.27 (d, J = 1.8 Hz, 3H), 2.94 (s, 6H), 3.65-3.75 (m, 2H), 4.24-4.26 (m, 1H), 5.32 (dd, J₁ = 9.2 Hz, J₂ = 5.6 Hz, 1H), 6.06 (dd, J₁ = 1.0 Hz, J₂ = 3.0 Hz, 1H), 6.28 (d, J = 3.1 Hz, 1H), 6.87 (m, 2H), 7.77 (m, 1H), 8.84 (m, 1H), 9.45 (s, 1H), 9.95 (s, 1H).

### EXEMPLE 12

### Préparation du 2-hydroxy-N,N-dimethyl-3-(2-{[(S)-(5-methyl-furan-2-yl)-tetrahydro-furan-2-yl-methyl]-amino}-3,4-dioxo-cyclobut-1-enylamino)-benzamide

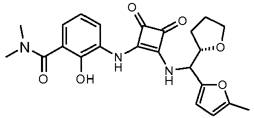

De manière analogue à l'EXEMPLE 11 (étapes 1 à 6), 2-hydroxy-N,N-diméthyl-3-(2-{[(S)-(5-méthyl-furan-2-yl)-tétrahydro-furan-2-yl-méthyl]-amino}-3,4-dioxo-cyclobut-1-enylamino)-benzamide a été préparé :
RMN ¹H (DMSO-d6, 400 MHz)_: 1.50-1.80 (m, 2H) ; 1.84 (m, 1H) ; 1.98 (m, 1H) ; 2.27 (s, 3H) ; 2.93 (s, 6H) ; 3.60-3.80 (m, 2H) ; 4.24 (m, 1H); 5.20-5.40 (m, 1H) ; 6.05 (d, J = 2.0 Hz, 1H) ; 6.28 (d, J = 3.0 Hz, 1H), 6.87 (m, 2H) ; 7.77 (m, 1H) ; 8.81 (d, J = 9.6 Hz, 1H), 9.46 (s, 1H), 9.97 (s, 1H).

### EXEMPLE 13

### Préparation du 3-(3,4-dioxo-2-{[phenyl-(tétrahydrofuran-2-yl)-méthy]-amino}-cyclobut-1-enyl-amino)-2-hydroxy-N,N-diméthyl-benzamide

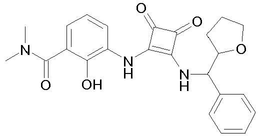

De manière analogue à l'EXEMPLE 1 (étapes 6 à 9), et à partir de C-phényl-C-(tétrahydrofuran-2-yl)-méthylamine commerciale et de 3-(2-méthoxy-3,4-dioxo-cyclobut-1-énylamino)-2-hydroxy-*N,N*-diméthyl-benzamide, 3-(3,4-dioxo-2-{[phenyl-(tétrahydrofuran-2-yl)-méthyl]-amino-cyclobut-1-enylamino)-2-hydroxy-N,N-diméthyl-benzamide a été préparé. (mélange de diastéréoisomères) (Tf = 120-125°C). LC/MS : 99.66% [435].
RMN ¹H (DMSO-d6, 400 MHz)_: 1.57-1.91 (m, 5H) ; 2.94 (s, 6H) ; 3.64-3.73 (m, 2H) ; 3.84-3.93 (m, 1H); 4.19-4.28 (m, 1H) ; 5.27 (t, 1H) ; 6.85 (d, J = 6.2 Hz, 2H), 7.3-7.42 (m, 6H) ; 7.73 (dd, J = 8.1 Hz, 1H) ; 8.86-8.96 (dd, J = 9.6 Hz, 1H).

### EXEMPLE 14

### Préparation de 3-(2-{[((R)-2,2-diméthyl-[1,3]dioxolan-4-yl)-(5-méthyl-furan-2-yl)-méthyl]-amino}-3,4-dioxo-cyclobut-1-enylamino)-2-hydroxy-N,N-diméthyl-benzamide (diastéréoisomères 1 et 2)

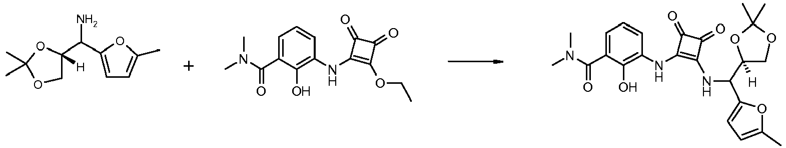

De manière analogue à l'EXEMPLE 1 (étapes 3 à 5), et à partir de (S)-glycéraldéhyde acétonide, C-((R)-2,2-diméthyl-[1,3]dioxolan-4-yl)-C-(5-méthyl-furan-2-yl)-méthylamine a été préparée. Rendement = 10%.

La séparation des 2 diastéréoisomères a été effectuée au niveau de l'intermédiaire azide (2^{ème} étape décrite ci-dessous).

### (R)-4-[Azido-(5-méthyl-furan-2-yl)-méthyl]-2,2-diméthyl-[1,3]dioxolane

4.15 g (15.0 mmol, 1.2 éq) de diphenylphosphoryl azide ont été ajoutés goutte à goutte sur une solution de 2.67 g (12.5 mmol, 1 éq) de ((S)-2,2-diméthyl-[1,3]dioxolan-4-yl)-(5-méthyl-furan-2-yl)-méthanol brut dans 40 mL de toluène. Le milieu réactionnel a été refroidi à 0°C puis 2.2 mL (15.0 mmol, 1.2 éq) de 1,8-diazabicyclo[5.4.0]undec-7-ene ont été ajoutés goutte à goutte. Le milieu réactionnel a été agité à température ambiante pendant 42 heures. Le milieu réactionnel (hétérogène) a été décanté, traité à l'eau et extrait à l'acétate d'éthyle. Les phases organiques ont été rassemblées, lavées avec une solution de dihydrogénophosphate de sodium 1N, séchées sur sulfate de magnésium, filtrées et évaporées. Le résidu a été chromatographié sur gel de silice (colonne puriFlash IR-50SI/200G, Spot II puis colonne Redi*Sep Rf* Gold 40g, Spot II) élué à l'heptane/acétate d'éthyle (95/5).
562 mg de (R)-4-[azido-(5-méthyl-furan-2-yl)-méthyl]-2,2-diméthyl-[1,3]dioxolane (diastéréoisomère 1) ont été obtenus.
257 mg de (R)-4-[azido-(5-méthyl-furan-2-yl)-méthyl]-2,2-diméthyl-[1,3]dioxolane (diastéréoisomère 2) ont été obtenus.
De manière analogue à l'EXEMPLE 1 (étapes 6 à 9), et à partir des 2 diastéréoisomères C-((R)-2,2-diméthyl-[1,3]dioxolan-4-yl)-C-(5-méthyl-furan-2-yl)-méthylamine et de 3-(2-éthoxy-3,4-dioxo-cyclobut-1-enylamino)-2-hydroxy-N,N-diméthyl-benzamide, les diastéréoisomères 1 et 2 de 3-(2-{[((R)-2,2-diméthyl-[1,3]dioxolan-4-yl)-(5-méthyl-furan-2-yl)-méthyl]-amino}-3,4-dioxo-cyclobut-1-enylamino)-2-hydroxy-N,N-diméthyl-benzamide ont été préparés. Rendement de 78% (diastéréoisomère 1) et 52% (diastéréoisomère 2).

### 3-(2-{[((R)-2,2-diméthyl-[1,3]-dioxolan-4-yl)-(5-méthyl-furan-2-yl)-méthyl]-amino}-3,4-dioxo-cyclobut-1-enylamino)-2-hydroxy-N,N-diméthyl-benzamide (diastéréoisomère 1).

RMN ¹H (DMSO-d6, 400 MHz): 1.27 (s, 3H) ; 1.29 (s, 3H) ; 2.27 (s, 3H) ; 2.94 (s, 6H) ; 3.86 (m, 1H) ; 4.14 (m, 1H) ; 4.49 (q, J = 5.9 Hz, 1H) ; 5.43 (dd, J = 9.4 Hz, 1H) ; 6.06 (dd, J = 3.0 Hz, 1H), 6.30 (d, J = 3.1 Hz, 1H) ; 6.87 (dd, J = 6.8 Hz, 2H) ; 7.76 (dd, J = 6.7 Hz, 1H) ; 8.82 (d, J = 9.6 Hz, 1H) ; 9.40 (s, 1H).

### 3-(2-{[((R)-2,2-diméthyl-[1,3]dioxolan-4-yl)-(5-méthyl-furan-2-yl)-méthyl]-amino}-3,4-dioxo-cyclobut-1-enylamino)-2-hydroxy-N,N-diméthyl-benzamide (diastéréoisomère 2).

RMN ¹H (DMSO-d6, 400 MHz): 1.30 (s, 3H) ; 1.36 (s, 3H) ; 2.27 (s, 3H) ; 2.93 (s, 6H) ; 3.67 (q, J = 5.5 Hz, 1H) ; 4.06 (q, J = 6.8 Hz, 1H) ; 4.51 (q, J = 6.6 Hz, 1H) ; 5.32 (dd, J = 9.3 Hz, 1H) ; 6.07 (dd, J = 3.0 Hz, 1H), 6.35 (d, J = 3.1 Hz, 1H) ; 6.87 (m, 2H) ; 7.76 (dd, J = 7.1 Hz, 1H) ; 8.76 (d, J = 9.5 Hz, 1H) ; 9.40 (s, 1H) ; 9.92 (s, 1H).

### EXEMPLE 15 :

### Préparation de (S)-1-[2-Fluoro-3-(2-{[(5-méthyl-furan-2-yl)-(tétrahydro-thiophen-2-yl)-méthyl]-amino}-3,4-dioxo-cyclobut-1-enylamino)-benzoyl]-pyrrolidine-2-carboxylate de méthyle

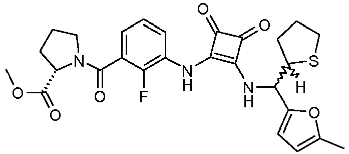

De manière analogue à l'EXEMPLE 2 (étapes 1 à 5), C-(5-méthyl-furan-2-yl)-C-(tétrahydro-thiophèn-2-yl)-méthylamine (diastéréoisomère 1*)* a été préparé.

De manière analogue à l'EXEMPLE 2 (étapes 6 à 9), et à partir du chlorhydrate de l'ester méthylique de la L-proline, (S)-1-[2-fluoro-3-(2-{[(5-méthyl-furan-2-yl)-(tétrahydro-thiophen-2-yl)-méthyl]-amino}-3,4-dioxo-cyclobut-1-enylamino)-benzoyl]-pyrrolidine-2-carboxylate de méthyle a été préparé.
RMN ¹H (DMSO-d6, 400 MHz) : 1.66 (m, 1H), 1.85-2.05 (2m, 6H), 2.26 (s, 3H), 2.30 (m, 1H), 2.80-2.83 (m, 2H), 3.35-3.38 (m, 2H), 3.46-3.68 (2s, 3H), 4.02 (m, 1H), 4.32-4.53 (2m, 1H), 5.40 (t, J = 9.0 Hz, 1H), 6.06 (d, J = 3.0 Hz, 1H), 6.28 (d, J = 3.0 Hz, 1H), 7.02 (t, J = 6.2 Hz, 1H), 7.27 (t, J = 7.9 Hz, 1H), 8.05 (t, J = 8.1 Hz, 1H), 8.63 (d, J = 9.6 Hz, 1H), 9.83 (s, 1H).

### EXEMPLE 16 :

### Préparation de 3-(2-hydroxy-pyridin-3-ylamino)-4-{[(5-méthyl-furan-2-yl)-(tétrahydro-thiophen-2-yl)-méthyl]-amino}-cyclobut-3-ène-1,2-dione

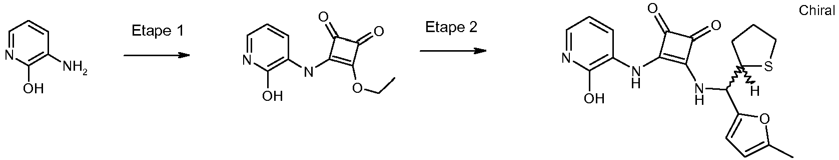

De manière analogue à l'EXEMPLE 2 (étapes 1 à 5), C-(5-méthyl-furan-2-yl)-C-(tétrahydro-thiophèn-2-yl)-méthylamine (diastéréoisomère 2) a été préparé.

### Etape 1 :

### 3-Ethoxy-4-(2-hydroxy-pyridin-3-ylamino)-cyclobut-3-ène-1,2-dione

Un mélande de 1.82 g (16.5 mmol, 1éq) de 3-amino-pyridin-2-ol et de 3.6 ml (24.8 mmol, 1.5 éq) de 3,4-diéthoxy-cyclobut-3-ène-1,2-dione (3.6 ml, 24.8 mmol) en solution dans 87 ml d'éthanol a été agité à température ambiante pendant 16 heures puis chauffé à 50°C pendant 3 jours avec formation d'un précipité. De l'éthanol a été additionnée pour favoriser la chute du précipité qui a été filtré, lavé à l'éther diéthylique et séché sous vide à 45°C. 3.47 g de 3-éthoxy-4-(2-hydroxy-pyridin-3-ylamino)-cyclobut-3-ène-1,2-dione ont été obtenus sous forme d'un solide marron. Rendement = 90%

### Etape 2 :

### 3-(2-Hydroxy-pyridin-3-ylamino)-4-{[(5-méthyl-furan-2-yl)-(tétrahydro-thiophen-2-yl)-méthyl]-amino}-cyclobut-3-ène-1,2-dione

Un mélange de 500 mg (2.1 mmol; 1.0 éq) de 3-éthoxy-4-(2-hydroxy-pyridin-3-ylamino)-cyclobut-3-ène-1,2-dione et de 505 mg (2.6 mmol; 1.2 éq) de C-[(R)-C-(5-méthyl-furan-2-yl)-C-tétrahydro-furan-2-yl]-méthylamine en solution dans 20 mL de méthanol a été chauffé à 50°C pendant 18 heures. Le milieu réactionnel a été évaporé et le résidu a été chromatographié sur gel de silice avec dépot solide (colonne puriFlash PF-15SI/40G, puriFlash) élué au dichlorométhane/méthanol (gradient). Le solide a été repris avec un peu d'éther diéthylique, filtré et séché sous vide à 45°C. 610 mg de 3-(2-hydroxy-pyridin-3-ylamino)-4-{[(5-méthyl-furan-2-yl)-(tétrahydro-thiophen-2-yl)-méthyl]-amino}-cyclobut-3-ène-1,2-dione ont été obtenus sous forme d'un solide blanc cassé. Rendement = 74%.
RMN ¹H (DMSO-d6, 400 MHz) : 1.79-1.84 (m, 1H), 1.88-1.92 (m, 1H), 2.00-2.06 (m, 2H), 2.26 (s, 3H), 2.75-2.83 (m, 2H), 3.81-3.86 (m, 1H), 5.17 (t, 1H), 6.05 (d, J = 2.1 Hz, 1H), 6.23-6.28 (m, 2H), 7.08 (dd, J = 6.5-1.5 Hz, 1H), 8.01 (dd, J = 7.3-1.5 Hz, 1H), 9.00 (d, 1H), 9.53 (s, 1H), 11.98 (s, 1H)

### EXEMPLE 17 :

### Préparation de 3-{[(5-méthyl-furan-2-yl)-(tétrahydro-thiophen-2-yl)-méthyl]-amino}-4-(1-méthyl-2-oxo-1,2-dihydro-pyridin-3-ylamino)-cyclobut-3-ène-1,2-dione

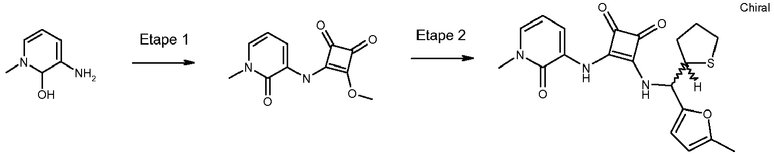

De manière analogue à l'EXEMPLE 2 (étapes 1 à 5), C-(5-méthyl-furan-2-yl)-C-(tétrahydro-thiophèn-2-yl)-méthylamine (diastéréoisomère 2) a été préparé.

### Etape 1:

### 3-Méthoxy-4-(1-méthyl-2-oxo-1,2-dihydro-pyridin-3-ylamino)-cyclobut-3-ène-1,2-dione

De manière analogue à l'EXEMPLE 16, (étape 1), et à partir du 3,4-diméthoxy-cyclobut-3-ène-1,2-dione, 3-méthoxy-4-(1-méthyl-2-oxo-1,2-dihydro-pyridin-3-ylamino)-cyclobut-3-ène-1,2-dione a été préparée. Rendement = 50%.

### Etape 2 :

### 3-{[(5-méthyl-furan-2-yl)-(tétrahydro-thiophen-2-yl)-méthyl]-amino}-4-(1-méthyl-2-oxo-1,2-dihydro-pyridin-3-ylamino)-cyclobut-3-ène-1,2-dione

De manière analogue à l'EXEMPLE 16, (étape 2), 3-{[(5-méthyl-furan-2-yl)-(tétrahydro-thiophen-2-yl)-méthyl]-amino}-4-(1-méthyl-2-oxo-1,2-dihydro-pyridin-3-ylamino)-cyclobut-3-ène-1,2-dione a été préparée. Rendement = 90%.
RMN ¹H (DMSO-d6, 400 MHz) : 1.77-1.84 (m, 1H), 1.86-1.94 (m, 1H), 1.98-2.08 (m, 2H), 2.26 (s, 3H), 2.73-2.85 (m, 2H), 3.52 (s, 3H), 3.82-3.88 (m, 1H), 5.18 (t, J = 9.5 Hz, 1H), 6.05-6.07 (m, 1H), 6.25-6.30 (m, 2H), 7.40 (dd, J = 6.8 Hz, J = 1.6 Hz, 1H), 7.99 (dd, J = 7.4 Hz, J = 1.6 Hz, 1H), 9.02 (d, J = 9.6 Hz, 1H), 9.57 (s, 1H)

### EXEMPLE 18 :

### Préparation de (-)-2-hydroxy-N-méthyl-3-(2-{[(S)-(5-méthyl-furan-2-yl)-(tétrahydro-thiophen-2-yl)-méthyl]-amino}-3,4-dioxo-cyclobut-1-enylamino)-N-(2,2,2-trifluoro-éthyl)-benzamide

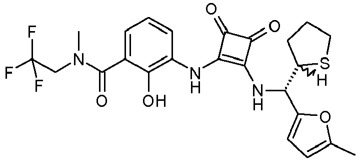

De manière analogue à l'EXEMPLE 10 (étapes 1), (-)-C-(5-méthyl-furan-2-yl)-C-(tétrahydro-thiophèn-2-yl)-méthylamine (énantiomère 1*)* a été préparé.

De manière analogue à l'EXEMPLE 8 (étapes 6 à 9), et à partir de (-)-C-(5-méthyl-furan-2-yl)-C-(tétrahydro-thiophèn-2-yl)-méthylamine (énantiomère 1), (-)-2-hydroxy-N-méthyl-3-(2-{[(S)-(5-méthyl-furan-2-yl)-(tétrahydro-thiophen-2-yl)-méthyl]-amino}-3,4-dioxo-cyclobut-1-enylamino)-N-(2,2,2-trifluoro-éthyl)-benzamide a été préparé. Rendement = 22%.
RMN ¹H (DMSO-d6, 400 MHz) : 1.91 (m, 1H), 1.96 (m, 1H), 2.05 (m, 1H), 2.27 (s, 3H), 2.81 (m, 2H), 2.96 (s, 3H), 3.86 (m, 1H), 4.11 (m, 1H), 4.33 (s, 1H), 5.20 (t, J = 9.6 Hz, 1H), 6.06 (dd, J = 0.9 Hz, 3.0 Hz, 1H), 6.30 (d, J = 3.1 Hz, 1H), 6.82 (d, J = 6.5 Hz, 1H), 6.92 (s, 1H), 7.78 (d, J = 8.4 Hz, 1H), 8.72 (d, J = 9.2 Hz, 1H), 9.34 (s, 1H), 9.79 (s, 1H)

### EXEMPLE 19 :

### Préparation de (-)-{[2-hydroxy-3-(2-{[(S)-(5-méthyl-furan-2-yl)-(tétrahydro-thiophen-2-yl)-méthyl]-amino}-3,4-dioxo-cyclobut-1-enylamino)-benzoyl]-méthyl-amino}-acétate de

### méthyle

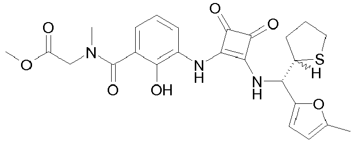

De manière analogue à l'EXEMPLE 10 (étapes 1), (-)-C-(5-méthyl-furan-2-yl)-C-(tétrahydro-thiophèn-2-yl)-méthylamine (énantiomère 1*)* a été préparé.

De manière analogue à l'EXEMPLE 6 (étapes 6 à 9), et à partir du méthylamino-acétate de méthyle et de (-)-C-(5-méthyl-furan-2-yl)-C-(tétrahydro-thiophèn-2-yl)-méthylamine (énantiomère 1), (-)-{[2-hydroxy-3-(2-{[(S)-(5-méthyl-furan-2-yl)-(tétrahydro-thiophen-2-yl)-méthyl]-amino}-3,4-dioxo-cyclobut-1-enylamino)-benzoyl]-méthyl-amino}-acétate de méthyle a été préparé. Rendement = 25%.
RMN ¹H (DMSO-d6, 400 MHz) : 1.80-1.96 (m, 2H), 1.99-2.10 (m, 2H), 2.37 (s, 3H), 2.74-2.85 (m, 2H), 2.95 (s1, 3H), 3.67 (s1, 3H), 3.83-3.89 (m, 1H), 4.00-4.35 (m, 2H), 5.20 (t, J = 9.6 Hz, 1H), 6.05-6.07 (m, 1H), 6.30 (d, J = 3.1 Hz, 1H), 6.65-6.95 (m, 2H), 7.80 (d, J = 7.8 Hz, 1H), 8.77 (d, J = 9.6 Hz, 1H), 9.36 (m, 1H), 9.82 (m, 1H)

### EXEMPLE 20 :

### Préparation de (-)-1-[2-hydroxy-3-(2-{[((S)-5-méthyl-furan-2-yl)-(tétrahydro-thiophen-2-yl)-méthyl]-amino}-3,4-dioxo-cyclobut-1-enylamino)-benzoyl]-pyrrolidine-2-(R)-carboxylate de méthyle

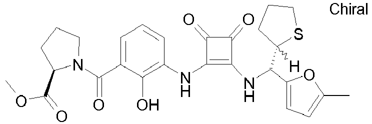

De manière analogue à l'EXEMPLE 10 (étapes 1), (-)-C-(5-méthyl-furan-2-yl)-C-(tétrahydro-thiophèn-2-yl)-méthylamine (énantiomère 1*)* a été préparé.

De manière analogue à l'EXEMPLE 6 (étapes 6 à 9), et à partir de l'ester méthylique de la L-proline et de (-)-C-(5-méthyl-furan-2-yl)-C-(tétrahydro-thiophèn-2-yl)-méthylamine (énantiomère 1), (-)-1-[2-hydroxy-3-(2-{[((S)-5-méthyl-furan-2-yl)-(tétrahydro-thiophen-2-yl)-méthyl]-amino}-3,4-dioxo-cyclobut-1-enylamino)-benzoyl]-pyrrolidine-2-(R)-carboxylate de méthyle a été préparé. Rendement = 7%.
RMN ¹H (DMSO-d6, 400 MHz) : 1.87-1.97 (m, 5H), 1.99-2.10 (m, 2H), 2.21-2.33 (m, 4H), 2.73-2.85 (m, 2H), 3.56-3.70 (m, 5H), 3.83-3.89 (m, 1H), 4.50-4.60 (m, 1H), 5.20 (t, J = 9.6 Hz, 1H), 6.05-6.07 (m, 1H), 6.30 (d, J = 3.1 Hz, 1H), 6.91 (t, J = 7.9 Hz, 1H), 7.12 (d, J = 7.5 Hz, 1H), 7.88 (d, J = 7.8 Hz, 1H), 8.78 (d, J = 9.5 Hz, 1H), 9.37 (m, 1H), 10.98 (m, 1H).

### EXEMPLE 21 :

### Préparation de (-)-6-chloro-2-hydroxy-N,N-diméthyl-3-(2-{[((S)-5-méthyl-furan-2-yl)-(tétrahydro-thiophen-2-yl)-méthyl]-amino}-3,4-dioxo-cyclobut-1-enylamino)-benzamide

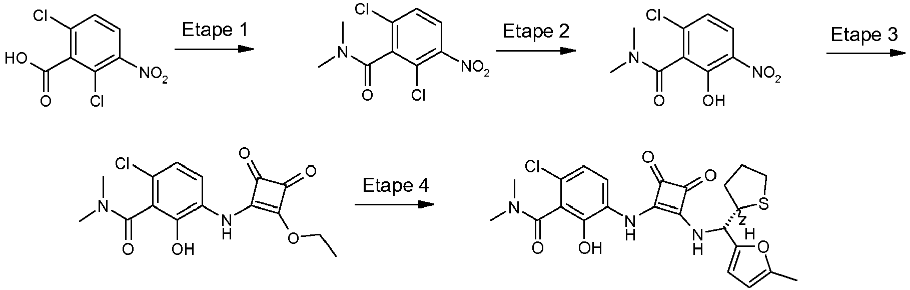

De manière analogue à l'EXEMPLE 10 (étapes 1), (-)-C-(5-méthyl-furan-2-yl)-C-(tétrahydro-thiophèn-2-yl)-méthylamine (énantiomère 1*)* a été préparé.

### Etape 1:

### 2,6-Dichloro-N,N-diméthyl-3-nitro-benzamide

Une solution de 10.0 g (42.4 mmol, 1éq) d'acide 2.6-dichloro-3-nitrobenzoïque dans 50 ml de chlorure de thionyle a été chauffée à reflux pendant 2 heures. Le milieu réactionnel a été concentré et coévaporé avec du toluène. Le résidu a été repris dans 35 ml de tétrahydrofuranne puis 48 ml d'une solution de diméthylamime dans le tétrahydrofuranne a été ajoutée goutte à goutte. Après 20 minutes d'agitation à température ambiante, de l'eau a été rajoutée ainsi que de l'acétate d'éthyle. La phase organique a été lavée à l'eau, séchée sur sulfate de sodium anhydre, filtrée et concentrée. 11.36 g de 2,6-dichloro-N,N-diméthyl-3-nitro-benzamide ont été obtenus sous forme d'une huile jaune. Rendement quantitatif.

### Etape 2:

### 6-Chloro-2-hydroxy-N,N-diméthyl-3-nitro-benzamide

A une suspension de 7.16 g (179.01 mmol; 4.3 éq) d'hydrure de sodium dans 250 ml de tétrahydrofuranne refroidie à 0°C ont été rajoutés 3.2 ml (177.6 mmol, 4.2 éq) d'eau et 11.04 g (41.96 mmol; 1.0 éq) de 2,6-dichloro-N,N-diméthyl-3-nitro-benzamide (41.96 mmol; 1.00 eq.) en solution dans 130.00 ml de tétrahydrofuranne. Après 10 minutes, le milieu réactionnel a été agité à température ambiante pendant 19 heures. Le milieu réactionnel a été hydrolysé avec une solution aqueuse d'acide chlorhydrique 1N et extrait à l'acétate d'éthyle. La phase organique a été lavée avec une solution aqueuse d'acide chlorhydrique 1N, séchée sur sulfate de sodium anhydre, filtrée et concentrée. Le résidu (11.82 g) a été chromatographié sur gel de silice (colonne prépackée de 300 g, éluant heptane/acétate d'éthyle, de 40 à 80% en acétate d'éthyle, 150 ml/min). 6.10 g de 6-chloro-2-hydroxy-N,N-dimethyl-3-nitro-benzamide ont été obtenus sous forme d'un solide jaune. Rendement = 59%

### Etape 3:

### 6-Chloro-3-(2-éthoxy-3,4-dioxo-cyclobut-1-enylamino)-2-hydroxy-N,N-diméthyl-benzamide

Une solution de 5.96 g (24.4 mmol, 1éq) de 6-chloro-2-hydroxy-N,N-diméthyl-3-nitro-benzamide dans 100 mL de méthanol en présence de 0.58 g d'oxyde de platinium hydraté a été agité sous pression atmosphérique d'hydrogène pendant 3 heures. Le milieu réactionnel a été filtré sur célite et le filtrat a été concentré. La solution obtenue a été ajoutée goutte à goutte sur 8.0 g (48.8 mmol, 2 éq) de 3.4-diéthoxy-3-cyclobutène-1.2-dione en solution dans 50 mL de méthanol. Le milieu réactionnel a été agité à température ambiante pendant 18 heures. Le solvant a été évaporé et le résidu a été chromatographié sur gel de silice (colonne prépackée de 300 g, éluant heptane/acétone, de 50 à 100% d'acétone). 4.42 g de 6-chloro-3-(2-éthoxy-3,4-dioxo-cyclobut-1-enylamino)-2-hydroxy-N,N-diméthyl-benzamide ont été obtenus sous forme d'un solide beige. Rendement = 54%

### Etape 4:

### (-)-6-Chloro-2-hydroxy-N,N-diméthyl-3-(2-{[((S)-5-méthyl-furan-2-yl)-(tétrahydro-thiophen-2-yl)-méthyl]-amino}-3,4-dioxo-cyclobut-1-enylamino)-benzamide

Un mélange de 560 mg (2.83 mmol, 1.2 éq) de (-)-C-(5-méthyl-furan-2-yl)-C-(tétrahydro-thiophèn-2-yl)-méthylamine (énantiomère 1, préparé dans l'EXEMPLE 10, étape 1) et de 800 mg (2.36 mmol, 1 éq) de 6-chloro-3-(2-éthoxy-3,4-dioxo-cyclobut-1-enylamino)-2-hydroxy-N,N-diméthyl-benzamide dans 50 mL de méthanol a été chauffé à 50°C pendant 23 heures. Le méthanol a été évaporé et le résidu a été repris avec de l'acétate d'éthyle et lavé
avec une solution aqueuse de dihydrogénophosphate de sodium 1M. La phase organique a été séchée sur sulfate de sodium anhydre, filtrée et concentrée. Le résidu (0.90 g) a été chromatographié sur gel de silice (colonne prépackée de 120 g, éluant dichlorométhane/méthanol, de 0 à 10% en méthanol. 530 mg de (-)-6-Chloro-2-hydroxy-N,N-diméthyl-3-(2-{[((S)-5-méthyl-furan-2-yl)-(tétrahydro-thiophen-2-yl)-méthyl]-amino}-3,4-dioxo-cyclobut-1-enylamino)-benzamide ont été obtenus sous forme d'un solide marron. Rendement = 45%. Tf = 153-154°C.
RMN ¹H (DMSO-d6, 400 MHz) : 1.75-1.95 (m, 2H), 2.00-2.10 (m, 2H), 2.26 (s, 3H), 2.73-2.85 (m, 5H), 3.00 (s, 1H), 3.75-3.90 (m, 1H), 5.15-5.22 (m, 1H), 6.05 (s, 1H), 6.27-6.30 (m, 1H), 6.98 (dl, J = 8.5 Hz, 1H), 7.73-7.77 (m, 1H), 8.73-8.78 (m, 1H), 9.37 (m, 1H), 9.90-10.30 (m, 1H).

### EXEMPLE 22 :

### Préparation de (-)-3-[4-chloro-2-hydroxy-3-(4-méthyl-pipérazine-1-sulfonyl)-phénylamino]-4-{[(5-méthyl-furan-2-yl)-(tétrahydro-thiophen-2-yl)-méthyl]-amino}-cyclobut-3-ène-1,2-dione

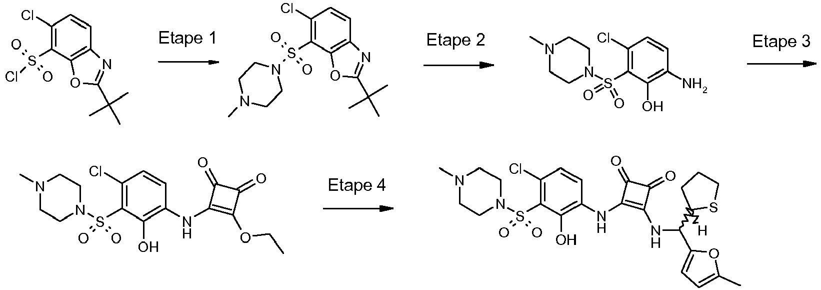

De manière analogue à l'EXEMPLE 10 (étapes 1), (-)-C-(5-méthyl-furan-2-yl)-C-(tétrahydro-thiophèn-2-yl)-méthylamine (énantiomère 1*)* a été préparé.

### Etape 1 :

### 2-tert-Butyl-6-chloro-7-(4-méthyl-pipérazine-1-sulfonyl)-benzooxazole

1.62 ml (11.68 mmol; 1.2 éq) de triéthylamine suivis de 1.20 ml (10.71 mmol; 1.1 éq) de 1-méthylpipérazine ont été ajoutés sur une solution de 3.0 g (9.73 mmol; 1.0 éq) de chlorure de 2-tert-butyl-6-chloro-benzoxazole-7-sulfonyle (commercial ?) dans 45 ml de tétrahydrofuranne. Le milieu réactionnel a été agité à température ambiante pendant 2 heures. De l'eau a été rajoutée et le milieu réactionnel a été extrait à l'acétate d'éthyle. Les phases organiques ont été réunies, séchées sur sulfate de magnésium, filtrées et évaporées. 3.57 g de 2-tert-butyl-6-chloro-7-(4-méthyl-pipérazine-1-sulfonyl)-benzooxazole ont été obtenus sous forme d'une mousse collante brune. Rendement = 98%

### Etape 2 :

### 6-Amino-3-chloro-2-(4-méthyl-pipérazine-1-sulfonyl)-phénol

4.27 ml (0.08 mol; 1.20 V) d'acide sulfurique dilué dans 4.3 ml d'eau ont été ajoutés goutte à goutte sur 3.56 g de 2-tert-butyl-6-chloro-7-(4-méthyl-pipérazine-1-sulfonyl)-benzooxazole (0.01 mol; 1.0 éq) en solution dans 15 ml de 1,4-dioxanne. Le milieu réactionnel a été chauffé à reflux pendant 6 heures et demie. Le milieu réactionnel a été concentré et de la soude 1N a été ajoutée (jusqu'à pH 7). La solution a été extraite au dichlorométhane. Les phases organiques ont été rassemblées, séchées sur sulfate de magnésium, filtrées et évaporées. Le résidu obtenu a été chromatographié sur gel de silice, éluant acétate d'éthyle/dichloro méthane 95/5. 2.0 g de 6-amino-3-chloro-2-(4-méthyl-pipérazine-1-sulfonyl)-phénol ont été obtenus sous forme d'une épaisse huile brune. Rendement = 68%.

### Etape 3 :

### 3-[4-Chloro-2-hydroxy-3-(4-méthyl-pipérazine-1-sulfonyl)-phénylamino]-4-éthoxy-cyclobut-3-ène-1,2-dione

Un mélange de 1.98 g (6.5 mmol, 1 éq) de 6-amino-3-chloro-2-(4-méthyl-pipérazine-1-sulfonyl)-phénol et de 2.20 g (48.8 mmol, 2 éq) de 3.4-diéthoxy-3-cyclobutène-1.2-dione a été mis en solution dans 20 mL d'éthanol. Le milieu réactionnel a été chauffé à 50°C pendant 16 heures. L'insoluble a été filtré, lavé à l'éthanol et séché sous vide à 45°C. 2.05 g de 3-[4-chloro-2-hydroxy-3-(4-méthyl-pipérazine-1-sulfonyl)-phénylamino]-4-éthoxy-cyclobut-3-ène-1,2-dione ont été obtenus sous forme d'un solide jaune. Rendement = 74%

### Etape 4 :

### (-)-3-[4-Chloro-2-hydroxy-3-(4-méthyl-pipérazine-1-sulfonyl)-phénylamino]-4-{[(5-méthyl-furan-2-yl)-(tétrahydro-thiophen-2-yl)-méthyl]-amino}-cyclobut-3-ène-1,2-dione

Un mélange de 280 mg (1.4 mmol, 1.2 éq) de (-)-C-(5-méthyl-furan-2-yl)-C-(tétrahydro-thiophen-2-yl)-méthylamine (énantiomère 1, préparé dans l'EXEMPLE 10, étape 1) et de 500 mg (1.16 mmol, 1 éq) de 3-[4-chloro-2-hydroxy-3-(4-méthyl-pipérazine-1-sulfonyl)-phénylamino]-4-éthoxy-cyclobut-3-ène-1,2-dione dans 20 mL de méthanol a été chauffé à 50°C pendant 20 heures. Le méthanol a été évaporé et le résidu a été repris avec du dichlorométhane et lavé avec une solution aqueuse de dihydrogénophosphate de sodium 1M. La phase organique a été séchée sur sulfate de sodium anhydre, filtrée et évaporée. Le résidu a été chromatographié sur gel de silice élué au dichlorométhane/méthanol (98/2). 410 mg de 3-[4-chloro-2-hydroxy-3-(4-méthyl-pipérazine-1-sulfonyl)-phénylamino]-4-{[(5-méthyl-furan-2-yl)-(tétrahydro-thiophen-2-yl)-méthyl]-amino}-cyclobut-3-ène-1,2-dione ont été obtenus sous forme d'un solide jaune vif. Rendement = 61%.
RMN ¹H (DMSO-d6, 400 MHz) : 1.81-1.85 (m, 1H) ; 1.90-1.93 (m, 1H) ; 1.99-2.09 (m,2H) ; 2.26 (s,3H) ; 2.41 (s,3H) ; 2.68 (se, 4H) ; 2.74-2.84 (m, 2H) ; 3.36 (se, 4H) ; 3.83-3.88 (m, 1H) ; 5.19 (t, j=9.6Hz, 1H) ; 6.06 (m, 1H) ; 6.29 (m, 1H) ; 6.80 (se,1H) ; 7.90 ( d, j=8.6Hz, 1H) ; 8.91 ( d, j=9.6Hz, 1H) ; 9.48 (s, 1H) ; 10.00 (se, 1H).

### TESTS BIOLOGIQUES

### EXEMPLE 23 : Affinité in vitro

L'affinité *In Vitro* des composés de la présente invention pour les récepteurs CXCR1 et CXCR2 a été déterminée sur un test fonctionnel de type recrutement de la β-arrestine après activation du récepteur.
Il a été démontré que l'activation par CXCL8 du récepteur CXCR2 dans les cellules de la lignée PathHunter HEK293-CXCR2 ou du récepteur CXCR1 dans les cellules de la lignée U2OS h CXCR1 β-arrestine conduit au recrutement de la β-arrestine (Richardson, R. M., R. J. Marjoram, L. S. Barak, R. Snyderman. 2003. Role of the cytoplasmic tails of CXCR1 and CXCR2 in mediating leukocyte migration, activation, and regulation. J. Immunol. 170 : 2904.- 2911.)
Pour évaluer l'interaction directe du récepteur CXCR2 ou CXCR1 avec la β-arrestine 2, un test de recrutement β-arrestine 2 pour CXCR2 ou CXCR1 basé sur la complémentation de l'enzyme β-galactosidase (Olson KR, Eglen RM. Beta galactosidase complementation: a cell-based luminescent assay platform for drug discovery. Assay Drug Dev Technol. 2007 Feb; 5(1) ; 137-44), tel qu'établi par DiscoveRx Corporation a été utilisé. La stimulation de ces deux lignées cellulaires avec CXCL8 (10 nM) induit le recrutement de la β-arrestine 2, comme indiqué par une augmentation significative du facteur d'induction. Tous les antagonistes CXCR2 ont été testés de manière dose-dépendante et la concentration correspondant à 50% d'inhibition de la réponse a été déterminée (IC₅₀ = concentration de demi inhibition).

Test de Recrutement de la β-arrestine : les cellules « PathHunter HEK293-CXCR2 » ou « U2OS hCXCR1 β-arrestine » (DiscoveRx Corporation) ont été ensemencées une nuit à 10 000 cellules / puits (384 puits format) dans 20 µl de milieu Opti MEM I. Une préincubation avec l'antagoniste ou le véhicule de 30 min à 37 ° C et 5% de CO2 a été suivie par 60 minutes de stimulation par CXCL8 à 37 ° C et 5% de CO2. Les cellules ont ensuite été placées à température ambiante pendant 30 minutes. Le réactif de détection PathHunter (DiscoveRx Corporation) a été ajouté. Après une incubation de 60 min à température ambiante, la β-galactosidase induite par la luminescence lors de l'interaction β-arrestine-CXCR2 a été mesurée pour 0,3 s dans un Envision 2102 Multilabel Reader (PerkinElmer Life and Analytical Sciences). Les données ont été analysées par une procédure de courbe non linéaire utilisant le logiciel d'exploitation XLFit4 (IDBS) et les IC50 ont été déterminées.

| **Composé N° exemple** | **CXCR1 (IC50 ; nM)** | **CXCR2 (IC50 ; nM)** |
|---|---|---|
| 1 | 244 | 53 |
| (Enantiomère 2 du diastéréoisomère 2) | | |
| 1 | 2075 | 89 |
| (Diastéréoisomère 1) | | |
| 1 | 819 | 94 |
| (Diastéréoisomère 2) | | |
| 1 | 9999* | 9999* |
| (Enantiomère 1 du diastéréoisomère 2) | | |
| 2 | 77 | 15 |
| (Enantiomère 1 du diastéréoisomère 2) | | |
| 2 | 44 | 35 |
| (diastéréoisomère 2) | | |
| 2 | 692 | 56 |
| (Enantiomère 2 du diastéréoisomère 1) | | |
| 2 | 1564 | 134 |
| (Enantiomère 2 du diastéréoisomère 2) | | |
| 2 | 661 | 135 |
| (diastéréoisomère 1) | | |
| 2 | 4554 | 273 |
| (Enantiomère 1 du diastéréoisomère 1) | | |
| 3 | 552 | 195 |
| (Enantiomère 1) | | |
| 3 | 812 | 221 |
| (Mélange d'énantiomères 1 et 2) | | |
| 3 | 8907 | 982 |
| (Enantiomère 2) | | |
| 4 | 1892 | 109 |
| 5 | 711 | 106 |
| 6 | 108 | 30 |
| 7 | 171 | 37 |
| (diastéréoisomère 2) | | |
| 7 | 859 | 86 |
| (diastéréoisomère 1) | | |
| 8 | 85 | 47 |
| (diastéréoisomère 2) | | |
| 8 | 508 | 115 |
| (diastéréoisomère 1) | | |
| 9 | 89 | 80 |
| (diastéréoisomère 2) | | |
| 9 | 580 | 153 |
| (diastéréoisomère 1) | | |
| 10 | 20 | 16 |
| (diastéréoisomère 1) | | |
| 10 | 1096 | 272 |
| (diastéréoisomère 2) | | |
| 11 | 1322 | 176 |
| 12 | 483 | 83 |
| 13 | 1148 | 170 |
| 14 | 9999* | 1253 |
| (diastéréoisomère 2) | | |
| 14 | 9999* | 9999* |
| (diastéréoisomère 1) | | |
| 15 | 3808 | 294 |
| 16 | 652 | 23 |
| 17 | 384 | 44 |
| 18 | 36 | 15 |
| 19 | 42 | 20 |
| 20 | 43 | 21 |
| 21 | 113 | 69 |
| 22 | 30 | 17 |

| | | |
|---|---|---|
| * : 9999 signifie non actif | | |

### EXEMPLE 24 : Polypharmacologie : « profiling réceptoriel »

**Mesure du flux calcique sur cellules** : Les expériences ont été réalisées sur la plateforme FLIPR TETRA® de Molecular Devices. Après une lecture du niveau basal, les composés ont été ajoutés aux cellules exprimant le récepteur aux chimiokines d'intérêt et l'activité agoniste a été lue à 10 secondes. Après une incubation supplémentaire de 10 minutes, les cellules ont été activées, avec une concentration équivalente à l'AC80, par un agoniste de référence afin de détecter si ce composé présente une activité antagoniste.
Chaque lignée cellulaire exprimant un récepteur aux chimiokines a été établie sur la base de cellule Chem-1 exprimant de manière stable la forme recombinante du récepteur aux chimiokines ainsi qu'une protéine G associée, dans le but de coupler le récepteur à la voie de signalisation du calcium. 21 récepteurs appartenant à la famille des récepteurs aux chimiokines (CCRs et CXCRs) ont été analysés. Tous les antagonistes CXCR2 ont été testés de manière dose-dépendante et la concentration correspondant à 50% d'inhibition de la réponse a été déterminée (IC₅₀).

Les composés répondant à la formule générale (I) ainsi que le composé de Schering SCH-527123 ont été profilés sur un panel de 20 récepteurs aux chimiokines. Il ressort de ce profilage que les composés répondant à la formule générale (I) présentent une polypharmacologie. Par exemple, le composé de l'exemple 1 (Enantiomère 2 du diastéréoisomère 2) inhibe les récepteurs CCR4, CCR6, CCR7 et CXCR3 avec des valeurs d'IC50 respectives de 410nM, 2.0nM, 8.7µM et 1.3nM. Le composé de l'exemple 2 (diastéréoisomère 2) inhibe les récepteurs CCR4, CCR6, CCR7, CCR8, CXCR3 et FPR1 avec des IC50 respectives de 52nM, 4.4nM, 1.5µM, 620nM, 1.7µM et 6.5µM.
Le composé de Schering SCH-527123 a été inactif ou très peu actif sur l'ensemble de ces récepteurs. Il a été également extrêmement intéressant de noter la forte activité du composé de l'exemple 1 (Enantiomère 2 du diastéréoisomère 2) et du composé de l'exemple 2 (diastéréoisomère 2) sur CXCR3 et CCR6.

| | IC50 (nM) | | |
|---|---|---|---|
| Antagoniste | CCR4 | CCR6 | CXCR3 |
| Exemple 1 | 410 | 2,0 | 1,3 |
| (Enantiomère 2 du diastéréoisomère 2) | | | |
| Exemple 2 | 52 | 8 | 1700 |
| (diastéréoisomère 2) | | | |
| Exemple 1 (diastéréoisomère 2) | ND | 2,2 | 55 |
| Exemple 2 | 250 | 6 | 216 |
| (Enantiomère 1 du diastéréoisomère 2) | | | |
| Exemple 6 | ND | 9,7 | 140 |
| Exemple 10 | 39 | 1,6 | 93 |
| Exemple 18 | 97 | 60 | 450 |
| Exemple 19 | 8,1 | 11 | 510 |
| Exemple 20 | 7,2 | 8,8 | 180 |
| Exemple 22 | 9,4 | 2,2 | 150 |

| | | | |
|---|---|---|---|
| ND : Non Déterminé | | | |

### EXEMPLE 25 : Constante de dissociation

La détermination des constantes de demi-dissociation des antagonistes CXCR2 a été basée sur le modèle *In Vitro* de recrutement de la β-Arrestin précédemment décrit : les cellules « PathHunter HEK293-CXCR2 » (DiscoveRx Corporation) ont été ensemencées une nuit à 20 000 cellules/puits (au format 96 puits) dans 100µL/puits de milieu de culture OptiMEM-1% SVF. Une préincubation avec l'antagoniste ou le véhicule a été réalisée pendant 1 heure à 37°C-5% CO₂. Les cellules ont été ensuite lavées 3 fois avec 100µL/puits de milieu OptiMEM-1% SVF puis une incubation variable (0h-0.5h-6h-12h-24h) des cellules à 37°C-5% CO₂ a été réalisée. Les cellules ont été ensuite stimulées par 4nM de CXCL8 à 37°C-5% CO₂ pendant 1h30. Le réactif de détection PathHunter (DiscoveRx Corporation) a été ajouté à raison de 50µL/puits. Après une incubation de 60 minutes à température ambiante, la luminescence émise, par l'hydrolyse du substrat par la β-galactosidase complémentée lors de l'interaction β-Arrestin-CXCR2, a été mesurée pendant 0,3 secondes/puits avec un Envision Multilabel Reader (PerkinElmer Life and Analytical Sciences). Les données ont été analysées par une procédure de courbe non linéaire utilisant le logiciel d'exploitation XLFit4 (IDBS) et les IC50 ont été déterminées. Le temps de demi-dissociation a été déterminé sur une régression de type y=(A*(1-exp(((-1)*B)*x))) (où x=temps et y=luminescence normalisée) à concentration saturante en antagoniste.

Résultats : Les molécules décrites dans les exemples de l'invention ont été comparées à la molécule SCH-527123 (décrite pour avoir une dissociation pseudo-irréversible) (Pharmacological Characterization of SCH-527123, a Potent Allosteric CXCR1/CXCR2 Antagonist. JPET 322:477-485, 2007).

### EXEMPLE 26 : A/ Stabilités métaboliques en microsomes hépatiques

Des microsomes hépatiques (Becton Dickinson) ont été incubés à une concentration de protéine de 0.5 mg/mL dans le milieu réactionnel.

Le milieu réactionnel des microsomes a été composé de tampon Phosphate pH : 7.4 à 100 mM, de MgCl₂ à 100 mM. (50/50), d'un système de génération d'ATP composé d'un mélange de Nicotinamide Adénine Di-Phosphate (NADP), de Glucose-6-Phosphate (G6P) à 1 mg/mL et de Glucose-6-Phosphate Deshydrogénase (G6PDH) à 4 U/mL. Les composés ont été testés à 1 µM (DMSO 0.1%)

Les prélèvements de milieu d'incubation après ajout des microsomes ont été réalisés aux temps 5, 10, 15, 30 et 60 minutes. A chaque temps, la réaction métabolique a été stoppée par ajout de méthanol (1 volume milieu incubation/3 volumes de méthanol). La disparition du produit parent a été mesurée par analyse LC/MS/MS. Le temps pour lequel 50% de produit parent a disparu (T1/2) a été calculé à partir de la cinétique de disparition du produit parent en fonction du temps.

| Antagoniste | Temps de demi-vie (min) |
|---|---|
| SCH-527123 | Stable (>60min) |
| Exemple 2 (diastéréoisomère 2) | 11 |
| Exemple 1 (diastéréoisomère 2) | 27 |
| Exemple 2 (diastéréoisomère 1) | 9 |
| Exemple 3 (couple d'énantiomères 1 et 2) | 3 |
| Exemple 6 | 5 |
| Exemple 10 | 8 |
| Exemple 18 | 15 |
| Exemple 19 | 8 |
| Exemple 20 | 17 |
| Exemple 22 | 7 |

| | |
|---|---|
| **B**/ Stabilités métaboliques en hépatocytes. | |

Les Hépatocytes humains ont été fournis par Biopredic en plaque 24 puits. Après 48 h d'adaptation en culture, les hépatocytes ont été placés dans un milieu de traitement contenant 0.1% Sérum Albumine Bovine et les composés ont été testés à 1 µM (DMSO de 0.1%)

Les prélèvements de milieu d'incubation après ajout du composé à tester ont été réalisés aux temps t=0, 1, 2, 4, 6 et 24 heures.

A chaque temps, la réaction métabolique a été stoppée par ajout de méthanol (1 volume milieu incubation/3 volumes de méthanol). La disparition du produit parent a été mesurée par analyse LC/MS/MS. Le temps pour lequel 50% (T1/2) de produit parent a disparu a été calculé à partir de la cinétique de disparition du produit parent en fonction du temps.

| Antagoniste | Temps de demi-vie (min) |
|---|---|
| SCH-527123 | 900 |
| Example 1 (Enantiomère 2 du diastéréoisomère 2) | 300 |
| Exemple 1 (diastéréoisomère 2) | 539 |
| Exemple 2 (diastéréoisomère 1) | 106 |
| Example 2 (Enantiomère 2 du diastéréoisomère 2) | 229 |
| Exemple 3 (couple d'énantiomères 1 et 2) | 141 |
| Exemple 2 (énantiomère 1 du diastéréoisomère 2) | 213 |
| Exemple 10 | 124 |
| Exemple 18 | 82 |
| Exemple 19 | 214 |
| Exemple 20 | 211 |
| Exemple 22 | 49 |

## Revendications

1. Composé di-substitués de la diamino-3,4- cyclobutène-3-dione-1,2 répondant à la formule générale (I) suivante ou un de ses sels ou solvates pharmaceutiquement acceptable: 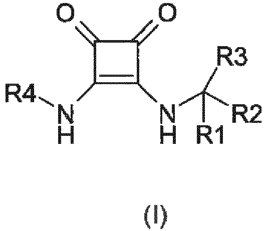 dans laquelle,
R1 représente un atome d'hydrogène ou un méthyle,
R2 représente un cycle à cinq atomes choisi parmi les structures (1), (2), (3) et (4) suivantes: 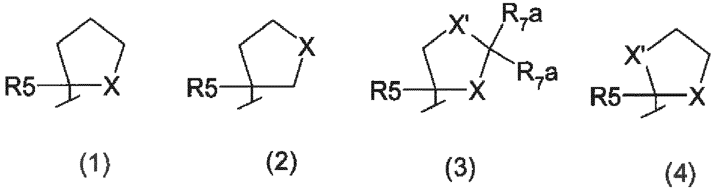 dans lesquelles R5, R7a, X et X' ont la signification donnée ci-après,
R3 représente un cycle aromatique ou hétéroaromatique sélectionné dans le groupe constitué par les cycles répondant aux formules (a) à (o) suivantes : 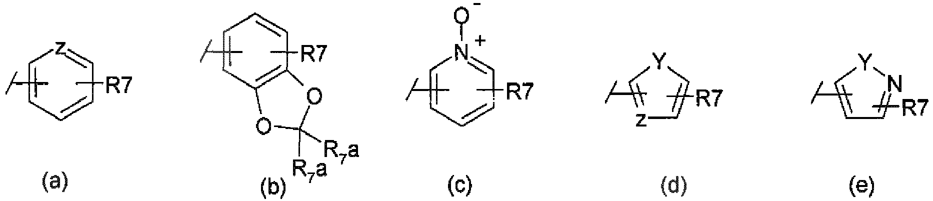 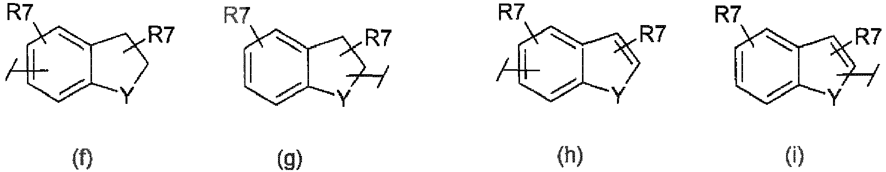 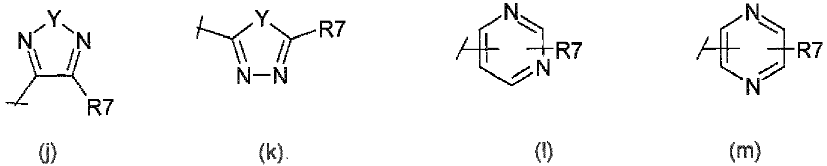 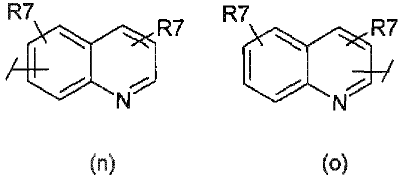 dans lesquelles R7, R7a, Y et Z ont la signification donnée ci-après, étant précisé que les cycles (a) (o) peuvent éventuellement porter plusieurs groupes R7, identiques ou différents, le nombre total de tels groupes R7 étant au maximum égal au nombre d'atomes substituables du cycle ;
R4 représente un cycle aromatique ou hétéroaromatique sélectionné dans le groupe constitué par les cycles répondant aux formules (p) à (z) et (aa) à (ak) suivantes : 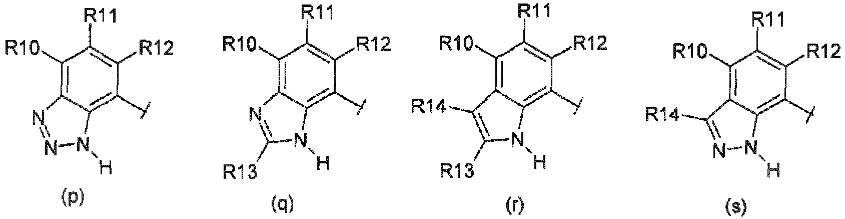 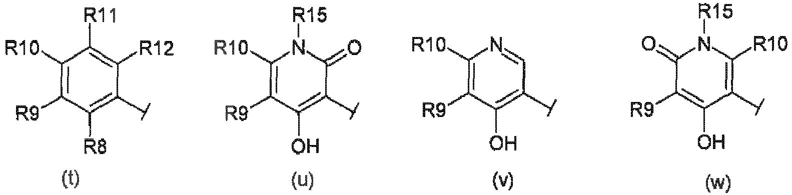 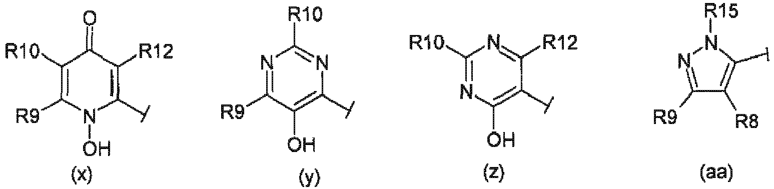 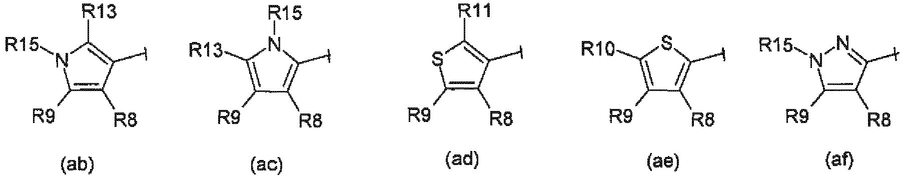 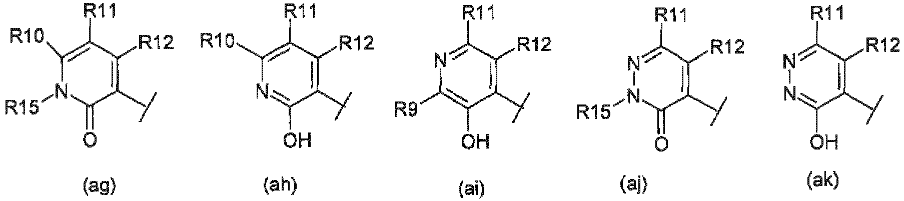 dans lesquelles R7, R8, R9, R10, R11, R12, R13, R14 et R15 ont la signification donnée ci-après,
R5 représente un atome d'hydrogène, un atome de fluor, un radical alkyle ayant de 1 à 5 atomes de carbones ou un radical alkyle fluoré ou perfluoré comportant de 1 à 5 atomes de carbones,
R6 représente un atome d'hydrogène, un radical -COOtBu ou un radical -COOBn,
R7 représente un halogène, un radical -R16, -CF3, -COR16, -OR16, -NR16R17, -NO2, -CN, - SO2R16, -SO2NR16R17, -NR16COR17, -CONR16R17, -NR16CO2R17 ou -CO2R16,
R7a représente un atome d'hydrogène ou un radical alkyle ayant de 1 à 5 atomes de carbones,
R8 représente un atome d'hydrogène, un atome d'halogène, un radical -OH, un radical -SH,-CONHOR16, -CONR160H, -NR16R17, -SO3H, -OCOR16, -NHSO2R16, -SO2NR16R17,-NHCOR16, -CONR16R17, -NR16CO2R17, -NHSO2NR16R17, -CO2R16, pyrrolyle, imidazolyle, triazolyle ou tetrazolyle,
R9, R10, R11 et R12 sont identiques ou différents et sont indépendamment choisis dans le groupe constitué par un hydrogène, un atome d'halogène, un radical alkyle, alkoxy, -CF3, -OCF3, -OH,-NO2, -CN, -SO2R16, -SO2NR16R17, -NR16COR17, -NR16CO2R17, -CONR16R17, -COR16 ou-CO2R16,
ou bien encore, lorsque deux des radicaux R9, R10, R11 et R12 se trouvent en position ortho sur un cycle aromatique ou hétéroaromatique sélectionné dans le groupe constitué par les cycles répondant aux formules (p) à (z) et (aa) à (ak) ci-dessus, alors ils peuvent former ensemble, avec la liaison qui les réunit, un cycle aryle, hétéroaryle, cycloalkyle ou hétérocycloalkyle,
R13 et R14 sont identiques ou différents et sont indépendamment choisis dans le groupe constitué par un atome d'hydrogène, un atome d'halogène, un radical alkyle, -CF3, -OCF3, -OH, -SH, -CN,-SO2R16, -SO2NR16R17, -NHSO2NR16R17, -NR16R17, -NR16CONR16R17, -NR16COR17,-NR16CO2R17, -CONR16R17, -COR16 ou -CO2R16,
R15 représente un atome d'hydrogène, un radical -OH, -S02R16, -COR16, -CO2R16, aryle, heteroaryle, arylalkyle, heteroarylalkyle, alkyle, cycloalkyle ou cycloalkylalkyle,
R16 et R17 sont identiques ou différents et sont indépendamment choisis dans le groupe constitué par un atome d'hydrogène, un radical aryle, heteroaryle, arylalkyle, heteroarylalkyle, alkyle, alkyle fluoré ayant de 1 à 5 atomes de carbone, cycloalkyle ou cycloalkylalkyle, un groupe -CH2COOR18 dans lequel R18 représente un radical alkyle ayant de 1 à 5 atomes de carbone,
ou bien encore lorsque R16 et R17 sont portés par un même atome d'azote ils forment un hétérocycle ayant entre 3 et 7 chainons et comportant éventuellement un ou deux hétéroatomes choisis parmi l'oxygène , le soufre et l'azote en plus de l'atome d'azote commun par lequel ils sont portés, ledit hétérocycle pouvant être substitué par un groupe alkyle ayant de 1 à 5 atomes de carbone ou un groupe -COOR18 dans lequel R18 représente un radical alkyle ayant de 1 à 5 atomes de carbone ;.
X et X', identiques ou différents, représentent un atome d'oxygène, un atome de soufre, ou un atome d'azote substitué par un radical R6,
Y représente un atome d'oxygène, un atome de soufre, ou un atome d'azote substitué par un radical R15, et
Z représente un atome de carbone ou d'azote

2. Composé selon la revendication 1, **caractérisé en ce que** dans la formule (I) précitée:
R1 représente un atome d'hydrogène,
R2 représente un cycle à cinq chainons choisi parmi les structures (1), (2) et (3) suivantes: 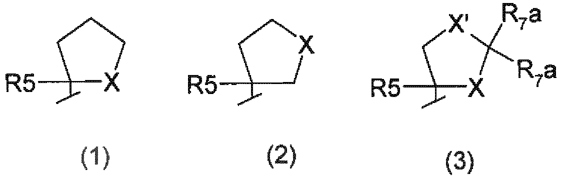 dans lesquelles R5, R7a, X et X' ont la signification donnée ci-après,
R3 représente un cycle aromatique ou hétéroaromatique sélectionné dans le groupe constitué par les cycles répondant aux formules (a), (b) et (d) suivantes : 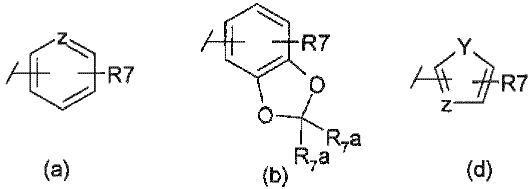 dans lesquelles R7, R7a, Y et Z ont la signification donnée ci-après, étant précisé que les cycles (a), (b) et (d) peuvent éventuellement porter plusieurs groupes R7, identiques ou différents, le nombre total de tels groupe R7 étant au maximum égal au nombre d'atomes substituables du cycle ;
R4 représente un cycle aromatique ou hétéroaromatique sélectionné dans le groupe constitué par les cycles répondant aux formules (p), (q), (t), (z), (ad), (ag) et (ah) suivantes : 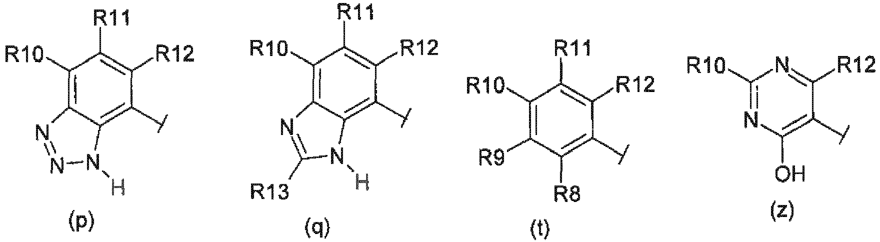 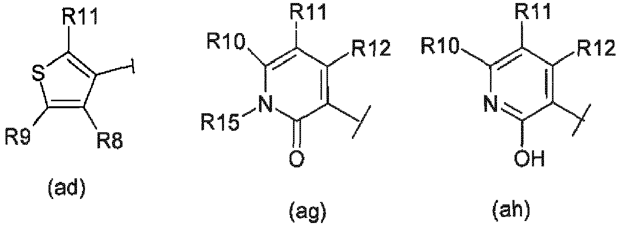 dans lesquelles R8, R9, R10, R11, R12, R13 et R15 ont la signification donnée ci-après,
R5 représente un atome d'hydrogène, un atome de fluor, un radical alkyle ayant de 1 à 5 atomes de carbones ou un radical alkyle fluoré ou perfluoré comportant de 1 à 5 atomes de carbones,
R6 représente un atome d'hydrogène, un radical -COOtBu ou un radical -COOBn,
R7 représente un halogène, un radical R16, -CF3, -COR16, -OR16, -NR16R17, -NO2, -CN,-SO2R16, -SO2NR16R17, -NR16COR17, -CONR16R17, -NR16CO2R17 ou -CO2R16,
R7a représente un hydrogène ou un radical alkyle ayant de 1 à 5 atomes de carbones,
R8 représente un atome d'hydrogène, un radical -OH, -SH, -CONHOR16, -CONR16OH,-NR16R17, -SO3H, -OCOR16, -NHSO2R16, -SO2NR16R17, -NHCOR16, -CONR16R17,-NR16CO2R17, -NHSO2NR16R17, -CO2R16, pyrrolyle, imidazolyle, triazolyle ou tétrazolyle,
R9, R10, R11 et R12 sont identiques ou différents et sont indépendamment choisis dans le groupe constitué par un atome d'hydrogène, un atome d'halogène, un radical alkyle, alkoxy, -CF3, -OCF3, - OH, -NO2, -CN, -SO2R16, -SO2NR16R17, -NR16COR17, -NR16CO2R17, -CONR16R17, -COR16 ou -CO2R16,
ou bien encore, lorsque deux des radicaux R9, R10, R11 est R12 se trouvent en position ortho sur un cycle aromatique ou hétéroaromatique sélectionné dans le groupe constitué par les cycles répondant aux formules (p), (q), (t), (z), (ad), (ag) et (ah) ci-dessus, alors ils peuvent former ensemble, avec la liaison qui les réunit, un cycle aryle, hétéroaryle, cycloalkyle ou hétérocycloalkyle,
R13 est choisi dans le groupe constitué par un atome d'hydrogène, un atome d'halogène, un radical alkyle, -CF3, -OCF3, -OH, -SH, -CN, -SO2R16, -SO2NR16R17, -NHSO2NR16R17, -NR16R17,-NR16CONR16R17, -NR16COR17, -NR16CO2R17, -CONR16R17, -COR16 ou -CO2R16,
R15 représente un atome d'hydrogène, un radical -OH, -SO2R16, -COR16, -CO2R16, aryle, hétéroaryle, arylalkyle, hétéroarylalkyle, alkyle, cycloalkyle ou cycloalkylalkyle,
R16 et R17 sont identiques ou différents et sont indépendamment choisis dans le groupe constitué par un atome d'hydrogène, un radical aryle, heteroaryle, arylalkyle, heteroarylalkyle, alkyle, alkyle fluoré ayant de 1 à 5 atomes de carbone, cycloalkyle ou cycloalkylalkyle, un groupe -CH2COOR18 dans lequel R18 représente un radical alkyle ayant de 1 à 5 atomes de carbone,
ou bien encore lorsque R16 et R17 sont portés par un même atome d'azote ils forment un hétérocycle ayant entre 3 et 7 chainons et comportant éventuellement un ou deux hétéroatomes choisis parmi l'oxygène , le soufre et l'azote en plus de l'atome d'azote commun par lequel ils sont portés, ledit hétérocycle pouvant être substitué par un groupe alkyle ayant de 1 à 5 atomes de carbone ou un groupe -COOR18 dans lequel R18 représente un radical alkyle ayant de 1 à 5 atomes de carbone;.
X et X', identiques ou différents, représentent un atome d'oxygène, un atome de soufre, ou un atome d'azote substitué par un radical R6,
Y représente un atome d'oxygène, un atome de soufre, ou un atome d'azote substitué par un radical R15, et
Z représente un atome de carbone ou d'azote.

3. Composé selon la revendication 1 ou 2, **caractérisé en ce que** dans la formule (I) précitée:
R1 représente un atome d'hydrogène,
R2 représente un cycle à cinq atomes de structure (1) suivante: 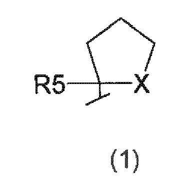 dans laquelle R5 et X ont la signification donnée ci-après,
R3 représente un cycle hétéroaromatique répondant à la formule (d) suivante : 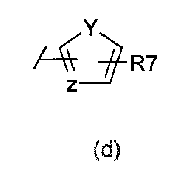 dans laquelle R7, Y et Z ont la signification donnée ci-après, étant précisé que le cycle (d) peut éventuellement porter plusieurs groupes R7, identiques ou différents, le nombre total de tels groupe R7 étant au maximum égal au nombre d'atomes substituables du cycle ;
R4 représente un cycle aromatique répondant à la formule (t) suivante : 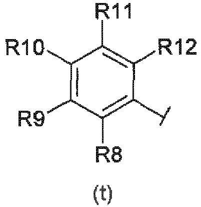 dans laquelle R8, R9, R10, R11 et R12 ont la signification donnée ci-après,
R5 représente un atome d'hydrogène, un atome de fluor, un radical alkyle ayant de 1 à 5 atomes de carbones ou un radical alkyle fluoré ou perfluoré comportant de 1 à 5 atomes de carbones,
R6 représente un atome d'hydrogène, un radical -COOtBu ou un radical -COOBn,
R7 représente un atome d'halogène, un radical R16, -CF3, -COR16, -OR16, -NR16R17, -NO2, -CN, -SO2R16, -SO2NR16R17, -NR16COR17, -CONR16R17, -NR16CO2R17 ou -CO2R16,
R8 représente un atome d'hydrogène, un radical -OH, -SH, -CONHOR16, -CONR16OH,-NR16R17, -SO3H, -OCOR16, -NHSO2R16, -SO2NR16R17, -NHCOR16, -CONR16R17,-NR16CO2R17, -NHSO2NR16R17, -CO2R16, pyrrolyle, imidazolyle, triazolyle ou tetrazolyle,
R9, R10, R11 et R12 sont identiques ou différents et sont indépendamment choisis dans le groupe constitué par un atome d'hydrogène, un atome d'halogène, un radical alkyle, alkoxy, -CF3, -OCF3, - OH, -NO2, -CN, -SO2R16, -SO2NR16R17, -NR16COR17, -NR16CO2R17, -CONR16R17, -COR16 ou -CO2R16,
ou bien encore, lorsque deux des radicaux R9, R10, R11 et R12 se trouvent en position ortho sur le cycle aromatique (t), ils peuvent former ensemble, avec la liaison qui les réunit, un cycle aryle, hétéroaryle, cycloalkyle ou hétérocycloalkyle,
R15 représente un atome d'hydrogène, un radical -OH, -SO2R16, -COR16, -CO2R16, aryle, hétéroaryle, arylalkyle, hétéroarylalkyle, alkyle, cycloalkyle ou cycloalkylalkyle,
R16 et R17 sont identiques ou différents et sont indépendamment choisis dans le groupe constitué par un atome d'hydrogène, un radical aryle, heteroaryle, arylalkyle, heteroarylalkyle, alkyle, alkyle fluoré ayant de 1 à 5 atomes de carbone, cycloalkyle ou cycloalkylalkyle, un groupe -CH2COOR18 dans lequel R18 représente un radical alkyle ayant de 1 à 5 atomes de carbone,
ou bien encore lorsque R16 et R17 sont portés par un même atome d'azote ils forment un hétérocycle ayant entre 3 et 7 chainons et comportant éventuellement un ou deux hétéroatomes choisis parmi l'oxygène , le soufre et l'azote en plus de l'atome d'azote commun par lequel ils sont portés, ledit hétérocycle pouvant être substitué par un groupe alkyle ayant de 1 à 5 atomes de carbone ou un groupe -COOR18 dans lequel R18 représente un radical alkyle ayant de 1 à 5 atomes de carbone ;.
X représente un atome d'oxygène, un atome de soufre, ou un atome d'azote substitué par un radical R6,
Y représente un atome d'oxygène, un atome de soufre, ou un atome d'azote substitué par un radical R15, et
Z représente un atome de carbone ou un d'azote.

4. Composition pharmaceutique comprenant une quantité efficace d'un composé ou d'un sel pharmaceutiquement acceptable dudit composé selon la revendication 1 en association avec un solvant ou un support pharmaceutiquement acceptable.

5. Composé selon la revendication 1 ou composition pharmaceutique selon la revendication 4 pour son utilisation en tant que médicament.

6. Composé selon la revendication 1 ou composition pharmaceutique selon la revendication 4 pour son utilisation dans le traitement de maladies médiées par les α-chimiokines, choisies parmi les maladies du groupe comprenant les dermatoses neutrophiliques, choisies parmi le psoriasis, les dermatites atopiques, l'acné, la rosacée, l'asthme, les maladies pulmonaire obstructives chroniques, les maladies respiratoires des adultes, l'arthrite, les maladies intestinales inflammatoires, la maladie de Crohn, le rejet de greffe, la mucoviscidose et les cancers cutanés.

7. Composé ou composition pharmaceutique selon la revendication 6 pour son utilisation dans le traitement de maladies dermatologiques comprenant les dermatoses neutrophiliques, choisies parmi le psoriasis, les dermatites atopiques, l'acné et la rosacée.

8. Composé selon la revendication 1 choisi dans la liste comprenant:
1/- 2-hydroxy-N,N-diméthyl-3-(2-{[(5-méthyl-furan-2-yl)-(2-méthyl-tétrahydrofuran-2-yl)-méthyl]-amino}-3,4-dioxo-cyclobut-1-enylamino)-benzamide
2/-2-hydroxy-*N*,*N*-dimethyl-3-(2-{[(5-méthyl-furan-2-yl)-(tétrahydro-thiophèn-2-yl)-méthyl]-amino}-3,4-dioxo-cyclobut-1-enylamino)-benzamide
3/- (S)-1-[2-fluoro-3-(2-{[(5-méthyl-furan-2-yl)-(tétrahydro-thiophèn-2-yl)-méthyl]-amino}-3,4-dioxo-cyclobut-1-énylamino)-benzoyl]-pyrrolidine-2-carboxylate de méthyle
4/- (S)-1-[2-fluoro-3-(2-{[(5-méthyl-furan-2-yl)-(tétrahydro-thiophen-2-yl)-méthyl]-amino}-3,4-dioxo-cyclobut-1-enylamino)-benzoyl]-pyrrolidine-2-carboxylate d'isopropyle
5/- (S)-1-[2-fluoro-3-(2-{[(5-méthyl-furan-2-yl)-(tétrahydro-thiophen-2-yl)-méthyl]-amino}-3,4-dioxo-cyclobut-1-enylamino)-benzoyl]-pyrrolidine-2-carboxylate d'éthyle
6/- (R)-1-[2-hydroxy-3-(2-{[(5-methyl-furan-2-yl)-(tetrahydro-thiophen-2-yl)-methyl]-amino}-3,4-dioxo-cyclobut-1-enylamino)-benzoyl]-pyrrolidine-2-carboxylate de méthyle
7/- (S)-1-[2-hydroxy-3-(2-{[(5-methyl-furan-2-yl)-(tetrahydro-thiophen-2-yl)-methyl]-amino}-3,4-dioxo-cyclobut-1-enylamino)-benzoyl]-pyrrolidine-2-carboxylate de méthyle
8/- 2-hydroxy-N-méthyl-3-(2-{[(S)-(5-méthyl-furan-2-yl)-tétrahydro-thiophen-2-yl-méthyl]-amino}-3,4-dioxo-cyclobut-1-enylamino)-N-(2,2,2-trifluoro-éthyl)-benzamide
9/- {[2-hydroxy-3-(2-{[(5-méthyl-furan-2-yl)-(tétrahydro-thiophen-2-yl)-méthyl]-amino}-3,4-dioxo-cyclobut-1-enylamino)-benzoyl]-méthyl-amino}-acétate de méthyle
10/- 6-chloro-2-hydroxy-N,N-diméthyl-3-(2-{[(5-méthyl-furan-2-yl)-(tétrahydro-thiophèn-2-yl)-méthyl]-amino}-3,4-dioxo-cyclobut-1-enylamino)-benzènesulfonamide
11/-2-hydroxy-N,N-dimethyl-3-(2-{[(R)-(5-methyl-furan-2-yl)-tetrahydro-furan-2-yl-methyl]-amino}-3,4-dioxo-cyclobut-1-enylamino)-benzamide
12/- 2-hydroxy-N,N-dimethyl-3-(2-{[(S)-(5-methyl-furan-2-yl)-tetrahydro-furan-2-yl-methyl]-amino}-3,4-dioxo-cyclobut-1-enylamino)-benzamide*)*
13/- 3-(3,4-dioxo-2-{[phenyl-(tétrahydrofuran-2-yl)-méthyl]-amino}-cyclobut-1-enyl-amino)-2-hydroxy-N,N-diméthyl-benzamide
14/- 3-(2-{[((R)-2,2-diméthyl-[1,3]dioxolan-4-yl)-(5-méthyl-furan-2-yl)-méthyl]-amino}-3,4-dioxo-cyclobut-1-enylamino)-2-hydroxy-N,N-diméthyl-benzamide
15/ (S)-1-[2-Fluoro-3-(2-{[(5-méthyl-furan-2-yl)-(tétrahydro-thiophen-2-yl)-méthyl]-amina}-3,4-dioxo-cyclobut-1-enylamino)-benzoyl]-pyrrolidine-2-carboxylate de méthyle
16/ 3-(2-hydroxy-pyridin-3-ylamino)-4-{[(5-méthyl-furan-2-yl)-(tétrahydro-thiophen-2-yl)-méthyl]-amino}-cyclobut-3-ène-1,2-dione
17/ 3-{[(5-méthyl-furan-2-yl)-(tétrahydro-thiophen-2-yl)-méthyl]-amino}-4-(1-méthyl-2-oxo-1,2-dihydro-pyridin-3-ylamino)-cyclobut-3-ène-1,2-dione
18/ (-)-2-hydroxy-N-méthyl-3-(2-{[(S)-(5-méthyl-furan-2-yl)-(tétrahydro-thiophen-2-yl)-méthyl]-amino}-3,4-dioxo-cyclobut-1-enylamino)-N-(2,2,2-trifluoro-éthyl)-benzamide
19/(-)-{[2-hydroxy-3-(2-{[(S)-(5-méthyl-furan-2-yl)-(tétrahydro-thiophen-2-yl)-méthyl]-amino}-3,4-dioxo-cyclobut-1-enylamino)-benzoyl]-méthyl-amino}-acétate de méthyle
20/ (-)-1-[2-hydroxy-3-(2-{[((S)-5-méthyl-furan-2-yl)-(tétrahydro-thiophen-2-yl)-méthyl]-amino}-3,4-droxo-cyclobut-1-enylamino)-benzoyl]-pyrrolidine-2-(R)-carboxylate de méthyle
21/(-)-6-chloro-2-hydroxy-N,N-diméthyl-3-(2-{[((S)-5-méthyl-furan-2-yl)-(tétrahydro-thiophen-2-yl)-méthyl]-amino}-3,4-dioxo-cyclobut-1-enylamino)-benzamide
22/(-)-3-[4-chloro-2-hydroxy-3-(4-méthyl-pipérazine-1-sulfonyl)-phénylamino]-4- {[(5-méthyl-furan-2-yl)-(tétrahydro-thiophen-2-yl)-méthyl]-amino}-cyclobut-3-ène-1,2-dione

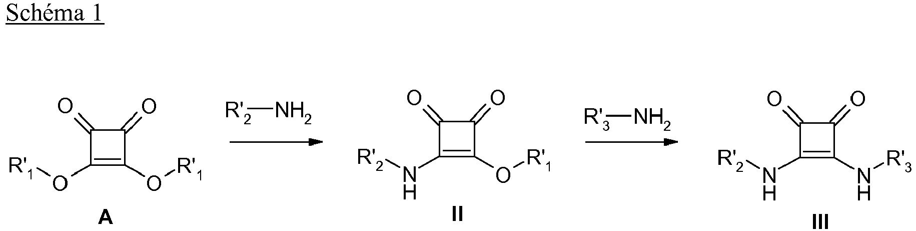

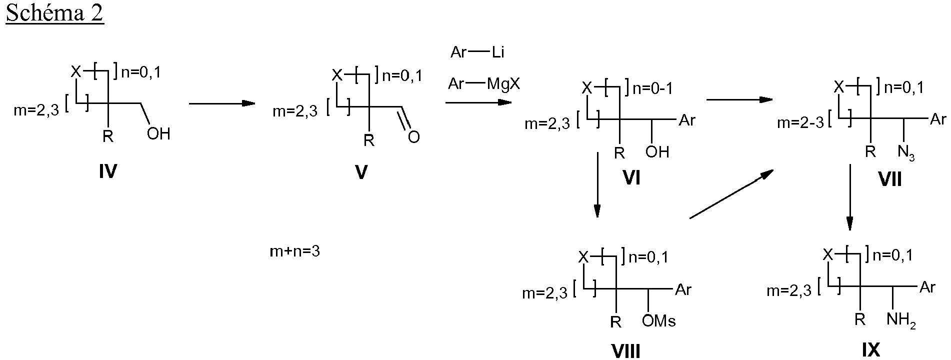

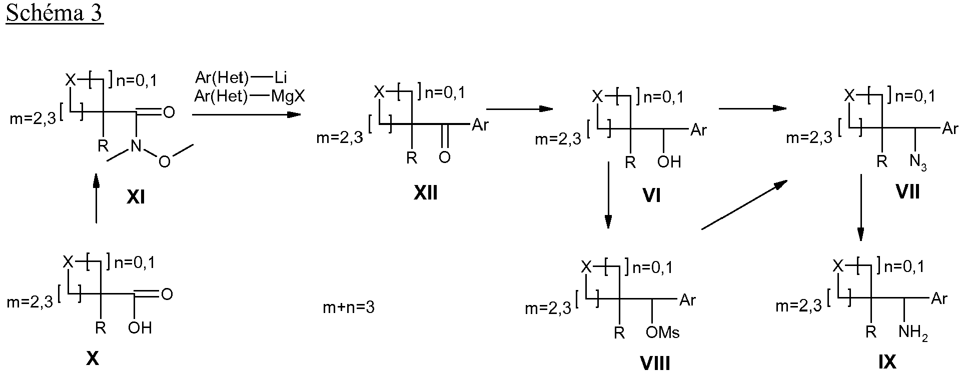

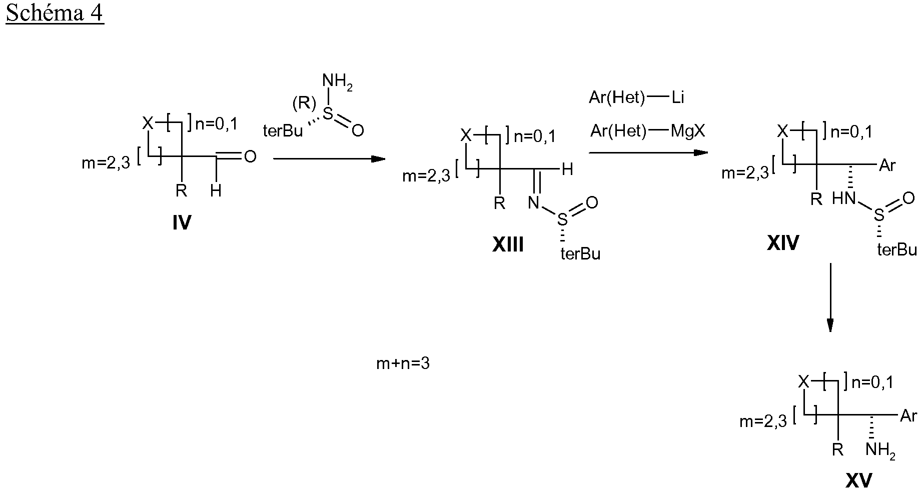

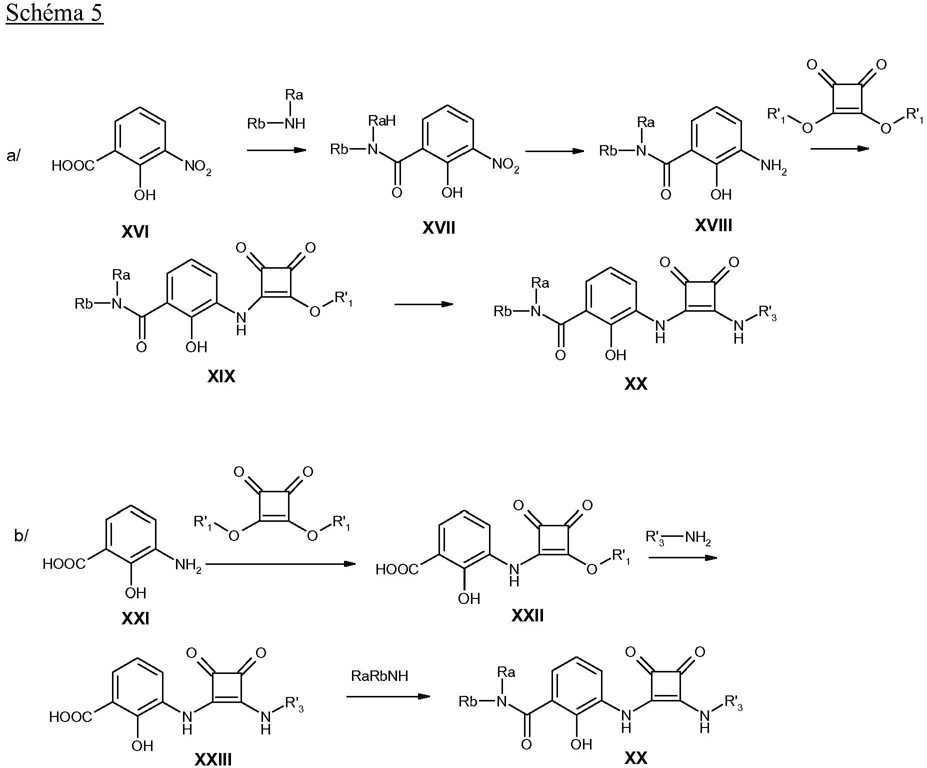

## Patentansprüche

1. Disubstituierte 3,4-Diamino-3-cyclobuten-1,2-dion-verbindung der folgenden allgemeinen Formel (I) oder eines ihrer pharmazeutisch unbedenklichen Salze oder Solvate: 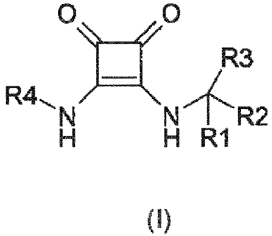 wobei
R1 für ein Wasserstoffatom oder ein Methyl steht,
R2 für einen fünfatomigen Ring steht, der aus den folgenden Strukturen (1), (2), (3) und (4) ausgewählt ist: 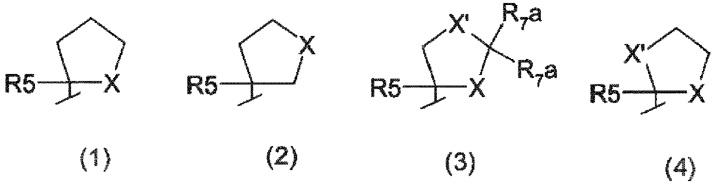 wobei R5, R7a, X und X' die nachstehend angegebene Bedeutung besitzen,
R3 für einen aromatischen oder heteroaromatischen Ring steht, der aus der Gruppe der Ringe der folgenden Formeln (a) bis (O) ausgewählt ist: 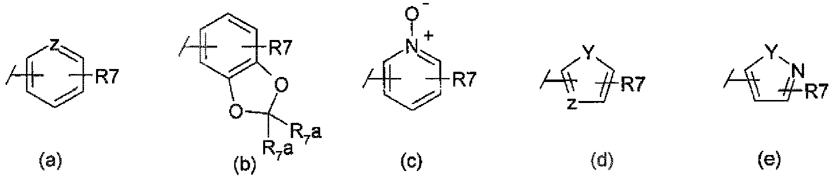 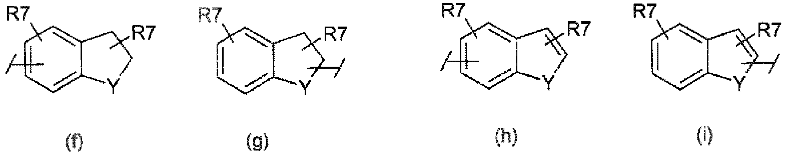 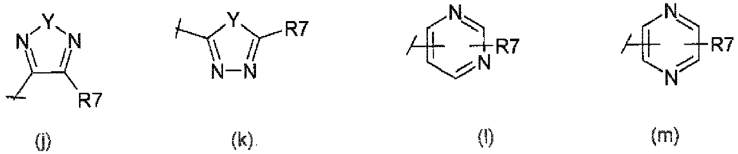 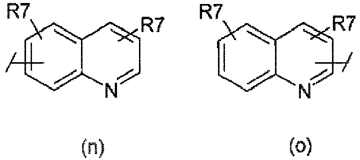 wobei R7, R7a, Y und Z die nachstehend angegebene Bedeutung besitzen, wobei die Ringe (a) bis (O) gegebenenfalls mehrere gleiche oder verschiedene R7-Gruppen tragen können, wobei die Gesamtzahl derartiger R7-Gruppen höchstens gleich der Zahl substituierbarer Atome des Rings ist;
R4 für einen aromatischen oder heteroaromatischen Ring steht, der aus der Gruppe der Ringe der folgenden Formeln (p) bis (z) und (aa) bis (ak) ausgewählt ist: 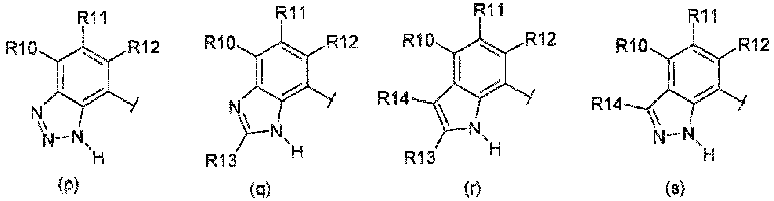 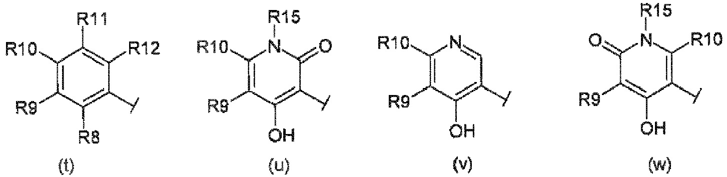 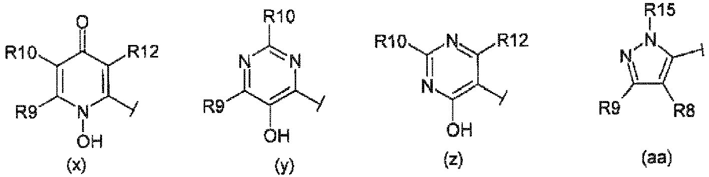 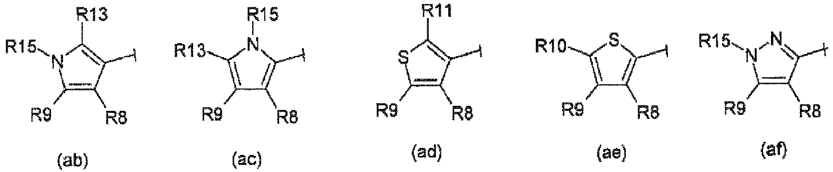 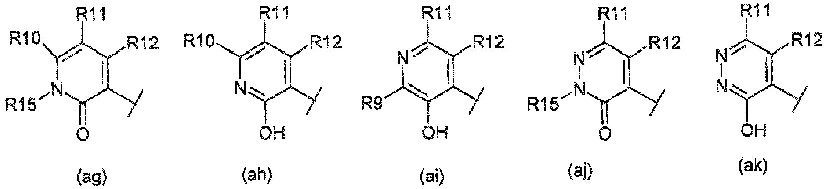 wobei R7, R8, R9, R10, R11, R12, R13, R14 und R15 die nachstehend angegebene Bedeutung besitzen,
R5 für ein Wasserstoffatom, ein Fluoratom, einen Alkylrest mit 1 bis 5 Kohlenstoffatomen oder einen Fluoralkyl- oder Perfluoralkylrest mit 1 bis 5 Kohlenstoffatomen steht,
R6 für ein Wasserstoffatom, einen -COOtBu-Rest oder einen -COOBn-Rest steht,
R7 für ein Halogen oder einen R16-, -CF₃-, -COR16-, -OR16-, -NR16R17-, -NO₂-, -CN-, -SO₂R16-, -SO₂NR16R17-, -NR16COR17-, -CONR16R17-, -NR16CO₂R17- oder -CO₂R16-Rest steht,
R7a für ein Wasserstoffatom oder einen Alkylrest mit 1 bis 5 Kohlenstoffatomen steht,
R8 für ein Wasserstoffatom, ein Halogenatom oder einen -OH-Rest oder einen -SH-, -CONHOR16-, -CONR160H-, -NR16R17-, -SO₃H-, -OCOR16-, -NHSO₂R16-, -SO₂NR16R17-, -NHCOR16-, -CONR16R17-, -NR16CO₂R17-, -NHSO₂NR16R17-, -CO₂R16-, Pyrrolyl-, Imidazolyl-, Triazolyl- oder Tetrazolylrest steht,
R9, R10, R11 und R12 gleich oder verschieden sind und unabhängig voneinander aus der Gruppe bestehend aus einem Wasserstoff, einem Halogenatom und einem Alkyl-, Alkoxy-, -CF₃-, -OCF₃-, -OH-, -NO₂-, -CN-, -SO₂R16-, -SO₂NR16R17-, -NR16COR17-, -NR16CO₂R17-, -CONR16R17-, -COR16- oder -CO₂R16-Rest ausgewählt sind,
oder auch zwei der Reste R9, R10, R11 und R12 dann, wenn sie in ortho-Position an einem aromatischen oder heteroaromatischen Ring, der aus der Gruppe bestehend aus den Ringen der obigen Formeln (p) bis (z) und (aa) bis (ak) ausgewählt ist, stehen, zusammen mit der sie verknüpfenden Bindung einen Aryl-, Heteroaryl-, Cycloalkyl- oder Heterocycloalkylring bilden können,
R13 und R14 gleich oder verschieden sind und unabhängig voneinander aus der Gruppe bestehend aus einem Wasserstoffatom, einem Halogenatom und einem Alkyl-, -CF₃-, -OCF₃-, -OH-, -SH-, -CN-, -SO₂R16-, -SO₂NR16R17-, -NHSO₂NR16R17-, -NR16R17-, -NR16CONR16R17-, -NR16COR17-, -NR16CO₂R17-, -CONR16R17-, -COR16- oder -CO₂R16-Rest ausgewählt sind,
R15 für ein Wasserstoffatom oder einen -OH-, -SO₂R16-, -COR16-, -CO₂R16-, Aryl-, Heteroaryl-, Arylalkyl-, Heteroarylalkyl-, Alkyl-, Cycloalkyl- oder Cycloalkylalkylrest steht,
R16 und R17 gleich oder verschieden sind und unabhängig voneinander aus der Gruppe bestehend aus einem Wasserstoffatom, einem Arylrest, Heteroarylrest, Arylalkylrest, Heteroarylalkylrest, Alkylrest, Fluoralkylrest mit 1 bis 5 Kohlenstoffatomen, Cycloalkylrest oder Cycloalkyl-alkylrest und einer -CH₂COOR18-Gruppe, worin R18 für einen Alkylrest mit 1 bis 5 Kohlenstoffatomen steht, ausgewählt sind,
oder R16 und R17 dann, wenn sie an dasselbe Stickstoffatom gebunden sind, einen Heterocyclus bilden, der zwischen 3 und 7 Glieder aufweist und neben dem gemeinsamen Stickstoffatom, an das sie gebunden sind, gegebenenfalls ein oder zwei Heteroatome, die aus Sauerstoff, Schwefel und Stickstoff ausgewählt sind, enthält, wobei der Heterocyclus durch eine Alkylgruppe mit 1 bis 5 Kohlenstoffatomen oder eine -COOR18-Gruppe, worin R18 für einen Alkylrest mit 1 bis 5 Kohlenstoffatomen steht, substituiert sein kann,
X und X' gleich oder verschieden sind und für ein Sauerstoffatom, ein Schwefelatom oder ein durch einen Rest R6 substituiertes Stickstoffatom stehen,
Y für ein Sauerstoffatom, ein Schwefelatom oder ein durch einen Rest R15 substituiertes Stickstoffatom steht und
Z für ein Kohlenstoff- oder Stickstoffatom steht.

2. Verbindung nach Anspruch 1, **dadurch gekennzeichnet, dass** in der obigen Formel (I):
R1 für ein Wasserstoffatom steht,
R2 für einen fünfgliedrigen Ring steht, der aus den folgenden Strukturen (1), (2) und (3) ausgewählt ist: 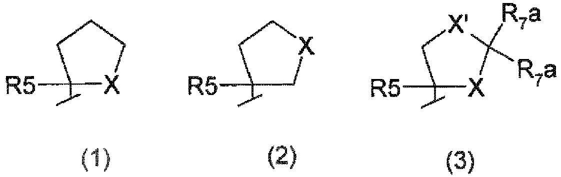 wobei R5, R7a, X und X' die nachstehend angegebene Bedeutung besitzen,
R3 für einen aromatischen oder heteroaromatischen Ring steht, der aus der Gruppe der Ringe der folgenden Formeln (a), (b) und (d) ausgewählt ist: 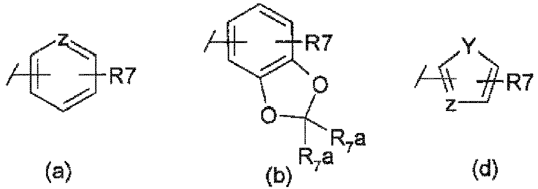 wobei R7, R7a, Y und Z die nachstehend angegebene Bedeutung besitzen, wobei die Ringe (a), (b) und (d) gegebenenfalls mehrere gleiche oder verschiedene R7-Gruppen tragen können, wobei die Gesamtzahl derartiger R7-Gruppen höchstens gleich der Zahl substituierbarer Atome des Rings ist;
R4 für einen aromatischen oder heteroaromatischen Ring steht, der aus der Gruppe der Ringe der folgenden Formeln (p), (q), (t), (z), (ad), (ag) und (ah) ausgewählt ist: 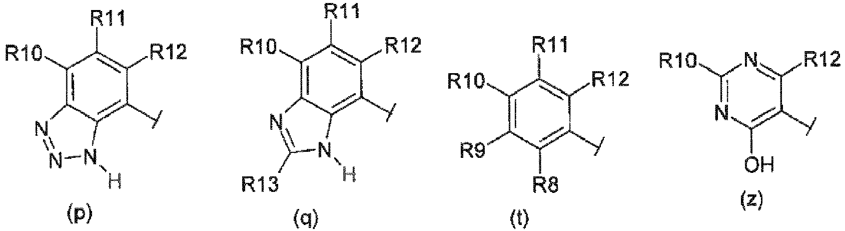 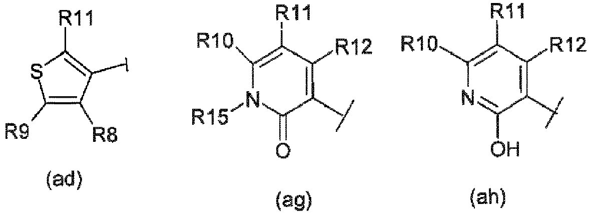 wobei R8, R9, R10, R11, R12, R13 und R15 die nachstehend angegebene Bedeutung besitzen,
R5 für ein Wasserstoffatom, ein Fluoratom, einen Alkylrest mit 1 bis 5 Kohlenstoffatomen oder einen Fluoralkyl- oder Perfluoralkylrest mit 1 bis 5 Kohlenstoffatomen steht,
R6 für ein Wasserstoffatom, einen -COOtBu-Rest oder einen -COOBn-Rest steht,
R7 für ein Halogen oder einen R16-, -CF₃-, -COR16-, -OR16-, -NR16R17-, -NO₂-, -CN-, -SO₂R16-, -SO₂NR16R17-, -NR16COR17-, -CONR16R17-, -NR16CO₂R17- oder -CO₂R16-Rest steht,
R7a für ein Wasserstoffatom oder einen Alkylrest mit 1 bis 5 Kohlenstoffatomen steht,
R8 für ein Wasserstoffatom oder einen -OH-, -SH-, -CONHOR16-, -CONR160H-, -NR16R17-, -SO₃H-, -OCOR16-, -NHSO₂R16-, -SO₂NR16R17-, -NHCOR16-, -CONR16R17-, -NR16CO₂R17-, -NHSO₂NR16R17-, -CO₂R16-, Pyrrolyl-, Imidazolyl-, Triazolyl- oder Tetrazolylrest steht,
R9, R10, R11 und R12 gleich oder verschieden sind und unabhängig voneinander aus der Gruppe bestehend aus einem Wasserstoffatom, einem Halogenatom und einem Alkyl-, Alkoxy-, -CF₃-, -OCF₃-, -OH-, -NO₂-, -CN-, -SO₂R16-, -SO₂NR16R17-, -NR16COR17-, -NR16CO₂R17-, -CONR16R17-, -COR16- oder -CO₂R16-Rest ausgewählt sind,
oder auch zwei der Reste R9, R10, R11 und R12 dann, wenn sie in ortho-Position an einem aromatischen oder heteroaromatischen Ring, der aus der Gruppe bestehend aus den Ringen der obigen Formeln (p), (q), (t), (z), (ad), (ag) und (ah) ausgewählt ist, stehen, zusammen mit der sie verknüpfenden Bindung einen Aryl-, Heteroaryl-, Cycloalkyl- oder Heterocycloalkylring bilden können,
R13 aus der Gruppe bestehend aus einem Wasserstoffatom, einem Halogenatom und einem Alkyl-, -CF₃-, -OCF₃-, -OH-, -SH-, -CN-, -SO₂R16-, -SO₂NR16R17-, -NHSO₂NR16R17-, -NR16R17-, -NR16CONR16R17-, -NR16COR17-, -NR16CO₂R17-, -CONR16R17-, -COR16- oder -CO₂R16-Rest ausgewählt ist,
R15 für ein Wasserstoffatom oder einen -OH-, -SO₂R16-, -COR16-, -CO₂R16-, Aryl-, Heteroaryl-, Arylalkyl-, Heteroarylalkyl-, Alkyl-, Cycloalkyl- oder Cycloalkylalkylrest steht,
R16 und R17 gleich oder verschieden sind und unabhängig voneinander aus der Gruppe bestehend aus einem Wasserstoffatom, einem Arylrest, Heteroarylrest, Arylalkylrest, Heteroarylalkylrest, Alkylrest, Fluoralkylrest mit 1 bis 5 Kohlenstoffatomen, Cycloalkylrest oder Cycloalkylalkylrest und einer -CH₂COOR18-Gruppe, worin R18 für einen Alkylrest mit 1 bis 5 Kohlenstoffatomen steht, ausgewählt sind,
oder R16 und R17 dann, wenn sie an dasselbe Stickstoffatom gebunden sind, einen Heterocyclus bilden, der zwischen 3 und 7 Glieder aufweist und neben dem gemeinsamen Stickstoffatom, an das sie gebunden sind, gegebenenfalls ein oder zwei Heteroatome, die aus Sauerstoff, Schwefel und Stickstoff ausgewählt sind, enthält, wobei der Heterocyclus durch eine Alkylgruppe mit 1 bis 5 Kohlenstoffatomen oder eine -COOR18-Gruppe, worin R18 für einen Alkylrest mit 1 bis 5 Kohlenstoffatomen steht, substituiert sein kann,
X und X' gleich oder verschieden sind und für ein Sauerstoffatom, ein Schwefelatom oder ein durch einen Rest R6 substituiertes Stickstoffatom stehen,
Y für ein Sauerstoffatom, ein Schwefelatom oder ein durch einen Rest R15 substituiertes Stickstoffatom steht und
Z für ein Kohlenstoff- oder Stickstoffatom steht.

3. Verbindung nach Anspruch 1 oder 2, **dadurch gekennzeichnet, dass** in der obigen Formel (I):
R1 für ein Wasserstoffatom steht,
R2 für einen fünfatomigen Ring der folgenden Struktur (1) steht: 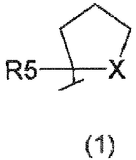 wobei R5 und X die nachstehend angegebene Bedeutung besitzen,
R3 für einen heteroaromatischen Ring der folgenden Formel (d) steht: 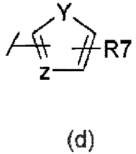 wobei R7, Y und Z die nachstehend angegebene Bedeutung besitzen, wobei der Ring (d) gegebenenfalls mehrere gleiche oder verschiedene R7-Gruppen tragen kann, wobei die Gesamtzahl derartiger R7-Gruppen höchstens gleich der Zahl substituierbarer Atome des Rings ist;
R4 für einen aromatischen Ring der folgenden Formel (t) steht: 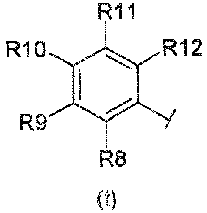 wobei R8, R9, R10, R11 und R12 die nachstehend angegebene Bedeutung besitzen,
R5 für ein Wasserstoffatom, ein Fluoratom, einen Alkylrest mit 1 bis 5 Kohlenstoffatomen oder einen Fluoralkyl- oder Perfluoralkylrest mit 1 bis 5 Kohlenstoffatomen steht,
R6 für ein Wasserstoffatom, einen -COOtBu-Rest oder einen -COOBn-Rest steht,
R7 für ein Halogenatom oder einen R16-, -CF₃-, -COR16-, -OR16-, -NR16R17-, -NO₂-, -CN-, -SO₂R16-, -SO₂NR16R17-, -NR16COR17-, -CONR16R17-, -NR16CO₂R17- oder -CO₂R16-Rest steht,
R8 für ein Wasserstoffatom oder einen -OH-, -SH-, -CONHOR16-, -CONR160H-, -NR16R17-, -SO₃H-, -OCOR16-, -NHSO₂R16-, -SO₂NR16R17-, -NHCOR16-, -CONR16R17-, -NR16CO₂R17-, -NHSO₂NR16R17-, -CO₂R16-, Pyrrolyl-, Imidazolyl-, Triazolyl- oder Tetrazolylrest steht,
R9, R10, R11 und R12 gleich oder verschieden sind und unabhängig voneinander aus der Gruppe bestehend aus einem Wasserstoffatom, einem Halogenatom und einem Alkyl-, Alkoxy-, -CF₃-, -OCF₃-, -OH-, -NO₂-, -CN-, -SO₂R16-, -SO₂NR16R17-, -NR16COR17-, -NR16CO₂R17-, -CONR16R17-, -COR16- oder -CO₂R16-Rest ausgewählt sind,
oder auch zwei der Reste R9, R10, R11 und R12 dann, wenn sie in ortho-Position an dem aromatischen Ring (t) stehen, zusammen mit der sie verknüpfenden Bindung einen Aryl-, Heteroaryl-, Cycloalkyl- oder Heterocycloalkylring bilden können,
R15 für ein Wasserstoffatom oder einen -OH-, -SO₂R16-, -COR16-, -CO₂R16-, Aryl-, Heteroaryl-, Arylalkyl-, Heteroarylalkyl-, Alkyl-, Cycloalkyl- oder Cycloalkylalkylrest steht,
R16 und R17 gleich oder verschieden sind und unabhängig voneinander aus der Gruppe bestehend aus einem Wasserstoffatom, einem Arylrest, Heteroarylrest, Arylalkylrest, Heteroarylalkylrest, Alkylrest, Fluoralkylrest mit 1 bis 5 Kohlenstoffatomen, Cycloalkylrest oder Cycloalkyl-alkylrest und einer -CH₂COOR18-Gruppe, worin R18 für einen Alkylrest mit 1 bis 5 Kohlenstoffatomen steht, ausgewählt sind,
oder R16 und R17 dann, wenn sie an dasselbe Stickstoffatom gebunden sind, einen Heterocyclus bilden, der zwischen 3 und 7 Glieder aufweist und neben dem gemeinsamen Stickstoffatom, an das sie gebunden sind, gegebenenfalls ein oder zwei Heteroatome, die aus Sauerstoff, Schwefel und Stickstoff ausgewählt sind, enthält, wobei der Heterocyclus durch eine Alkylgruppe mit 1 bis 5 Kohlenstoffatomen oder eine -COOR18-Gruppe, worin R18 für einen Alkylrest mit 1 bis 5 Kohlenstoffatomen steht, substituiert sein kann,
X für ein Sauerstoffatom, ein Schwefelatom oder ein durch einen Rest R6 substituiertes Stickstoffatom steht,
Y für ein Sauerstoffatom, ein Schwefelatom oder ein durch einen Rest R15 substituiertes Stickstoffatom steht und
Z für ein Kohlenstoff- oder Stickstoffatom steht.

4. Pharmazeutische Zusammensetzung, umfassend eine wirksame Menge einer Verbindung oder eines pharmazeutisch unbedenklichen Salzes der Verbindung nach Anspruch 1 in Kombination mit einem pharmazeutisch unbedenklichen Lösungsmittel oder Träger.

5. Verbindung nach Anspruch 1 oder pharmazeutische Zusammensetzung nach Anspruch 4 zur Verwendung als Medikament.

6. Verbindung nach Anspruch 1 oder pharmazeutische Zusammensetzung nach Anspruch 4 zur Verwendung bei der Behandlung von durch α-Chemokine vermittelten Erkrankungen, ausgewählt aus den Erkrankungen aus der Gruppe umfassend neutrophile Dermatosen, ausgewählt aus Psoriasis, atopischen Dermatiden, Akne und Rosacea, Asthma, chronisch-obstruktive Lungenerkrankungen, Atemwegserkrankungen bei Erwachsenen, Arthritis, entzündliche Darmerkrankungen, Morbus Crohn, Transplantatabstoßung, Mukoviszidose und Hautkrebserkrankungen.

7. Verbindung oder pharmazeutische Zusammensetzung nach Anspruch 6 zur Verwendung bei der Behandlung von dermatologischen Erkrankungen, umfassend neutrophile Dermatosen, ausgewählt aus Psoriasis, atopischen Dermatiden, Akne und Rosacea.

8. Verbindung nach Anspruch 1, ausgewählt aus der Liste, umfassend:
1/- 2-Hydroxy-N,N-dimethyl-3-(2-{[(5-methylfuran-2-yl)-(2-methyltetrahydrofuran-2-yl)methyl]amino}-3,4-dioxocyclobut-1-enylamino)benzamid
2/- 2-Hydroxy-N,N-dimethyl-3-(2-{[(5-methylfuran-2-yl)-(tetrahydrothiophen-2-yl)methyl]amino}-3,4-dioxocyclobut-1-enylamino)benzamid
3/- (S)-1-[2-Fluor-3-(2-{[(5-methylfuran-2-yl)-(tetrahydrothiophen-2-yl)methyl]amino}-3,4-dioxocyclobut-l-enylamino)benzoyl]pyrrolidin-2-carbonsäuremethylester
4/- (S)-1-[2-Fluor-3-(2-{[(5-methylfuran-2-yl)-(tetrahydrothiophen-2-yl)methyl]amino}-3,4-dioxocyclobut-l-enylamino)benzoyl]pyrrolidin-2-carbonsäureisopropylester
5/- (S)-1-[2-Fluor-3-(2-{[(5-methylfuran-2-yl)-(tetrahydrothiophen-2-yl)methyl]amino}-3,4-dioxocyclobut-l-enylamino)benzoyl]pyrrolidin-2-carbonsäureethylester
6/- (R)-1-[2-Hydroxy-3-(2-{[(5-methylfuran-2-yl)-(tetrahydrothiophen-2-yl)methyl]amino}-3,4-dioxocyclobut-l-enylamino)benzoyl]pyrrolidin-2-carbonsäuremethylester
7/- (S)-1-[2-Hydroxy-3-(2-{[(5-methylfuran-2-yl)-(tetrahydrothiophen-2-yl)methyl]amino}-3,4-dioxocyclobut-1-enylamino)benzoyl]pyrrolidin-2-carbonsäuremethylester
8/- 2-Hydroxy-N-methyl-3-(2-{[(S)-(5-methylfuran-2-yl)tetrahydrothiophen-2-ylmethyl]amino}-3,4-dioxocyclobut-1-enylamino)-N-(2,2,2-trifluorethyl)benzamid
9/- {[2-Hydroxy-3-(2-{[(5-methylfuran-2-yl)-(tetrahydrothiophen-2-yl)methyl]amino}-3,4-dioxocyclobut-1-enylamino)benzoyl]methylamino}-essigsäuremethylester
10/- 6-Chlor-2-hydroxy-N,N-dimethyl-3-(2-{[(5-methylfuran-2-yl)-(tetrahydrothiophen-2-yl)methyl]amino}-3,4-dioxocyclobut-1-enylamino)benzolsulfonamid
11/- 2-Hydroxy-N,N-dimethyl-3-(2-{[(R)-(5-methyl-furan-2-yl)tetrahydro-furan-2-ylmethyl]amino}-3,4-dioxocyclobut-1-enylamino)benzamid
12/- 2-Hydroxy-N,N-dimethyl-3-(2-{[(S)-(5-methylfuran-2-yl)tetrahydrofuran-2-ylmethyl]-amino}-3,4-dioxocyclobut-1-enylamino)benzamid
13/- 3-(3,4-Dioxo-2-{[phenyl(tetrahydrofuran-2-yl)methyl]amino}cyclobut-1-enylamino)-2-hydroxy-N,N-dimethylbenzamid
14/- 3-(2-{[((R)-2,2-Dimethyl-[1,3]dioxolan-4-yl)-(5-methylfuran-2-yl)methyl]amino}-3,4-dioxocyclobut-1-enylamino)-2-hydroxy-N,N-dimethylbenzamid
15/ (S)-1-[2-Fluor-3-(2-{[(5-methylfuran-2-yl)-(tetrahydrothiophen-2-yl)methyl]amino}-3,4-dioxocyclobut-1-enylamino)benzoyl]pyrrolidin-2-carbonsäuremethylester
16/ 3-(2-Hydroxypyridin-3-ylamino)-4-{[(5-methyl-furan-2-yl)-(tetrahydrothiophen-2-yl)methyl]-amino}cyclobut-3-en-1,2-dion
17/ 3-{[(5-Methylfuran-2-yl)-(tetrahydrothiophen-2-yl)methyl]amino}-4-(1-methyl-2-oxo-1,2-dihydropyridin-3-ylamino)cyclobut-3-en-1,2-dion
18/ (-)-2-Hydroxy-N-methyl-3-(2-{[(S)-(5-methyl-furan-2-yl)-(tetrahydrothiophen-2-yl)methyl]-amino}-3,4-dioxocyclobut-1-enylamino)-N-(2,2,2-trifluorethyl)benzamid
19/ (-)-{[2-Hydroxy-3-(2-{[(S)-(5-methylfuran-2-yl)-(tetrahydrothiophen-2-yl)methyl]amino}-3,4-dioxocyclobut-1-enylamino)benzoyl]methylamino}-essigsäuremethylester
20/ (-)-1-[2-Hydroxy-3-(2-{[((S)-5-methylfuran-2-yl)-(tetrahydrothiophen-2-yl)methyl]amino}-3,4-dioxocyclobut-1-enylamino)benzoyl]pyrrolidin-2-(R)-carbonsäuremethylester
21/ (-)-6-Chlor-2-hydroxy-N,N-dimethyl-3-(2-{[((S)-5-methylfuran-2-yl)-(tetrahydrothiophen-2-yl)methyl]amino}-3,4-dioxocyclobut-1-enylamino)-benzamid
22/ (-)-3-[4-Chlor-2-hydroxy-3-(4-methylpiperazin-1-sulfonyl)phenylamino]-4-{[(5-methylfuran-2-yl)-(tetrahydrothiophen-2-yl)methyl]amino}cyclobut-3-en-1,2-dion.

## Claims

1. Disubstituted 3,4-diamino-3-cyclobutene-1,2-dione compound corresponding to general formula (I) below, or one of the pharmaceutically acceptable salts or solvates thereof: 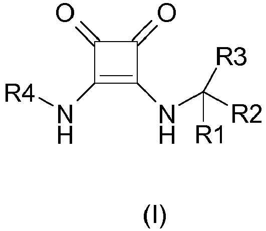 in which:
R1 represents a hydrogen atom or a methyl,
R2 represents a ring comprising five atoms, chosen from the structures (1), (2), (3) and (4) below: 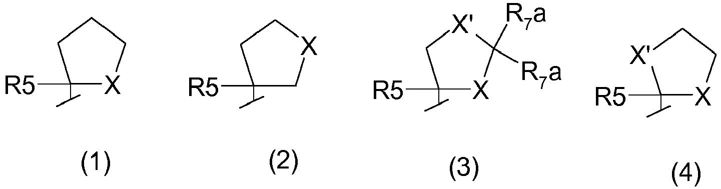 in which R5, R7a, X and X' have the meaning given hereinafter,
R3 represents an aromatic or heteroaromatic ring selected from the group consisting of the rings corresponding to formulae (a) to (O) below: 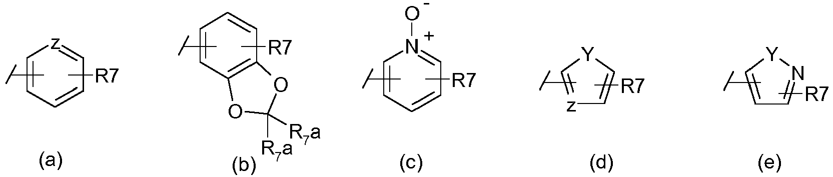 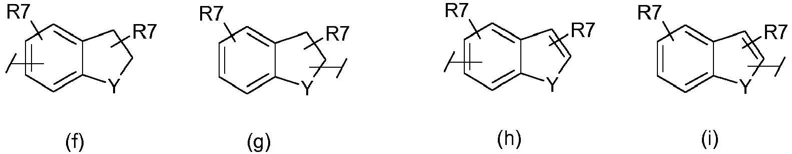 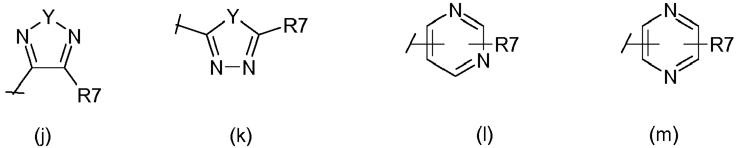 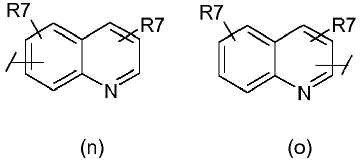 in which R7, R7a, Y and Z have the meaning given hereinafter, it being specified that the rings (a) to (O) can optionally bear several R7 groups, which may be identical or different, the total number of such R7 groups being at most equal to the number of substitutable atoms of the ring;
R4 represents an aromatic or heteroaromatic ring selected from the group consisting of the rings corresponding to formulae (p) to (z) and (aa) to (ak) below: 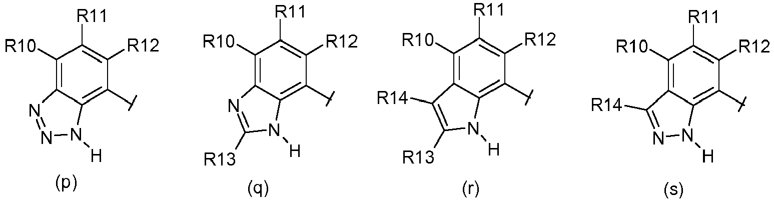 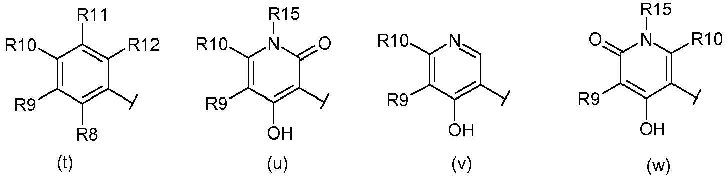 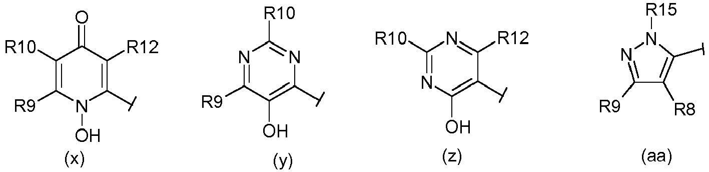 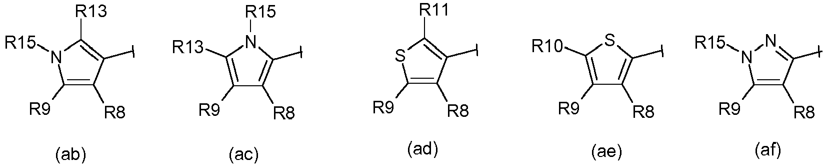 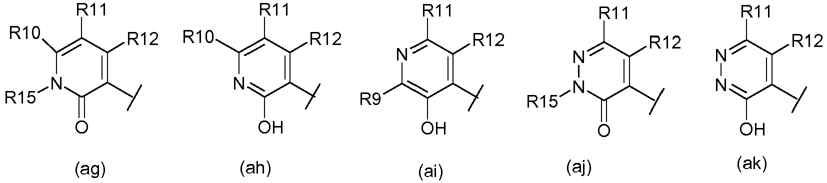 in which R7, R8, R9, R10, R11, R12, R13, R14 and R15 have the meaning given hereinafter,
R5 represents a hydrogen atom, a fluorine atom, an alkyl radical having from 1 to 5 carbon atoms or a fluoroalkyl or perfluoroalkyl radical comprising from 1 to 5 carbon atoms,
R6 represents a hydrogen atom, a -COOtBu radical or a -COOBn radical,
R7 represents a halogen, or an -R16, -CF₃, -COR16, -OR16, -NR16R17, -NO₂, -CN, -SO₂R16, -SO₂NR16R17, -NR16COR17, -CONR16R17, -NR16CO₂R17 or -CO₂R16 radical,
R7a represents a hydrogen atom or an alkyl radical having from 1 to 5 carbon atoms,
R8 represents a hydrogen atom, a halogen atom, an -OH radical, or an -SH, -CONHOR16, -CONR16OH, -NR16R17, -SO₃H, -OCOR16, -NHSO₂R16, -SO₂NR16R17, -NHCOR16, CONR16R17, -NR16CO2R17, -NHSO₂NR16R17, -CO₂R16, pyrrolyl, imidazolyl, triazolyl or tetrazolyl radical,
R9, R10, R11 and R12 are identical or different and are independently chosen from the group consisting of a hydrogen, a halogen atom and an alkyl, alkoxy, -CF₃, -OCF₃, -OH, -NO₂, -CN, -SO₂R16, -SO₂NR16R17, -NR16COR17, -NR16CO2R17, -CONR16R17, -COR16 or -CO₂R16 radical,
or alternatively, when two of the R9, R10, R11 and R12 radicals are in the ortho position on an aromatic or heteroaromatic ring selected from the group consisting of the rings corresponding to formulae (p) to (z) and (aa) to (ak) above, then they can together form, with the bond which links them together, an aryl, heteroaryl, cycloalkyl or heterocycloalkyl ring,
R13 and R14 are identical or different and are independently chosen from the group consisting of a hydrogen atom, a halogen atom, and an alkyl, -CF₃, -OCF₃, -OH, -SH, -CN, -SO₂R16, -SO₂NR16R17, -NHSO₂NR16R17, -NR16R17, -NR16CONR16R17, -NR16COR17, -NR16CO2R17, -CONR16R17, -COR16 or -CO₂R16 radical,
R15 represents a hydrogen atom or an -OH, -SO₂R16, -COR16, -CO₂R16, aryl, heteroaryl, arylalkyl, heteroarylalkyl, alkyl, cycloalkyl or cycloalkylalkyl radical,
R16 and R17 are identical or different and are independently chosen from the group consisting of a hydrogen atom, one of the following radicals: aryl, heteroaryl, arylalkyl, heteroarylalkyl, alkyl, fluoroalkyl having from 1 to 5 carbon atoms, cycloalkyl or cycloalkylalkyl, and a -CH₂COOR18 group in which R18 represents an alkyl radical having from 1 to 5 carbon atoms,
or alternatively, when R16 and R17 are borne by the same nitrogen atom, they form a heterocycle having between 3 and 7 ring members and optionally comprising one or two heteroatoms chosen from oxygen, sulfur and nitrogen in addition to the common nitrogen atom by which they are borne, it being possible for said heterocycle to be substituted with an alkyl group having from 1 to 5 carbon atoms or a -COOR18 group in which R18 represents an alkyl radical having from 1 to 5 carbon atoms;
X and X', which may be identical or different, represent an oxygen atom, a sulfur atom, or a nitrogen atom substituted with an R6 radical,
Y represents an oxygen atom, a sulfur atom, or a nitrogen atom substituted with an R15 radical, and
Z represents a carbon or nitrogen atom.

2. Compound according to Claim 1, **characterized in that**, in the abovementioned formula (I):
R1 represents a hydrogen atom,
R2 represents a five-membered ring chosen from the structures (1), (2) and (3) below: 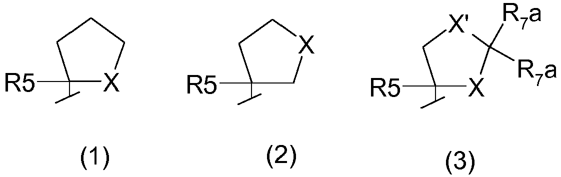 in which R5, R7a, X and X' have the meaning given hereinafter,
R3 represents an aromatic or heteroaromatic ring selected from the group consisting of the rings corresponding to formulae (a), (b) and (d) below: 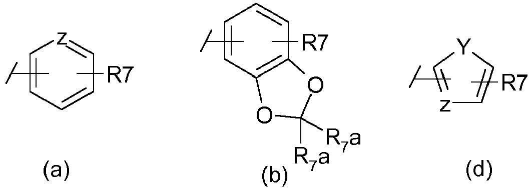 in which R7, R7a, Y and Z have the meaning given hereinafter, it being specified that the rings (a), (b) and (d) can optionally bear several R7 groups, which may be identical or different, the total number of such R7 groups being at most equal to the number of substitutable atoms of the ring;
R4 represents an aromatic or heteroaromatic ring selected from the group consisting of the rings corresponding to formulae (p), (q), (t), (z), (ad), (ag) and (ah) below: 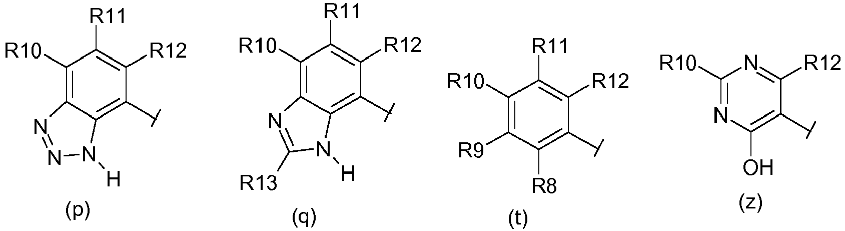 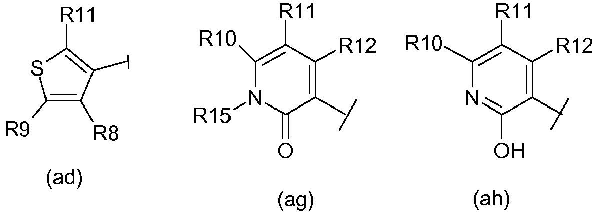 in which R8, R9, R10, R11, R12, R13 and R15 have the meaning given hereinafter,
R5 represents a hydrogen atom, a fluorine atom, an alkyl radical having from 1 to 5 carbon atoms or a fluoroalkyl or perfluoroalkyl radical comprising from 1 to 5 carbon atoms,
R6 represents a hydrogen atom, a -COOtBu radical or a -COOBn radical,
R7 represents a halogen, or an R16, -CF₃, -COR16, -OR16, -NR16R17, -NO₂, -CN, -SO₂R16, -SO₂NR16R17, -NR16COR17, -CONR16R17, -NR16CO₂R17 or -CO₂R16 radical,
R7a represents a hydrogen or an alkyl radical having from 1 to 5 carbon atoms,
R8 represents a hydrogen atom, or an -OH, -SH, -CONHOR16, -CONR16OH, -NR16R17, -SO₃H, -OCOR16, -NHSO2R16, -SO₂NR16R17, -NHCOR16, -CONR16R17, -NR16CO2R17, -NHSO₂NR16R17, -CO₂R16, pyrrolyl, imidazolyl, triazolyl or tetrazolyl radical,
R9, R10, R11 and R12 are identical or different and are independently chosen from the group consisting of a hydrogen atom, a halogen atom and an alkyl, alkoxy, -CF₃, -OCF₃, -OH, -NO₂, -CN, -SO₂R16, -SO₂NR16R17, -NR16COR17, -NR16CO2R17, -CONR16R17, -COR16 or -CO₂R16 radical,
or alternatively, when two of the R9, R10, R11 and R12 radicals are in the ortho position on an aromatic or heteroaromatic ring selected from the group consisting of the rings corresponding to formulae (p), (q), (t), (z), (ad), (ag) and (ah) above, then they can together form, with the bond which links them together, an aryl, heteroaryl, cycloalkyl or heterocycloalkyl ring,
R13 is chosen from the group consisting of a hydrogen atom, a halogen atom, and an alkyl, -CF₃, -OCF₃, -OH, -SH, -CN, -SO₂R16, -SO₂NR16R17, -NHSO₂NR16R17, -NR16R17, -NR16CONR16R17, -NR16COR17, -NR16CO₂R17, -CONR16R17, -COR16 or -CO₂R16 radical,
R15 represents a hydrogen atom or an -OH, -SO₂R16, -COR16, -CO₂R16, aryl, heteroaryl, arylalkyl, heteroarylalkyl, alkyl, cycloalkyl or cycloalkylalkyl radical,
R16 and R17 are identical or different and are independently chosen from the group consisting of a hydrogen atom, one of the following radicals: aryl, heteroaryl, arylalkyl, heteroarylalkyl, alkyl, fluoroalkyl having from 1 to 5 carbon atoms, cycloalkyl or cycloalkylalkyl, and a -CH₂COOR18 group in which R18 represents an alkyl radical having from 1 to 5 carbon atoms,
or alternatively, when R16 and R17 7 are borne by the same nitrogen atom, they form a heterocycle having between 3 and 7 ring members and optionally comprising one or two heteroatoms chosen from oxygen, sulfur and nitrogen in addition to the common nitrogen atom by which they are borne, it being possible for said heterocycle to be substituted with an alkyl group having from 1 to 5 carbon atoms or a -COOR18 group in which R18 represents an alkyl radical having from 1 to 5 carbon atoms;
X and X', which may be identical or different, represent an oxygen atom, a sulfur atom, or a nitrogen atom substituted with an R6 radical,
Y represents an oxygen atom, a sulfur atom, or a nitrogen atom substituted with an R15 radical, and
Z represents a carbon or nitrogen atom.

3. Compound according to Claim 1 or 2, **characterized in that**, in the abovementioned formula (I):
R1 represents a hydrogen atom,
R2 represents a ring comprising five atoms, having the structure (1) below: 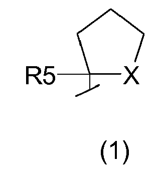 in which R5 and X have the meaning given hereinafter,
R3 represents a heteroaromatic ring corresponding to formula (d) below: 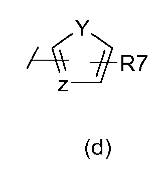 in which R7, Y and Z have the meaning given hereinafter, it being specified that the ring (d) can optionally bear several R7 groups, which may be identical or different, the total number of such R7 groups being at most equal to the number of substitutable atoms of the ring;
R4 represents an aromatic ring corresponding to formula (t) below: 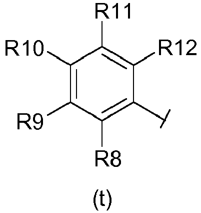 in which R8, R9, R10, R11 and R12 have the meaning given hereinafter,
R5 represents a hydrogen atom, a fluorine atom, an alkyl radical having from 1 to 5 carbon atoms or a fluoroalkyl or perfluoroalkyl radical comprising from 1 to 5 carbon atoms,
R6 represents a hydrogen atom, a -COOtBu radical or a -COOBn radical,
R7 represents a halogen atom, or an R16, -CF₃, -COR16, -OR16, -NR16R17, -NO₂, -CN, -SO₂R16, -SO₂NR16R17, -NR16COR17, -CONR16R17, -NR16CO₂R17 or -CO₂R16 radical,
R8 represents a hydrogen atom, or an -OH, -SH, -CONHOR16, -CONR16OH, -NR16R17, -SO₃H, -OCOR16, -NHSO₂R16, -SO₂NR16R17, -NHCOR16, -CONR16R17, -NR16CO2R17, -NHSO₂NR16R17, -CO₂R16, pyrrolyl, imidazolyl, triazolyl or tetrazolyl radical,
R9, R10, R11 and R12 are identical or different and are independently chosen from the group consisting of a hydrogen atom, a halogen atom and an alkyl, alkoxy, -CF₃, -OCF₃, -OH, -NO₂, -CN, -SO₂R16, -SO₂NR16R17, -NR16COR17, -NR16CO2R17, -CONR16R17, -COR16 or -CO₂R16 radical,
or alternatively, when two of the R9, R10, R11 and R12 radicals are in the ortho position on the aromatic ring (t), they can together form, with the bond which links them together, an aryl, heteroaryl, cycloalkyl or heterocycloalkyl ring,
R15 represents a hydrogen atom or an -OH, -SO₂R16, -COR16, -CO₂R16, aryl, heteroaryl, arylalkyl, heteroarylalkyl, alkyl, cycloalkyl or cycloalkylalkyl radical,
R16 and R17 are identical or different and are independently chosen from the group consisting of a hydrogen atom, one of the following radicals: aryl, heteroaryl, arylalkyl, heteroarylalkyl, alkyl, fluoroalkyl having from 1 to 5 carbon atoms, cycloalkyl or cycloalkylalkyl, and a -CH₂COOR18 group in which R18 represents an alkyl radical having from 1 to 5 carbon atoms,
or alternatively, when R16 and R17 are borne by the same nitrogen atom, they form a heterocycle having between 3 and 7 ring members and optionally comprising one or two heteroatoms chosen from oxygen, sulfur and nitrogen in addition to the common nitrogen atom by which they are borne, it being possible for said heterocycle to be substituted with an alkyl group having from 1 to 5 carbon atoms or a -COOR18 group in which R18 represents an alkyl radical having from 1 to 5 carbon atoms;
X represents an oxygen atom, a sulfur atom, or a nitrogen atom substituted with an R6 radical,
Y represents an oxygen atom, a sulfur atom, or a nitrogen atom substituted with an R15 radical, and
Z represents a carbon or nitrogen atom.

4. Pharmaceutical composition comprising an effective amount of a compound, or of a pharmaceutically acceptable salt of said compound, according to Claim 1, in combination with a pharmaceutically acceptable solvent or support.

5. Compound according to Claim 1 or pharmaceutical composition according to Claim 4, for use as a medicament.

6. Compound according to Claim 1 or pharmaceutical composition according to Claim 4, for use in the treatment of α-chemokine-mediated diseases, chosen from diseases of the group comprising neutrophilic dermatoses, chosen from psoriasis, atopic dermatitis, acne and rosacea, asthma, chronic obstructive pulmonary diseases, respiratory diseases in adults, arthritis, inflammatory bowel diseases, Crohn's disease, transplant rejection, cystic fibrosis and skin cancers.

7. Compound or pharmaceutical composition according to Claim 6, for use in the treatment of skin diseases such as neutrophilic dermatoses, chosen from psoriasis, atopic dermatitis, acne and rosacea.

8. Compound according to Claim 1, chosen from the list comprising:
1/- 2-hydroxy-N,N-dimethyl-3-(2-{[(5-methylfuran-2-yl)-(2-methyltetrahydrofuran-2-yl)methyl]amino}-3,4-dioxocyclobut-1-enylamino)benzamide
2/- 2-hydroxy-*N*,*N*-dimethyl-3-(2-{[(5-methylfuran-2-yl)-(tetrahydrothiophen-2-yl)methyl]amino}-3,4-dioxo-cyclobut-1-enylamino)benzamide
3/- methyl (S)-1-[2-fluoro-3-(2-{[(5-methylfuran-2-yl)-(tetrahydrothiophen-2-yl)methyl]amino}-3,4-dioxo-cyclobut-1-enylamino)benzoyl]pyrrolidine-2-carboxylate
4/- isopropyl (S)-1-[2-fluoro-3-(2-{[(5-methyl-furan-2-yl)-(tetrahydrothiophen-2-yl)methyl]amino}-3,4-dioxocyclobut-1-enylamino)benzoyl]pyrrolidine-2-carboxylate
5/- ethyl (S)-1-[2-fluoro-3-(2-{[(5-methylfuran-2-yl)-(tetrahydrothiophen-2-yl)methyl]amino}-3,4-dioxo-cyclobut-1-enylamino)benzoyl]pyrrolidine-2-carboxylate
6/- methyl (R)-1-[2-hydroxy-3-(2-{[(5-methylfuran-2-yl)-(tetrahydrothiophen-2-yl)methyl]amino}-3,4-dioxo-cyclobut-1-enylamino)benzoyl]pyrrolidine-2-carboxylate
7/- methyl (S)-1-[2-hydroxy-3-(2-{[(5-methylfuran-2-yl)-(tetrahydrothiophen-2-yl)methyl]amino}-3,4-dioxo-cyclobut-1-enylamino)benzoyl]pyrrolidine-2-carboxylate
8/- 2-hydroxy-N-methyl-3-(2-{[(S)-(5-methylfuran-2-yl)tetrahydrothiophen-2-ylmethyl]amino}-3,4-dioxocyclo-but-1-enylamino)-N-(2,2,2-trifluoroethyl)benzamide
9/- methyl {[2-hydroxy-3-(2-{[(5-methylfuran-2-yl)-(tetrahydrothiophen-2-yl)methyl]amino}-3,4-dioxocyclo-but-1-enylamino)benzoyl]methylamino}acetate
10/- 6-chloro-2-hydroxy-N,N-dimethyl-3-(2-{[(5-methylfuran-2-yl)-(tetrahydrothiophen-2-yl)methyl]-amino}-3,4-dioxocyclobut-1-enylamino)benzenesulfonamide
11/- 2-hydroxy-N,N-dimethyl-3-(2-{[(R)-(5-methyl-furan-2-yl)tetrahydro-furan-2-ylmethyl]amino}-3,4-dioxocyclobut-1-enylamino)benzamide
12/- 2-hydroxy-N,N-dimethyl-3-(2-{[(S)-(5-methyl-furan-2-yl)tetrahydrofuran-2-ylmethyl]amino}-3,4-dioxo-cyclobut-1-enylamino)benzamide
13/- 3-(3,4-dioxo-2-{[phenyl(tetrahydrofuran-2-yl)methyl]amino}cyclobut-1-enylamino)-2-hydroxy-N,N-dimethylbenzamide
14/- 3-(2-{[((R)-2,2-dimethyl-[1,3]dioxolan-4-yl)-(5-methylfuran-2-yl)methyl]amino}-3,4-dioxocyclobut-1-enylamino)-2-hydroxy-N,N-dimethylbenzamide
15/ methyl (S)-1-[2-fluoro-3-(2-{[(5-methylfuran-2-yl)-(tetrahydrothiophen-2-yl)methyl]amino}-3,4-dioxo-cyclobut-1-enylamino)benzoyl]pyrrolidine-2-carboxylate
16/ 3-(2-hydroxypyridin-3-ylamino)-4-{[(5-methylfuran-2-yl)-(tetrahydrothiophen-2-yl)methyl]-amino}cyclobut-3-ene-1,2-dione
17/ 3-{[(5-methylfuran-2-yl)-(tetrahydrothiophen-2-yl)-methyl]-amino}-4-(1-methyl-2-oxo-1,2-dihydropyridin-3-ylamino)cyclobut-3-ene-1,2-dione
18/ (-)-2-hydroxy-N-methyl-3-(2-{[(S)-(5-methylfuran-2-yl)-(tetrahydrothiophen-2-yl)methyl]amino}-3,4-dioxocyclobut-1-enylamino)-N-(2,2,2-trifluoroethyl)-benzamide
19/ methyl (-)-{[2-hydroxy-3-(2-{[(S)-(5-methylfuran-2-yl)-(tetrahydrothiophen-2-yl)methyl]amino}-3,4-dioxocyclobut-1-enylamino)benzoyl]methylamino}acetate
20/ methyl (-)-1-[2-hydroxy-3-(2-{[((S)-5-methylfuran-2-yl)-(tetrahydrothiophen-2-yl)methyl]amino}-3,4-dioxocyclobut-1-enylamino)benzoyl]pyrrolidine-2-(R)-carboxylate
21/ (-)-6-chloro-2-hydroxy-N,N-dimethyl-3-(2-{[((S)-5-methylfuran-2-yl)-(tetrahydrothiophen-2-yl)-methyl]amino}-3,4-dioxocyclobut-1-enylamino)benzamide
22/ (-)-3-[4-chloro-2-hydroxy-3-(4-methylpiperazine-1-sulfonyl)phenylamino]-4-{[(5-methylfuran-2-yl)-(tetrahydrothiophen-2-yl)methyl]amino}-cyclobut-3-ene-1,2-dione.
